# EUROPEAN PATENT SPECIFICATION

(11) **EP 2 415 474 B1**
(45) Date of publication and mention of the grant of the patent: **28.08.2013**
(21) Application number: 10758729.7
(22) Date of filing: 30.03.2010
(51) Int. Cl.: A61K 31/55, A61P 9/00, A61P 27/02, C07D 487/04

(54) **PHARMACEUTICAL COMPOSITION FOR TREATMENT OR PREVENTION OF OPHTHALMIC DISEASES**
PHARMAZEUTISCHE ZUSAMMENSETZUNG ZUR BEHANDLUNG UND PRÄVENTION VON AUGENLEIDEN
COMPOSITION PHARMACEUTIQUE POUR LA PRÉVENTION OU LE TRAITEMENT DE MALADIES OPHTALMIQUES

(30) Priority: 30.03.2009 JP 2009083582
(43) Date of publication of application: 08.02.2012
(73) Proprietor: Ube Industries, Ltd., Ube-shi Yamaguchi 755-8633 (JP)
(72) Inventor: HAGIHARA, Masahiko, Ube-shi Yamaguchi 755-8633 (JP); AGA, Yasuhiro, Ube-shi Yamaguchi 755-8633 (JP); HASEGAWA, Tohru, Ube-shi Yamaguchi 755-8633 (JP)
(74) Representative: HOFFMANN EITLE
(86) International application number: PCT/JP2010/055720
(87) International publication number: WO 2010/113958

(56) References cited:
- EP-A1- 2 221 308
- WO-A1-98/14192
- WO-A1-03/013511
- WO-A1-2009/063990
- JP-T- 2001 501 936
- JP-T- 2004 528 373
- ROSEMARY J SANTULLI ET AL: "Studies with an orally bioavailable alpha(v) integrin antagonist in animal models of ocular vasculopathy: Retinal neovascularization in mice and retinal vascular permeability in diabetic rats", JOURNAL OF PHARMACOLOGY AND EXPERIMENTAL THERAPEUTICS, AMERICAN SOCIETY FOR PHARMACOLOGY AND EXPERIMENTAL THERAPEUTICS, US, vol. 324, no. 3, 1 March 2008 (2008-03-01) , pages 894-901, XP002624844, ISSN: 0022-3565, DOI: 10.1124/JPET.107.131656 [retrieved on 2007-12-14]
- WILKINSON-BERKA JENNIFER L ET AL: "SB-267268, a nonpeptidic antagonist of alpha(v)beta(3) and alpha(v)beta(5) integrins, reduces angiogenesis and VEGF expression in a mouse model of retinopathy of prematurity", IOVS, vol. 47, no. 4, April 2006 (2006-04), pages 1600-1605, XP002681074, ISSN: 0146-0404
- LETOURNEAU, J.J. ET AL.: 'Synthesis and initial evaluation of novel, non-peptidic antagonists of the av-integrins avp3 and alphavbeta5' BIOORGANIC & MEDICINAL CHEMISTRY LETTERS vol. 19, no. 2, January 2009, pages 352 - 355
- JENNIFER L. WILKINSON-BERKA ET AL.: 'SB-267268, a Nonpeptidic Antagonist of vs3 and vs5 Integrins, Reduces Angiogenesis and VEGF Expression in a Mouse Model of Retinopathy of Prematurity' INVEST. OPHTHALMOL. VIS. SCI. vol. 47, 2006, pages 1600 - 1605

## Description

### TECHNICAL FIELD

The present invention relates to a medical composition containing a novel benzazepinone compound which is useful as a medicine and a pharmaceutically acceptable salt thereof as an effective ingredient. More specifically, the benzazepinone compound according to the present invention is useful for a treatment agent and/or prophylaxis agent of diseases to which an αv integrin receptor pertains such as angiogenesis in eyes, since it is an antagonistic drug of the αv integrin receptor (particularly, αvβ3 and αvβ5).

### BACKGROUND ART

Intraocular neovascular diseases such as diabetic retinopathy, retinopathy of prematurity, age-related macular degeneration (hereinafter abbreviated to as "AMD"), etc. cause severe reduction of vision, which is a disease state frequently being a problem in clinical ophthalmology. In particular, AMD becomes main cause of loss of eyesight or reduction of vision in advanced nations including Europe and United States, and appears in aged people of mainly 50-years old or more. AMD shows symptoms that the center of the visual field becomes blurred, distorted, invisible, dark, etc., by degeneration of macular area due to abnormal aging at the retinal pigment epithelial cells.
InAMD, there exist two types of forms, which are so-called "dry" and "wet" forms. The dry form is one in which macular area is damaged and withered, but there is no subjective symptoms so that no treatment is particularly required. The wet form is one in which angiogenesis is generated due to stimulation by wastes and the retina is degenerated, which causes detachment of retinal pigment epithelium, neogenesis of choroid blood vessels or subretinal hemorrhage, which leads to loss of vision so that a therapy is definitely required.
As a treating method for the wet type AMD, a laser photocoagulation has been reported to be effective. However, the photocoagulation is a method which destroys retinal tissue, and reduction of vision is remarkable due to the therapy itself in the case that a focus is large, so that there are many unsuitable cases for applying the therapy. As the other therapeutic method, a drug therapy which targets inhibition of angiogenesis has been carried out.
VEGF (vascular endothelial growth factor) is a glycoprotein that has proliferation and/or differentiation of a vascular endothelial cell, induction of vascular permeability, vasodilator action, etc., and in recent years, it has been clarified that it pertains to diseases related to abnormal angiogenesis or elevated vascular permeability LUCENTIS (Tradename), etc., are antibodies of the VEGF, and LUCENTIS has already been commercially available as an AMD treatment agent. However, these VEGF antibodies have been reported to have various side effects.
The integrin inhibitor also inhibits angiogenesis, so that it has been investigated as one of AMD treatment agents.
The integrin is a transmembrane glycoprotein complex of a hetero dimer constituted by an α-subunit and a β-subunit, which intermediates cell adhesion phenomenon and signaling process. A relative affinity and specificity regarding ligand binding are determined by combination of various α-subunits and β-subunits.
The integrin αvβ3 is expressed in many cell forms, and has been reported to intermediate some biologically related processes including adhesion of osteoclasts to bone matrix, migration of vascular endothelial cell and angiogenesis. Also, the integrin αvβ5 has been classified into the same vitronectin receptor as in the integrin αvβ3. However, it has been suggested that biological functions of these two kinds of integrins exerted on proangiogenic are different from each other.
As an integrin inhibitor against AMD, it has now been reported to use an RGD-containing polypeptide compound which is an integrin αvβ3 and/or αvβ5 antagonist or a non-peptide type compound which imitates RGD (see Patent Literature 1). However, the administration method thereof is an injection to the vitreous body of the eye, so that there are limits in administration intervals or a number of administration times in the viewpoints of invasiveness or QOL.
With regard to non-peptide type compounds, it has been described to be effective for the treatment by oral administration (see Non-Patent Literature 1). However, adhesion inhibitory activities are different depending on the kinds of animal cells to be used, so that it is difficult to expect the medical effects on human, and it cannot be said that sufficient effects can be obtained in the aspects of medical effects and pharmacokinetics, etc.
Heretofore, benzazepinone compounds similar to the compounds of the present invention have been disclosed, and it has been known that these compounds have integrin αvβ3 and αvβ5 inhibitory activity (see Patent Literatures 2 to 5). Also, benzazepinone compounds having treatment effects on AMD have also been reported (see Non-Patent Literature 2), but its administration form is intraperitoneal administration, so that improvement in pharmacokinetic aspects is necessary, and it cannot be said that sufficient medical effects can be obtained.
Accordingly, it has been desired to develop a compound which is a low molecule and having a non-peptide type αv integrin receptor antagonistic action, which is further excellent in effects, pharmacodynamic properties, and, pharmacokinetic properties such as bioavailability (BA) and duration of action, etc.
Moreover, in any of the above-mentioned literatures, there is no specific disclosure about a benzazepinone compound having a ring-fused imidazole ring structure at the side chain of the benzazepin ring according to the compound of the present invention.

[Patent Literature 1] WO 03/13511A
[Patent Literature 2] WO 98/14192A
[Patent Literature 3] WO 99/15170A
[Patent Literature 4] WO 99/15178A
[Patent Literature 5] WO 02/90325A

[Non-Patent Literature 1] J. Pharmacol. Exp. Ther., 324, 894(2008)
[Non-Patent Literature 2] Invest. Ophthal mol. Vis. Sci., 47, 1600(2006)

### DISCLOSURE OF THE INVENTION

### PROBLEMS TO BE SOLVED BY THE INVENTION

The present inventors have carried out researches for the purpose of providing a medical composition containing a specific benzazepinone compound, which is capable of orally administering, less side effects, and useful for treatment or prophylaxis of diseases caused by ocular angiogenesis, whereby they have accomplished the present invention. That is, the present invention is to provide a medical composition containing a novel benzazepinone compound and a pharmaceutically acceptable salt thereof which has a potent αv integrin receptor antagonistic action, and excellent angiogenesis inhibitory activity provided by oral absorbability and continuity of a function.

### MEANS TO SOLVE THE PROBLEMS

The present inventors have intensively researched on a benzazepinone compound, and as a result, they have found that a group of benzazepinone compound having a specific heteroarylethoxy group at the 8-position of the benzazepinone ring, i.e., a group of benzazepinone compound having a 5,6,7,8-tetrahydro-imidazo[1,2-a]-pyrimidin-2-yl group or 1,2,3,4-tetrahydro-imidazo[1,2-b][1,2,4]triazin-6-yl group as a heteroaryl group has a potent αv integrin receptor (in particular, αvβ3 and αvβ5) antagonistic action, and excellent oral absorbability and continuity of a function, whereby they have accomplished the present invention.

The present invention is directed to
(1) a medical composition for use in a method of the treatment or prophylaxis of diseases caused by angiogenesis of eyes which comprises a compound represented by the following formula (I):

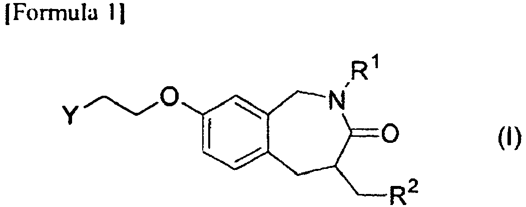

wherein R¹ represents a C₁-C₆ alkyl group or halogeno-C₁-C₆ alkyl group, R² represents a carboxyl group which may be protected, Y represents a group represented by the formula (II):

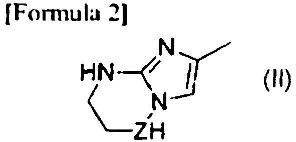

wherein Z represents CH or a nitrogen atom, or a pharmaceutically acceptable salt thereof as an active ingredient,
(2) the medical composition as described in the above (1), wherein R¹ is a C₁-C₄ alkyl group, fluoro-C₁-C₄ alkyl group or chloro-C₁-C₄ alkyl group,
(3) the medical composition as described in the above (1), wherein R¹ is an ethyl group, propyl group, isopropyl group, butyl group, isobutyl group, sec-butyl group, difluoromethyl group, trifluoromethyl group, 2-fluoroethyl group, 2,2-difluoroethyl group, 2,2,2-trifluoroethyl group or 2,2,2-trichloroethyl group,
(4) the medical composition as described in the above (1), wherein R¹ is an ethyl group, difluoromethyl group, trifluoromethyl group, 2-fluoroethyl group, 2,2-difluoroethyl group or 2,2,2-trifluoroethyl group,
(5) the medical composition as described in the above (1), wherein R¹ is an ethyl group, trifluoromethyl group or 2,2,2-trifluoroethyl group,
(6) the medical composition as described in the above (1), wherein R² is a carboxyl group which may be protected by a C₁-C₁₂ alkyl group, C₇-C₁₈ aralkyl group, C₁-C₂ alkyl group substituted by C₂-C₅ alkanoyloxy group, C₁-C₂ alkyl group substituted by (C₁-C₄ alkoxy)carbonyloxy group, N,N-dimethylaminocarbonylmethyl group, 2-(morpholin-4-yl)ethyl group, (5-methyl-2-oxo-1,3-dioxolen-4-yl)methyl group or (5-phenyl-2-oxo-1,3-dioxolen-4-yl)methyl group,
(7) the medical composition as described in the above (1), wherein R² is a carboxyl group which may be protected by a methyl group, ethyl group, propyl group, isopropyl group, 1-ethylpropyl group, butyl group, isobutyl group, sec-butyl group, tert-butyl group, 3,3-dimethylbutyl group, pentyl group, isopentyl group, neopentyl group, tert-pentyl group, 1-methylbutyl group, hexyl group, 1-methylpentyl group, 2-methylpentyl group, 3-methylpentyl group, 1-ethylbutyl group, 2-ethylbutyl group, heptyl group, octyl group, nonyl group, decyl group, undecyl group, dodecyl group, benzyl group, phenethyl group, phenylpropyl group, phenylbutyl group, phenylpentyl group, phenylhexyl group, phenylheptyl group, phenyloctyl group, phenylnonyl group, phenyldecyl group, phenylundecyl group, phenyldodecyl group, acetoxymethyl group, 1-acetoxy-ethyl group, pivaloyloxymethyl group, 1-pivaloyloxyethyl group, ethoxycarbonyloxymethyl group, 1-ethoxycarbonyloxyethyl group, N,N-dimethylaminocarbonylmethyl group, 2-(morpholin-4-yl)ethyl group or (5-methyl-2-oxo-1,3-dioxolen-4-yl)methyl group,
(8) the medical composition as described in the above (1), wherein R² is a carboxyl group which may be protected by a methyl group, ethyl group, propyl group, isopropyl group, 1-ethylpropyl group, butyl group, 3,3-dimethylbutyl group, pentyl group, hexyl group, heptyl group, octyl group, nonyl group, decyl group, undecyl group, dodecyl group, benzyl group, phenethyl group, phenylpropyl group, phenylbutyl group, phenylpentyl group, phenylhexyl group, phenylheptyl group, phenyloctyl group, phenylnonyl group, phenyldecyl group, phenylundecyl group, phenyldodecyl group, acetoxymethyl group, pivaloyloxymethyl group, 1-pivaloyloxyethyl group, N,N-dimethylamino-carbonylmethyl group, 2-(morpholin-4-yl)ethyl group or (5-methyl-2-oxo-1,3-dioxolen-4-yl)methyl group,
(9) the medical composition as described in the above (1), wherein R² is a carboxyl group which may be protected by an ethyl group, propyl group, isopropyl group, butyl group, pentyl group, hexyl group, heptyl group, octyl group, nonyl group, decyl group, undecyl group, benzyl group, 2-phenethyl group, 3-phenylpropyl group, 4-phenylbutyl group, 5-phenylpentyl group, 6-phenylhexyl group, 7-phenylheptyl group, 8-phenyloctyl group, 9-phenylnonyl group, 10-phenyldecyl group, pivaloyloxymethyl group, N,N-dimethylaminocarbonylmethyl group, 2-(morpholin-4-yl)ethyl group or (5-methyl-2-oxo-1,3-dioxolen-4-yl)methyl group,
(10) the medical composition as described in the above (1), wherein R¹ is a C₁-C₄ alkyl group, fluoro-C₁-C₄ alkyl group or chloro-C₁-C₄ alkyl group, and
   R² is a carboxyl group which may be protected by a C₁-C₁₂ alkyl group, C₇-C₁₈ aralkyl group, C₁-C₂ alkyl group substituted by C₂-C₅ alkanoyloxy group, C₁-C₂ alkyl group substituted by (C₁-C₄ alkoxy)carbonyloxy group, N,N-dimethylaminocarbonylmethyl group, 2-(morpholin-4-yl)ethyl group, (5-methyl-2-oxo-1,3-dioxolen-4-yl)methyl group or (5-phenyl-2-oxo-1,3-dioxolen-4-yl)methyl group,
(11) the medical composition as described in the above (1), wherein R¹ is an ethyl group, propyl group, isopropyl group, butyl group, isobutyl group, sec-butyl group, difluoromethyl group, trifluoromethyl group, 2-fluoroethyl group, 2,2-difluoroethyl group, 2,2,2-trifluoroethyl group or 2,2,2-trichloroethyl group, and
   R² is a carboxyl group which may be protected by a methyl group, ethyl group, propyl group, isopropyl group, 1-ethylpropyl group, butyl group, isobutyl group, sec-butyl group, tert-butyl group, 3,3-dimethylbutyl group, pentyl group, isopentyl group, neopentyl group, tert-pentyl group, 1-methylbutyl group, hexyl group, 1-methylpentyl group, 2-methylpentyl group, 3-methylpentyl group, 1-ethylbutyl group, 2-ethylbutyl group, heptyl group, octyl group, nonyl group, decyl group, undecyl group, dodecyl group, benzyl group, phenethyl group, phenylpropyl group, phenylbutyl group, phenylpentyl group, phenylhexyl group, phenylheptyl group, phenyloctyl group, phenylnonyl group, phenyldecyl group, phenylundecyl group, phenyldodecyl group, acetoxymethyl group, 1-acetoxyethyl group, pivaloyloxymethyl group, 1-pivaloyl-oxyethyl group, ethoxycarbonyloxymethyl group, 1-ethoxycarbonyloxyethyl group, N,N-dimethylaminocarbonylmethyl group, 2-(morpholin-4-yl)ethyl group or (5-methyl-2-oxo-1,3-dioxolen-4-yl)methyl group,
(12) the medical composition as described in the above (1), wherein R¹ is an ethyl group, difluoromethyl group, trifluoromethyl group, 2-fluoroethyl group, 2,2-difluoroethyl group or 2,2,2-trifluoroethyl group, and
   R² is a carboxyl group which may be protected by a methyl group, ethyl group, propyl group, isopropyl group, 1-ethylpropyl group, butyl group, 3,3-dimethylbutyl group, pentyl group, hexyl group, heptyl group, octyl group, nonyl group, decyl group, undecyl group, dodecyl group, benzyl group, phenethyl group, phenylpropyl group, phenylbutyl group, phenylpentyl group, phenylhexyl group, phenylheptyl group, phenyloctyl group, phenylnonyl group, phenyldecyl group, phenylundecyl group, phenyldodecyl group, acetoxymethyl group, pivaloyloxymethyl group, 1-pivaloyl-oxyethyl group, N,N-dimethylaminocarbonylmethyl group, 2-(morpholin-4-yl)ethyl group or (5-methyl-2-oxo-1,3-dioxolen-4-yl)methyl group,
(13) the medical composition as described in the above (1), wherein R¹ is an ethyl group, trifluoromethyl group or 2,2,2-trifluoroethyl group, and
   R² is a carboxyl group which may be protected by an ethyl group, propyl group, isopropyl group, butyl group, pentyl group, hexyl group, heptyl group, octyl group, nonyl group, decyl group, undecyl group, benzyl group, 2-phenethyl group, 3-phenylpropyl group, 4-phenylbutyl group, 5-phenylpentyl group, 6-phenylhexyl group, 7-phenylheptyl group, 8-phenyloctyl group, 9-phenylnonyl group, 10-phenyldecyl group, pivaloyloxymethyl group, N,N-dimethylaminocarbonylmethyl group, 2-(morpholin-4-yl)ethyl group or (5-methyl-2-oxo-1,3-dioxolen-4-yl)methyl group,
(14) the medical composition as described in the above (1), wherein the compound is represented by the following formula (III):

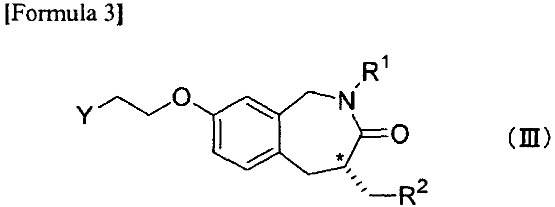

wherein the carbon atom to which * is attached has a configuration (S), and R¹, R² and Y have the same meanings as defined above,
(15) the medical composition as described in the above (2), wherein the compound is shown by the formula (III),
(16) the medical composition as described in the above (3), wherein the compound is shown by the formula (III),
(17) the medical composition as described in the above (4), wherein the compound is shown by the formula (III),
(18) the medical composition as described in the above (5), wherein the compound is shown by the formula (III),
(19) the medical composition as described in the above (6), wherein the compound is shown by the formula (III),
(20) the medical composition as described in the above (7), wherein the compound is shown by the formula (III),
(21) the medical composition as described in the above (8), wherein the compound is shown by the formula (III),
(22) the medical composition as described in the above (9), wherein the compound is shown by the formula (III),
(23) the medical composition as described in the above (10), wherein the compound is shown by the formula (III),
(24) the medical composition as described in the above (11), wherein the compound is shown by the formula (III),
(25) the medical composition as described in the above (12), wherein the compound is shown by the formula (III),
(26) the medical composition as described in the above (13), wherein the compound is shown by the formula (III),
(27) the medical composition as described in the above (1), wherein the benzazepinone compound is
   3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetic acid,
   (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetic acid,
   ethyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
   ethyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
   propyl 3-oxo-8-[2-(5,6,7,8-tetrahydroimidazo[1,2-a]pyrimidin-2-yl)ethoxy]-2-(2,2,2-trifluoroethyl)-2,3,4,5-tetrabydro-1H-2-benzazepin-4-acetate,
   propyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
   isopropyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
   isopropyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
   heptyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
   heptyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
   undecyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
   undecyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
   benzyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
   benzyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
   (10-phenyl)decyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
   (10-phenyl)decyl (4S)-3-oxo-8-[2-(5,6,7,8-tetrahydroimidazo[1,2-a]pyrimidin-2-yl)-ethoxy]-2-(2,2,2-trifluoroethyl)-2,3,4,5-tetrahydro-1H-2-benzazepin-4-acetate, pivaloyloxymethyl 3-oxo-8-[2-(5,6,7,8-tetrahydroimidazo[1,2-a]pyrimidin-2-yl)-ethoxy]-2-(2,2,2-trifluoroethyl)-2,3,4,5-tetrahydro-1H-2-benzazepin-4-acetate,
   pivaloyloxymethyl (4S)-3-oxo-8-[2-(5,6,7,8-tetrahydroimidazo[1,2-a]pyrimidin-2-yl)-ethoxy]-2-(2,2,2-trifluoroethyl)-2,3,4,5-tetrahydro-1H-2-benzazepin-4-acetate,
   (N,N-dimethylaminocarbonyl)methyl 3-oxo-8-[2-(5,6,7,8-tetrahydroimidazo[1,2-a]-pyrimidin-2-yl)ethoxy]-2-(2,2,2-trifluoroethyl)-2,3,4,5-tetrahydro-1H-2-benzazepin-4-acetate,
   (N,N-dimethylaminocarbonyl)methyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
   [2-(morpholin-4-yl)]ethyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
   [2-(morpholin-4-yl)]ethyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
   (5-methyl-2-oxo-[1,3]dioxolen-4-yl)methyl 3-oxo-8-[2-(5,6,7,8-tetrahydroimidazo-[1,2-a]pyrimidin-2-yl)ethoxy]-2-(2,2,2-trifluoroethyl)-2,3,4,5-tetrahydro-1H-2-benzazepin-4-acetate,
   (5-methyl-2-oxo-[1,3]dioxolen-4-yl)methyl (4S)-3-oxo-8-[2-(5,6,7,8-tetrahydro-imidazo [1,2-a]pyrimidin-2-yl)ethoxy]-2-(2,2,2-trifluoroethyl)-2,3,4,5-tetrahydro-1H-2-benzazepin-4-acetate,
   3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetic acid,
   (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetic acid,
   ethyl 3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
   ethyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
   propyl 3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
   propyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
   isopropyl 3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
   isopropyl (45)-3-oxo-8-[2-(1 ,2,3,4-tetrahydroimidazo[1,2-b][1,2,4]triazin-6-yl)-ethoxy]-2-(2,2,2-trifluoroethyl)-2,3,4,5-tetrahydro-1H-2-benzazepin-4-acetate,
   heptyl 3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
   heptyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
   undecyl 3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
   undecyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
   benzyl 3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
   benzyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
   (10-phenyl)decyl 3-oxo-8-[2-(1,2,3,4-tetrahydroimidazo[1,2-b][1,2,4]triazin-6-yl)-ethoxy]-2-(2,2,2-trifluoroethyl)-2,3,4,5-tetrahydro-1H-2-benzazepin-4-acetate,
   (10-phenyl)decyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
   pivaloyloxymethyl 3-oxo-8-[2-(1,2,3,4-tetrahydroimidazo[1,2-b][1,2,4]triazin-6-yl)-ethoxy]-2-(2,2,2-trifluoroethyl)-2,3,4,5-tetrahydro-1H-2-benzazepin-4-acetate,
   pivaloyloxymethyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
   (N,N-dimethylaminocarbonyl)methyl 3-oxo-8-[2-(1,2,3,4-tetrahydroimidazo[1,2-b]-[1,2,4]triazin-6-yl)ethoxy]-2-(2,2,2-trifluoroethyl)-2,3,4,5-tetrahydro-1H-2-benzazepin-4-acetate,
   (N,N-dimethylaminocarbonyl)methyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
   [2-(morpholin-4-yl)]ethyl 3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
   [2-(morpholin-4-yl)]ethyl (4S)-3-oxo-8-[2-(1,2,3,4-tetrahydroimidazo[1,2-b][1,2,4]-triazin-6-yl)ethoxy]-2-(2,2,2-trifluoroethyl)-2,3,4,5-tetrahydro-1H-2-benzazepin-4-acetate,
   (5-methyl-2-oxo-[1,3]dioxolen-4-yl)methyl 3-oxo-8-[2-(1,2,3,4-tetrahydroimidazo-[1,2-b][1,2,4]triazin-6-yl)ethoxy]-2-(2,2,2-trifluoroethyl)-2,3,4,5-tetrahydro-1H-2-benzazepin-4-acetate, or
   (5-methyl-2-oxo-[1,3]dioxolen-4-yl)methyl (4S)-3-oxo-8-[2-(1,2,3,4-tetrahydro-imidazo[1,2-b][1,2,4]triazin-6-yl)ethoxy]-2-(2,2,2-trifluoroethyl)-2,3,4,5-tetrahydro-1H-2-benzazepin-4-acetate,
(28) the medical composition as described in the above (1) to (27), wherein an administration form is oral administration,
(29) the medical composition as described in the above (1) to (27), wherein the eye disease is intraocular neovascular disease,
(30) the medical composition as described in the above (1) to (27), wherein the eye disease is an age-related macular degeneration.

### EFFECTS OF THE INVENTION

The medical composition of the present invention comprising a benzazepinone compound represented by the formula (I) or a pharmaceutically acceptable salt thereof as an effective ingredient has, in addition to excellent α vintegrin receptor (in particular, αvβ3 and αvβ5) antagonistic action, excellent angiogenesis inhibitory activity, and, has excellent properties in the points of excellent solubility, oral absorbability, concentration in blood, metabolic stability, tissue distribution, bioavailability (BA), *in vitro* activity, *in vivo* activity, fast-acting pharmaceutical effect, sustained pharmaceutical effect, physical stability, drug interaction, influence to the activities based on the differences in animal species, toxicity, etc., and has high safety, so that it is useful as a medicine (in particular, a medicine for treatment or prophylaxis of diseases caused by ocular angiogenesis) for warm blooded one (in particular, for human).

### BRIEF DESCRIPTION OF THE DRAWING

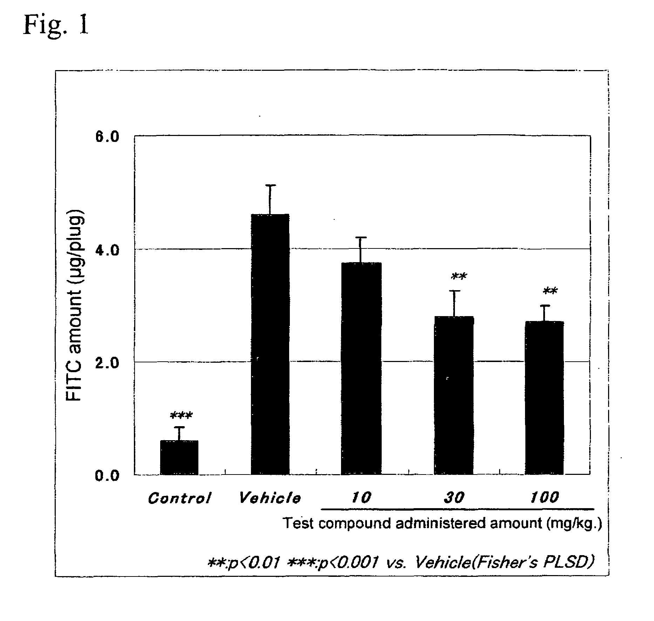

[Fig. 1] It is a graph showing an amount of extracted FITC-dextran from extracted Matrigel.

### BEST MODE TO CARRY OUT THE INVENTION

In the compound represented by the above-mentioned formula (I), as the "C1-C6 alkyl group" shown by R1, there may be mentioned, for example, a linear or branched C1-C6 alkyl group such as a methyl group, ethyl group, propyl group, isopropyl group, butyl group, isobutyl group, sec-butyl group, tert-butyl group, pentyl group, isopentyl group, neopentyl group, tert-pentyl group, 1-ethylpropyl group, 1-methylbutyl group, 2-methylbutyl group, 1,2-dimethylpropyl group, hexyl group, 1-methylpentyl group, 2-methylpentyl group, 3-methylpentyl group, 4-methylpentyl group, 1-ethylbutyl group, 2-ethylbutyl group, 1,1-dimethylbutyl group, 2,2-dimethylbutyl group, 3,3-dimethylbutyl group, 1,2-dimethylbutyl group, 1,3-dimethylbutyl group or 2,3-dimethylbutyl group, preferably a C1-C4 alkyl group, more preferably an ethyl group, propyl group, isopropyl group, butyl group, isobutyl group or see-butyl group, and particularly preferably an ethyl group.

The "halogeno-" portion of the "halogeno-C₁-C₆ alkyl group" shown by R¹ means a halogen atom, and such a halogen atom may be mentioned, for example, a fluorine atom, chlorine atom, bromine atom or iodine atom, preferably a fluorine atom, chlorine atom or bromine atom, more preferably a fluorine atom or chlorine atom, and particularly preferably a fluorine atom.

The "halogeno-C₁-C₆ alkyl group" shown by R¹ may be mentioned, for example, the above-mentioned "C₁-C₆ alkyl group" to which 1 or 2 or more halogen atoms are substituted such as a fluoromethyl group, chloromethyl group, bromomethyl group, iodomethyl group, difluoromethyl group, dichloromethyl group, dibromomethyl group, diiodomethyl group, trifluoromethyl group, trichloromethyl group, 1-fluoroethyl group, 2-fluoroethyl group, 2-chloroethyl group, 2-bromoethyl group, 2,2-difluoroethyl group, 2,2,2-trifluoroethyl group, pentafluoroethyl group, 2,2-dichloroethyl group, 2,2,2-trichloroethyl group, 1-fluoropropyl group, 2-fluoropropyl group, 3-fluoropropyl group, 3,3,3-trifluoropropyl group, perfluoropropyl group, 1-fluoromethylethyl group, 1-difluoromethylethyl group, 1-trifluoromethylethyl group, 1-fluoro-1-methylethyl group, 4-fluorobutyl group, perfluorobutyl group, 5-fluoropentyl group, perfluoropentyl group, 6-fluorohexyl group and perfluorohexyl group, preferably a fluoro-C₁-C₄ alkyl group or chloro-C₁-C₄ alkyl group, more preferably a difluoromethyl group, trifluoromethyl group, 2-fluoroethyl group, 2,2-difluoroethyl group, 2,2,2-trifluoroethyl group or 2,2,2-trichloroethyl group, and particularly preferably a trifluoromethyl group or 2,2,2-trifluoroethyl group.

R¹ is preferably a C₁-C₄ alkyl group, fluoro-C₁-C₄ alkyl group or chloro-C₁-C₄ alkyl group, more preferably an ethyl group, propyl group, isopropyl group, butyl group, isobutyl group, sec-butyl group, difluoromethyl group, trifluoromethyl group, 2-fluoroethyl group, 2,2-difluoroethyl group, 2,2,2-trifluoroethyl group or 2,2,2-trichloroethyl group, further more preferably an ethyl group, difluoromethyl group, trifluoromethyl group, 2-fluoroethyl group, 2,2-difluoroethyl group or 2,2,2-trifluoroethyl group, and particularly preferably an ethyl group, trifluoromethyl group or 2,2,2-trifluoro ethyl group.

The protective group for the carboxyl group which may be protected shown by R² of the formula (I) may be mentioned, for example, a C₁-C₁₂ alkyl group such as a methyl group, ethyl group, propyl group, isopropyl group, 1-ethylpropyl group, butyl group, isobutyl group, sec-butyl group, tert-butyl group, 3,3-dimethylbutyl group, pentyl group, isopentyl group, neopentyl group, tert-pentyl group, 1-methylbutyl group, hexyl group, 1-methylpentyl group, 2-methylpentyl group, 3-methylpentyl group, 1-ethylbutyl group, 2-ethylbutyl group, heptyl group, octyl group, nonyl group, decyl group, undecyl group, and dodecyl group; a C₇-C₁₈ aralkyl group such as a benzyl group, phenethyl group, phenylpropyl group, phenylbutyl group, phenylpentyl group, phenylhexyl group, phenylheptyl group, phenyloctyl group, phenylnonyl group, phenyldecyl group, phenylundecyl group, and phenyldodecyl group; a C₁-C₄ alkyl group substituted by a C₂-C₅ alkanoyloxy group such as an acetoxymethyl group, 1-acetoxyethyl group, 1-acetoxypropyl group, 1-acetoxybutyl group, propanoyloxymethyl group, 1-propanoyloxyethyl group, butanoyloxymethyl group, 1-butanoyloxyethyl group, pivaloyloxymethyl group, 1-pivaloyloxyethyl group, 1-pivaloyloxypropyl group and 1-pivaloyloxybutyl group; a C₁-C₄ alkyl group substituted by a (C₁-C₄ alkoxy)carbonyloxy group such as a methoxycarbonyloxymethyl group, 1-methoxycarbonyloxyethyl group, ethoxycarbonyloxymethyl group, 1-ethoxycarbonyloxyethyl group, propoxycarbonyloxymethyl group, 1-propoxycarbonyloxyethyl group, isopropoxycarbonyloxymethyl group, 1-isopropoxycarbonyloxyethyl group, butoxycarbonyloxymethyl group, 1-butoxycarbonyloxyethyl group, tert-butoxycarbonyloxymethyl group and 1-tert-butoxycarbonyloxyethyl group; an N,N-dialkylaminocarbonylalkyl group such as an N,N-dimethylaminocarbonylmethyl group and N,N-diethylaminocarbonylmethyl group; a 2-(N,N-dialkylamino)ethyl group such as 2-(N,N-dimethylamino)ethyl group and 2-(N,N-diethylamino)ethyl group; a 5-membered or 6-membered saturated heteromonocyclic ring-substituted alkyl group containing 1 or 2 hetero atoms selected from N, O and S such as 2-(morpholin-4-yl)ethyl group, 2-piperidinoethyl group and 2-(4-methylpiperidino)ethyl group; and a group in which it can be easily converted into the carboxyl group by deprotection in a living body such as a (5-methyl-2-oxo-1,3-dioxolen-4-yl)methyl group and (5-phenyl-2-oxo-1,3-dioxolen-4-yl)methyl group, etc.,
preferably a C₁-C₁₂ alkyl group, C₇-C₁₈ aralkyl group, C₁-C₂ alkyl group substituted by C₂-C₅ alkanoyloxy group, C₁-C₂ alkyl group substituted by (C₁-C₄ alkoxy)carbonyloxy group, N,N-dimethylaminocarbonylmethyl group, 2-(morpholin-4-yl)ethyl group, (5-methyl-2-oxo-1,3-dioxolen-4-yl)methyl group or (5-phenyl-2-oxo-1,3-dioxolen-4-yl)methyl group,
more preferably a methyl group, ethyl group, propyl group, isopropyl group, 1-ethylpropyl group, butyl group, isobutyl group, sec-butyl group, tert-butyl group, 3,3-dimethylbutyl group, pentyl group, isopentyl group, neopentyl group, tert-pentyl group, 1-methylbutyl group, hexyl group, 1-methylpentyl group, 2-methylpentyl group, 3-methylpentyl group, 1-ethylbutyl group, 2-ethylbutyl group, heptyl group, octyl group, nonyl group, decyl group, undecyl group, dodecyl group, benzyl group, phenethyl group, phenylpropyl group, phenylbutyl group, phenylpentyl group, phenylhexyl group, phenylheptyl group, phenyloctyl group, phenylnonyl group, phenyldecyl group, phenylundecyl group, phenyldodecyl group, acetoxymethyl group, 1-acetoxyethyl group, pivaloyloxymethyl group, 1-pivaloyloxyethyl group, ethoxycarbonyloxymethyl group, 1-ethoxycarbonyloxyethyl group, N,N-dimethylaminocarbonyl-methyl group, 2-(morpholin-4-yl)ethyl group or (5-methyl-2-oxo-1,3-dioxolen-4-yl)-methyl group,
further more preferably a methyl group, ethyl group, propyl group, isopropyl group, 1-ethylpropyl group, butyl group, 3,3-dimethylbutyl group, pentyl group, hexyl group, heptyl group, octyl group, nonyl group, decyl group, undecyl group, dodecyl group, benzyl group, phenethyl group, phenylpropyl group, phenylbutyl group, phenylpentyl group, phenylhexyl group, phenylheptyl group, phenyloctyl group, phenylnonyl group, phenyldecyl group, phenylundecyl group, phenyldodecyl group, acetoxymethyl group, pivaloyloxymethyl group, 1-pivaloyloxyethyl group, N,N-dimethylaminocarbonylmethyl group, 2-(morpholin-4-yl)ethyl group or (5-methyl-2-oxo-1,3-dioxolen-4-yl)methyl group,
and particularly preferably an ethyl group, propyl group, isopropyl group, butyl group, pentyl group, hexyl group, heptyl group, octyl group, nonyl group, decyl group, undecyl group, benzyl group, 2-phenethyl group, 3-phenylpropyl group, 4-phenylbutyl group, 5-phenylpentyl group, 6-phenylhexyl group, 7-phenylheptyl group, 8-phenyloctyl group, 9-phenylnonyl group, 10-phenyldecyl group, pivaloyloxymethyl group, N,N-dimethylaminocarbonylmethyl group, 2-(morpholin-4-yl)ethyl group or (5-methyl-2-oxo-1,3-dioxolen-4-yl)methyl group.

Z represents CH or nitrogen atom.

Y is a 5,6,7,8-tetrahydro-imidazo[1,2-a]pyrimidin-2-yl group or 1,2,3,4-tetrahydro-imidazo[1,2-b][1,2,4]triazin-6-yl group.

In the benzazepinone compound represented by the formula (I) of the present invention, preferred are
(1) a compound wherein R¹ is a C₁-C₄ alkyl group, fluoro-C₁-C₄ alkyl group or chloro-C₁-C₄ alkyl group,
(2) a compound wherein R¹ is an ethyl group, propyl group, isopropyl group, butyl group, isobutyl group, sec-butyl group, difluoromethyl group, trifluoromethyl group, 2-fluoroethyl group, 2,2-difluoroethyl group, 2,2,2-trifluoroethyl group or 2,2,2-trichloroethyl group,
(3) a compound wherein R¹ is an ethyl group, difluoromethyl group, trifluoromethyl group, 2-fluoroethyl group, 2,2-difluoroethyl group or 2,2,2-trifluoroethyl group,
(4) a compound wherein R¹ is an ethyl group, trifluoromethyl group or 2,2,2-trifluoroethyl group,
(5) a compound wherein R² is a carboxyl group which may be protected by a C₁-C₁₂ alkyl group, C₇-C₁₈ aralkyl group, C₁-C₂ alkyl group substituted by C₂-C₅ alkanoyloxy group, C₁-C₂ alkyl group substituted by (C₁-C₄ alkoxy)carbonyloxy group, N,N-dimethylaminocarbonylmethyl group, 2-(morpholin-4-yl)ethyl group, (5-methyl-2-oxo-1,3-dioxolen-4-yl)methyl group or (5-phenyl-2-oxo-1,3-dioxolen-4-yl)methyl group,
(6) a compound wherein R² is a carboxyl group which may be protected by a methyl group, ethyl group, propyl group, isopropyl group, 1-ethylpropyl group, butyl group, isobutyl group, sec-butyl group, tert-butyl group, 3,3-dimethylbutyl group, pentyl group, isopentyl group, neopentyl group, tert-pentyl group, 1-methylbutyl group, hexyl group, 1-methylpentyl group, 2-methylpentyl group, 3-methylpentyl group, 1-ethylbutyl group, 2-ethylbutyl group, heptyl group, octyl group, nonyl group, decyl group, undecyl group, dodecyl group, benzyl group, phenethyl group, phenylpropyl group, phenylbutyl group, phenylpentyl group, phenylhexyl group, phenylheptyl group, phenyloctyl group, phenylnonyl group, phenyldecyl group, phenylundecyl group, phenyldodecyl group, acetoxymethyl group, 1-acetoxyethyl group, pivaloyloxymethyl group, 1-pivaloyloxyethyl group, ethoxycarbonyloxymethyl group, 1-ethoxycarbonyl-oxyethyl group, N,N-dimethylaminocarbonylmethyl group, 2-(morpholin-4-yl)ethyl group or (5-methyl-2-oxo-1,3-dioxolen-4-yl)methyl group,
(7) a compound wherein R² is a carboxyl group which may be protected by a methyl group, ethyl group, propyl group, isopropyl group, 1-ethylpropyl group, butyl group, 3,3-dimethylbutyl group, pentyl group, hexyl group, heptyl group, octyl group, nonyl group, decyl group, undecyl group, dodecyl group, benzyl group, phenethyl group, phenylpropyl group, phenylbutyl group, phenylpentyl group, phenylhexyl group, phenylheptyl group, phenyloctyl group, phenylnonyl group, phenyldecyl group, phenylundecyl group, phenyldodecyl group, acetoxymethyl group, pivaloyloxymethyl group, 1-pivaloyloxyethyl group, N,N-dimethylaminocarbonylmethyl group, 2-(morpholin-4-yl)ethyl group or (5-methyl-2-oxo-1,3-dioxolen-4-yl)methyl group,
(8) a compound wherein R² is a carboxyl group which may be protected by an ethyl group, propyl group, isopropyl group, butyl group, pentyl group, hexyl group, heptyl group, octyl group, nonyl group, decyl group, undecyl group, benzyl group, 2-phenethyl group, 3-phenylpropyl group, 4-phenylbutyl group, 5-phenylpentyl group, 6-phenylhexyl group, 7-phenylheptyl group, 8-phenyloctyl group, 9-phenylnonyl group, 10-phenyldecyl group, pivaloyloxymethyl group, N,N-dimethylaminocarbonylmethyl group, 2-(morpholin-4-yl)ethyl group or (5-methyl-2-oxo-1,3-dioxolen-4-yl)methyl group.

Further, in the above-mentioned groups of (1)-(4) and (5)-(8), as the number becomes larger, a more preferred compound is indicated, and a compound obtained by optionally selecting R¹ from the groups (1)-(4), and R² from the groups (5)-(8), or by optionally combining them is also a preferred compound.
Such a compound may include, (9) a compound wherein R¹ is a C₁-C₄ alkyl group, fluoro-C₁-C₄ alkyl group or chloro-C₁-C₄ alkyl group, and
R² is a carboxyl group which may be protected by a C₁-C₁₂ alkyl group, C₇-C₁₈ aralkyl group, C₁-C₂ alkyl group substituted by C₂-C₅ alkanoyloxy group, C₁-C₂ alkyl group substituted by (C₁-C₄ alkoxy)carbonyloxy group, N,N-dimethylamino-carbonylmethyl group, 2-(morpholin-4-yl)ethyl group, (5-methyl-2-oxo-1,3-dioxolen-4-yl)methyl group or (5-phenyl-2-oxo-1,3-dioxolen-4-yl)methyl group, (10) a compound wherein R¹ is an ethyl group, propyl group, isopropyl group, butyl group, isobutyl group, sec-butyl group, difluoromethyl group, trifluoromethyl group, 2-fluoroethyl group, 2,2-difluoroethyl group, 2,2,2-trifluoroethyl group or 2,2,2-trichloroethyl group, and
R² is a carboxyl group which may be protected by a methyl group, ethyl group, propyl group, isopropyl group, 1-ethylpropyl group, butyl group, isobutyl group, sec-butyl group, tert-butyl group, 3,3-dimethylbutyl group, pentyl group, isopentyl group, neopentyl group, tert-pentyl group, 1-methylbutyl group, hexyl group, 1-methylpentyl group, 2-methylpentyl group, 3-methylpentyl group, 1-ethylbutyl group, 2-ethylbutyl group, heptyl group, octyl group, nonyl group, decyl group, undecyl group, dodecyl group, benzyl group, phenethyl group, phenylpropyl group, phenylbutyl group, phenylpentyl group, phenylhexyl group, phenylheptyl group, phenyloctyl group, phenylnonyl group, phenyldecyl group, phenylundecyl group, phenyldodecyl group, acetoxymethyl group, 1-acetoxyethyl group, pivaloyloxymethyl group, 1-pivaloyloxyethyl group, ethoxycarbonyloxymethyl group, 1-ethoxycarbonyloxyethyl group, N,N-dimethylaminocarbonylmethyl group, 2-(morpholin-4-yl)ethyl group or (5-methyl-2-oxo-1,3-dioxolen-4-yl)methyl group, (11) a compound wherein R¹ is an ethyl group, difluoromethyl group, trifluoromethyl group, 2-fluoroethyl group, 2,2-difluoroethyl group or 2,2,2-trifluoroethyl group, and
R² is a carboxyl group which may be protected by a methyl group, ethyl group, propyl group, isopropyl group, 1-ethylpropyl group, butyl group, 3,3-dimethylbutyl group, pentyl group, hexyl group, heptyl group, octyl group, nonyl group, decyl group, undecyl group, dodecyl group, benzyl group, phenethyl group, phenylpropyl group, phenylbutyl group, phenylpentyl group, phenylhexyl group, phenylheptyl group, phenyloctyl group, phenylnonyl group, phenyldecyl group, phenylundecyl group, phenyldodecyl group, acetoxymethyl group, pivaloyloxymethyl group, 1-pivaloyloxyethyl group, N,N-dimethylaminocarbonylmethyl group, 2-(morpholin-4-yl)ethyl group or (5-methyl-2-oxo-1,3-dioxolen-4-yl)methyl group, (12) a compound wherein R¹ is an ethyl group, trifluoromethyl group or 2,2,2-trifluoroethyl group, and
R² is a carboxyl group which may be protected by a ethyl group, propyl group, isopropyl group, butyl group, pentyl group, hexyl group, heptyl group, octyl group, nonyl group, decyl group, undecyl group, benzyl group, 2-phenethyl group, 3-phenylpropyl group, 4-phenylbutyl group, 5-phenylpentyl group, 6-phenylhexyl group, 7-phenylheptyl group, 8-phenyloctyl group, 9-phenylnonyl group, 10-phenyldecyl! group, pivaloyloxymethyl group, N,N-dimethylaminocarbonylmethyl group, 2-(morpholin-4-yl)ethyl group or (5-methyl-2-oxo-1,3-dioxolen-4-yl)methyl group.

In the present invention, preferred compounds having the formula (I) may be mentioned,
2-ethyl-3-oxo-8-[2-(5,6,7,8-tetrahydroimidazo[1,2-a]pyrimidin-2-yl)ethoxy]-2,3,4,5-tetrahydro-1H-2-benzazepin-4-acetic acid,
3-oxo-2-propyl-8-[2-(5,6,7,8-tetrahydroimidazo[1,2-a]pyrimidin-2-yl)ethoxy]-2,3,4,5-tetrahydro-1H-2-benzazepin-4-acetic acid,
2-isopropyl-3-oxo-8-[2-(5,6,7,8-tetrahydroimidazo[1,2-a]pyrimidin-2-yl)ethoxy]-2,3,4,5-tetrahydro-1H-2-benzazepin-4-acetic acid,
2-butyl-3-oxo-8-[2-(5,6,7,8-tetrahydroimidazo[1,2-a]pyrimidin-2-yl)ethoxy]-2,3,4,5-tetrahydro-1H-2-benzazepin-4-acetic acid,
2-isobutyl-3-oxo-8-[2-(5,6,7,8-tetrahydroimidazo[1,2-a]pyrimidin-2-yl)ethoxy]-2,3,4,5-tetmhydro-1H-2-benzazepin-4-acetic acid,
2-(sec-butyt)-3-oxo-8-[2-(5,6,7,8-tetrahydroimidazo[1,2-a]pyrimidin-2-yl)ethoxy]-2,3,4,5-tetrahydro-1H-2-benzazepin-4-acetic acid,
2-difluoromethyl-3-oxo-8-[2-(5,6,7,8-tetrahydroimidazo[1,2-a]pyrimidin-2-yl)ethoxy]-2,3,4,5-tetrahydro-1H-2-benzazepin-4-acetic acid,
3-oxo-8-[2-(5,6,7,8-tetrahydroimidazo[1,2-a]pyrimidin-2-yl)ethoxy]-2-trifluoromethyl-2,3,4,5-tetrahydro-1H-2-benzazepin-4-acetic acid,
2-(2-fluoromethyl)-3-oxo-8-[2-(5,6,7,8-tetrahydroimidazo[1,2-a]pyrimidin-2-yl)ethoxy]-2,3,4,5-tetrahydro-1H-2-benzazepin-4-acetic acid,
2-(2,2-difluoroethyl)-3-oxo-8-[2-(5,6,7,8-tetrahydroimidazo[1,2-a]pyrimidin-2-yl)-ethoxy]-2,3,4,5-tetrahydro-1H-2-benzazepin-4-acetic acid,
3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetic acid,
3-oxo-8-[2-(5,6,7,8-tetrahydroimidazo[1,2-a]pyrimidin-2-yl)ethoxy]-2-(2,2,2-trichloroethyl)-2,3,4,5-tetrahydro-1H-2-benzazepin-4-acetic acid,
2-ethyl-3-oxo-8-[2-(1,2,3,4-tetrahydroimidazo[1,2-b][1,2,4]triazin-6-yl)ethoxy]-2,3,4,5-tetrahydro-1H-2-benzazepin-4-acetic acid,
3-oxo-2-propyl-8-[2-(1,2,3,4-tetrahydroimidazo[1,2-b][1,2,4]triazin-6-yl)ethoxy]-2,3,4,5-tetrahydro-1H-2-benzazepin-4-acetic acid,
2-isopropyl-3-oxo-8-[2-(1,2,3,4-tetrahydroimidazo[1,2-b][1,2,4]triazin-6-yl)ethoxy]-2,3,4,5-tetrahydro-1H-2-benzazepin-4-acetic acid,
2-butyl-3-oxo-8-[2-(1,2,3,4-tetrahydroimidazo[1,2-b][1,2,4]triazin-6-yl)ethoxy]-2,3,4,5-tetrahydro-1H-2-benzazepin-4-acetic acid,
2-isobutyl-3-oxo-8-[2-(1,2,3,4-tetrahydroimidazo[1,2-b][1,2,4]triazin-6-yl)ethoxy]-2,3,4,5-tetrahydro-1H-2-benzazepin-4-acetic acid,
2-(sec-butyl)-3-oxo-8-[2-(1,2,3,4-tetrahydroimidazo[1,2-b][1,2,4]triazin-6-yl)ethoxy]-2,3,4,5-tetrahydro-1H-2-benzazepin-4-acetic acid,
2-difluoromethyl-3-oxo-8-[2-(1,2,3,4-tetrahydroimidazo[1,2-b][1,2,4]triazin-6-yl)-ethoxy]-2,3,4,5-tetrahydro-1H-2-benzazepin-4-acetic acid,
3-oxo-8-[2-(1,2,3,4-tetrahydroimidazo[1,2-b][1,2,4]triazin-6-yl)ethoxy]-2-trifluoromethyl-2,3,4,5-tetrahydro-1H-2-benzazepin-4-acetic acid,
2-(2-fluoroethyl)-3-oxo-8-[2-(1,2,3,4-tetrahydroimidazo[1,2-b][1,2,4]triazin-6-yl)-ethoxy]-2,3,4,5-tetrahydro-1H-2-benzazepin-4-acetic acid,
2-(2,2-difluoroethyl)-3-oxo-8-[2-(1,2,3,4-tetrahydroimidazo[1,2-b][1,2,4]triazin-6-yl)-ethoxy]-2,3,4,5-tetrahydro-1H-2-benzazepin-4-acetic acid,
3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetic acid or
3-oxo-8-[2-(1,2,3,4-tetrahydroimidazo[1,2-b][1,2,4]triazin-6-yl)ethoxy]-2-(2,2,2-trichloroethyl)-2,3,4,5-tetrahydro-1H-2-benzazepin-4-acetic acid,
or compounds in which the carboxyl group thereof is protected by a protective group (said protective group is a C₁-C₁₂ alkyl group, C₇-C₁₈ aralkyl group, C₁-C₂ alkyl group substituted by C₂-C₅ alkanoyloxy group, C₁-C₂ alkyl group substituted by (C₁-C₄ alkoxy)carbonyloxy group, N,N-dimethylaminocarbonylmethyl group, 2-(morpholin-4-yl)ethyl group, (5-methyl-2-oxo-1,3-dioxolen-4-yl)methyl group or (5-phenyl-2-oxo-1,3-dioxolen-4-yl)methyl group.),

more preferably compunds of
2-ethyl-3-oxo-8-[2-(5,6,7,8-tetrahydroimidazo[1,2-a]pyrimidin-2-yl)ethoxy]-2,3,4,5-tetrahydro-1H-2-benzazepin-4-acetic acid,
2-difluoromethyl-3-oxo-8-[2-(5,6,7,8-tetrahydroimidazo[1,2-a]pyrimidin-2-yl)ethoxy]-2,3,4,5-tetrahydro-1H-2-benzazepin-4-acetic acid,
3-oxo-8-[2-(5,6,7,8-tetrahydroimidazo[1,2-a]pyrimidin-2-yl)ethoxy]-2-trifluoromethyl-2,3,4,5-tetrahydro-1H-2-benzazepin-4-acetic acid,
2-(2-fluoroethyl)-3-oxo-8-[2-(5,6,7,8-tetrahydroimidazo[1,2-a]pyrimidin-2-yl)ethoxy]-2,3,4,5-tetrahydro-1H-2-benzazepin-4-acetic acid,
2-(2,2-difluoroethyl)-3-oxo-8-[2-(5,6,7,8-tetrahydroimidazo[1,2-a]pyrimidin-2-yl)-ethoxy]-2,3,4,5-tetrahydro-1H-2-benzazepin-4-acetic acid,
3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetic acid,
2-ethyl-3-oxo-8-[2-(1,2,3,4-tetrahydroimidazo[1,2-b][1,2,4]triazin-6-yl)ethoxy]-2,3,4,5-tetrahydro-1H-2-benzazepin-4-acetic acid,
2-difluoromethyl-3-oxo-8-[2-(1,2,3,4-tetrahydroimidazo[1,2-b][1,2,4]triazin-6-yl)-ethoxy]-2,3,4,5-tetrahydro-1H-2-benzazepin-4-acetic acid,
3-oxo-8-[2-(1,2,3,4-tetrahydroimidazo[1,2-b][1,2,4]triazin-6-yl)ethoxy]-2-trifluoromethyl-2,3,4,5-tetrahydro-1H-2-benzazepin-4-acetic acid,
2-(2-fluoroethyl)-3-oxo-8-[2-(1,2,3,4-tetrahydroimidazo[1,2-b][1,2,4]triazin-6-yl)-ethoxy]-2,3,4,5-tetrahydro-1H-2-benzazepin-4-acetic acid,
2-(2,2-difluoroethyl)-3-oxo-8-[2-(1,2,3,4-tetrahydroimidazo[1,2-b][1,2,4]triazin-6-yl)-ethoxy]-2,3,4,5-tetrahydro-1H-2-benzazepin-4-acetic acid or
3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetic acid,
   or compounds in which the carboxyl group thereof is protected by a protective group (said protective group is a methyl group, ethyl group, propyl group, isopropyl group, 1-ethylpropyl group, butyl group, isobutyl group, sec-butyl group, tert-butyl group, 3,3-dimethylbutyl group, pentyl group, isopentyl group, neopentyl group, tert-pentyl group, 1-methylbutyl group, hexyl group, 1-methylpentyl group, 2-methylpentyl group, 3-methylpentyl group, 1-ethylbutyl group, 2-ethylbutyl group, heptyl group, octyl group, nonyl group, decyl group, undecyl group, dodecyl group, benzyl group, phenethyl group, phenylpropyl group, phenylbutyl group, phenylpentyl group, phenylhexyl group, phenylheptyl group, phenyloctyl group, phenylnonyl group, phenyldecyl group, phenylundecyl group, phenyldodecyl group, acetoxymethyl group, 1-acetoxy-ethyl group, pivaloyloxymethyl group, 1-pivaloyloxyethyl group, ethoxycarbonyloxymethyl group, 1-ethoxycarbonyloxyethyl group, N,N-dimethylaminocarbonylmethyl group, 2-(morpholin-4-yl)ethyl group or (5-methyl-2-oxo-1,3-dioxolen-4-yl)methyl group.),

further more preferably compounds of
2-ethyl-3-oxo-8-[2-(5,6,7,8-tetrahydroimidazo[1,2-a]pyrimidin-2-yl)ethoxy]-2,3,4,5-tetrahydro-1H-2-benzazepin-4-acetic acid,
(4S)-2-ethyl-3-oxo-8-[2-(5,6,7,8-tetrahydroimidazo[1,2-a]pyrimidin-2-yl)ethoxy]-2,3,4,5-tetrahydro-1H-2-benzazepin-4-acetic acid,
3-oxo-8-[2-(5,6,7,8-tetrahydroimidazo[1,2-a]pyrimidin-2-yl)ethoxy]-2-trifluoromethyl-2,3,4,5-tetrahydro-1H-2-benzazepin-4-acetic acid,
(4S)-3-oxo-8-[2-(5,6,7,8-tetrahydroimidazo[1,2-a]pyrimidin-2-yl)ethoxy]-2-trifluoromethyl-2,3,4,5-tetrahydro-1H-2-benzazepin-4-acetic acid,
3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetic acid,
(4S)-3-oxo-8-[2-(5,6,7,8-tetrahydrolmidazo[1,2-a]pyrimidin-2-yl)ethoxy]-2-(2,2,2-trifluoroethyl)-2,3,4,5-tetrahydro-1H-2-benzazepin-4-acetic acid,
2-ethyl-3-oxo-8-[2-(1,2,3,4-tetrahydroimidazo[1,2-b][1,2,4]triazin-6-yl)ethoxy]-2,3,4,5-tetrahydro-1H-2-benzazepin-4-acetic acid,
(4S)-2-ethyl-3-oxo-8-[2-(1,2,3,4-tetrahydroimidazo[1,2-b][1,2,4]triazin-6-yl)ethoxy]-2,3,4,5-tetrahydro-1H-2-benzazepin-4-acetic acid,
3-oxo-8-[2-(1,2,3,4-tetrahydroimidazo[1,2-b][1,2,4]triazin-6-yl)ethoxy]-2-trifluoromethyl-2,3,4,5-tetrahydro-1H-2-benzazepin-4-acetic acid,
(4S)-3-oxo-8-[2-(1,2,3,4-tetrahydroimidazo[1,2-b][1,2,4]triazin-6-yl)ethoxy]-2-trifluoromethyl-2,3,4,5-tetrahydro-1H-2-benzazepin-4-acetic acid,
3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetic acid or
(4S)-3-oxo-8-[2-(1,2,3,4-tetrahydroimidazo[1,2-b][1,2,4]triazin-6-yl)ethoxy]-2-(2,2,2 trifluoroethyl)-2,3,4,5-tetrahydro-1H-2-benzazepin-4-acetic acid,
   or compounds in which the carboxyl group thereof is protected by a protective group (said protective group is a methyl group, ethyl group, propyl group, isopropyl group, 1-ethylpropyl group, butyl group, 3,3-dimethylbutyl group, pentyl group, hexyl group, heptyl group, octyl group, nonyl group, decyl group, undecyl group, dodecyl group, benzyl group, phenethyl group, phenylpropyl group, phenylbutyl group, phenylpentyl group, phenylhexyl group, phenylheptyl group, phenyloctyl group, phenylnonyl group, phenyldecyl group, phenylundecyl group, phenyldodecyl group, acetoxymethyl group, pivaloyloxymethyl group, 1-pivaloyloxyethyl group, N,N-dimethylaminocarbonyl-methyl group, 2-(morpholin-4-yl)ethyl group or (5-methyl-2-oxo-1,3-dioxolen-4-yl)-methyl group.),

and particularly preferably compounds of
3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetic acid,
(4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetic acid,
ethyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
ethyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
propyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
propyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
isopropyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2 -benzazepin-4-acetate,
isopropyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
heptyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
heptyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
undecyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
undecyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
benzyl 3-oxo-8-[2-(5,6,7,8-tetrahydroimidazo[1,2-a]pyrimidin-2-yl)ethoxy]-2-(2,2,2-trifluoroethyl)-2,3,4, 5-tetrahydro-1H-2-benzazepin-4-acetate,
benzyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
(10-phenyl)decyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
(10-phenyl)decyl (4S)-3-oxo-8-[2-(5,6,7,8-tetrahydroimidazo[1,2-a]pyrimidin-2-yl)-ethoxy]-2-(2,2,2-trifluoroethyl)-2,3,4,5-tetrahydro-1H-2-benzazepin-4-acetate,
pivaloyloxymethyl 3-oxo-8-[2-(5,6,7,8-tetrahydroimidazo[1,2-a]pyrimidin-2-yl)-ethoxy]-2-(2,2,2-trifluoroethyl)-2,3,4,5-tetrahydro-1H-2-benzazepin-4-acetate,
pivaloyloxymethyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
(N,N-dimethylaminocarbonyl)methyl 3-oxo-8-[2-(5,6,7,8-tetrahydroimidazo[1,2-a]-pyrimidin-2-yl)ethoxy]-2-(2,2,2-trifluoroethyl)-2,3,4,5-tetrahydro-1H-2-benzazepin-4-acetate,
(N,N-dimethylaminocarbonyl)methyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
[2-(morpholin-4-yl)]ethyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
[2-(morpholin-4-yl)]ethyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
(5-methyl-2-oxo-[1,3]dioxolen-4-yl)methyl 3-oxo-8-[2-(5,6,7,8-tetrahydroimidazo-[1,2-a]pyrimidin-2-yl)ethoxy]-2-(2,2,2-trifluoroethyl)-2,3,4,5-tetrahydro-1H-2-benzazepin-4-acetate,
(5-methyl-2-oxo-[1,3]dioxolen-4-yl)methyl (4S)-3-oxo-8-[2-(5,6,7,8-tetrahydro-imidazo[1,2-a]pyrimidin-2-yl)ethoxy]-2-(2,2,2-trifluoroethyl)-2,3,4,5-tetrahydro- H-2-benzazepin-4-acetate,
3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetic acid,
(4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetic acid,
ethyl 3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
ethyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
propyl 3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
propyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
isopropyl 3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
isopropyl (4S)-3-oxo-8-[2-(1,2,3,4-tetrahydroimidazo[1,2-b][1,2,4]triazin-6-yl)-ethoxy]-2-(2,2,2-trifluoroethyl)-2,3,4,5-tetrahydro-1H-2-benzazepin-4-acetate,
heptyl 3-oxo-8-[2-(1,2,3,4-tetrahydroimidazo[1,2-b][1,2,4]triazin-6-yl)ethoxy]-2-(2,2,2-trifluoroethyl)-2,3 ,4,5-tetrahydro-1H-2-benzazepin-4-acetate,
heptyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
undecyl 3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
undecyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
benzyl 3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
benzyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
(10-phenyl)decyl 3-oxo-8-[2-(1,2,3,4-tetrahydroimidazo[1,2-b][1,2,4]triazin-6-yl)-ethoxy]-2-(2,2,2-trifluoroethyl)-2,3,4,5-tetrahydro-1H-2-benzazepin-4-acetate,
(10-phenyl)decyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
pivaloyloxymethyl 3-oxo-8-[2-(1,2,3,4-tetrahydroimidazo[1,2-b][1,2,4]triazin-6-yl)-ethoxy]-2-(2,2,2-trifluoroethyl)-2,3,4,5-tetrahydro-1H-2-benzazepin-4-acetate,
pivaloyloxymethyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate, (N,N-dimethylaminocarbonyl)methyl 3-oxo-8-[2-(1,2,3,4-tetrahydroimidazo[1,2-b]-[1,2,4]triazin-6-yl)ethoxy]-2-(2,2,2-trifluoroethyl)-2,3,4,5-tetrahydro-1H-2-benzazepin-4-acetate,
(N,N-dimethylaminocarbonyl)methyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
[2-(morpholin-4-yl)]ethyl 3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
[2-(morpholin-4-yl)]ethyl (4S)-3-oxo-8-[2-(1,2,3,4-tetrahydroimidazo[1,2-b][1,2,4]-triazin-6-yl)ethoxy]-2-(2,2,2-trifluoroethyl)-2,3,4,5-tetrahydro-1H-2-benzazepin-4-acetate,
(5-methyl-2-oxo-[1,3]dioxolen-4-yl)methyl 3-oxo-8-[2-(1,2,3,4-tetrahydroimidazo-[1,2-b][1,2,4]triazin-6-yl)ethoxy]-2-(2,2,2-trifluoroethyl)-2,3,4,5-tetrahydro-1H-2-benzazepin-4-acetate, or
(5-methyl-2-oxo-[1,3]dioxolen-4-yl)methyl (4S)-3-oxo-8-[2-(1,2,3,4-tetrahydro-imidazo[1,2-b][1,2,4]triazin-6-yl)ethoxy]-2-(2,2,2-trifluoroethyl)-2,3,4,5-tetrahydro-1H-2-benzazepin-4-acetate, etc.

The compound having the formula (I) of the present invention can be prepared, for example, by the following methods.

### [Method 1]

"Method 1" is a method for generally preparing Compound (Ia) of the present invention, wherein R² is a carboxyl group in the formula (I).

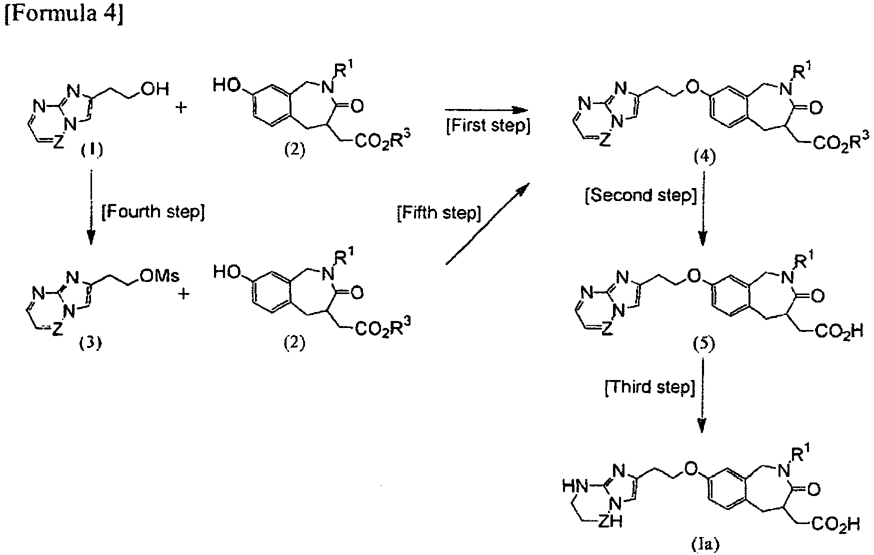

wherein R¹ and Z have the same meanings as defined above, R³ has the same meaning as the protective group for the carboxyl group which may be protected shown by R², and Ms represents a methanesulfonyl group.

The first step is a step of preparing Compound (4) by reacting Compound (1) and Compound (2) using triphenylphosphine and a condensing agent in an organic solvent.
Compound (1) can be prepared according to the method as described in, for example, WO 97/25325A, and Compound (2) can be prepared according to the method as described in, for example, WO 98/14192A.
An amount of Compound (1) to be used is generally 1 to 10-fold mol, preferably 1 to 3-fold mol based on Compound (2).
The condensing agent to be used may be mentioned diethyl azodicarboxylate, diisopropyl azodicarboxylate, N,N,N',N'-tetramethylazodicarboxyamide or 1,1'-(azodicarbonyl)dipiperidine, etc., preferably diisopropyl azodicarboxylate.
An amount of the triphenylphosphine to be used is generally 1 to 5-fold mol, preferably 1 to 3-fold mol based on Compound (2).
An amount of the condensing agent to be used is generally 1 to 5-fold mol, preferably 1 to 3-fold mol based on Compound (2).
The organic solvent to be used is not particularly limited so long as it does not inhibit the reaction and dissolves the starting materials with a certain extent, and there may be mentioned, for example, ethers such as tetrahydrofuran, diethyl ether, diisopropyl ether, ehyleneglycol dimethyl ether, 1,4-dioxane and diglyme, etc.; aromatic hydrocarbons such as benzene, toluene, xylene, mesitylene and nitrobenzene, etc.; amides such as N,N-dimethylformamide, N,N-dimethylacetamide, N-methyl-2-pyrrolidone and hexamethyl phosphorylamide, etc.; sulfoxides such as dimethylsulfoxide, etc.; or a mixed solvent thereof, preferably ethers or amides, and particularly preferably tetrahydrofuran or N,N-dimethylformamide.
The reaction temperature may vary depending on the starting materials, reagent(s) or solvent(s), etc., and is generally -50°C to 150°C, preferably -20°C to 100°C.
The reaction time may vary depending on a reaction temperature, and is generally 1 hour to 48 hours, preferably 1 hour to 24 hours.

The second step is a step of preparing Compound (5) by hydrolyzing Compound (4) in water or a mixed solvent of water and an organic solvent, by using a base.
The base to be used may be mentioned, for example, inorganic hydroxides such as lithium hydroxide, sodium hydroxide, potassium hydroxide and calcium hydroxide, etc., and preferably lithium hydroxide or sodium hydroxide.
An amount of the base to be used is generally 1 to 100-fold mol, preferably 1 to 50-fold mol based on Compound (4).
The solvent to be used may be mentioned, for example, water or a mixed solvent of water and an organic solvent(s) (for example, there may be mentioned alcohols such as methanol, ethanol, propanol, butanol and ethylene glycol, etc.; ethers such as tetrahydrofuran, diethyl ether, diisopropyl ether, 1,4-dioxane and diglyme, etc.; amides such as N,N-dimethylformamide, N,N-dimethylacetamide and N-methyl-2-pyrrolidone, etc.; or sulfoxides such as dimethylsulfoxide, etc. or a mixed solvent thereof, preferably alcohols or ethers).
The reaction temperature may vary depending on the starting materials, reagent(s) or solvent(s), etc., and is generally -50°C to 150°C, preferably -20°C to 100°C.
The reaction time may vary depending on a reaction temperature, and is generally 1 hour to 24 hours, preferably 1 hour to 12 hours.

The third step is a step of preparing Compound (Ia) of the present invention by carrying out a reaction of Compound (5) in an organic solvent using a reducing agent.
The reducing agent to be used may be mentioned hydrides such as sodium borohydride, lithium borohydride, sodium cyanoborohydride, sodium trimethoxyborohydride or lithium aluminum hydride, etc., or metal palladiums such as palladium-active carbon or palladium black, etc.; or metal platinums such as platinum-carbon, etc. in an organic solvent containing hydrogen chloride under hydrogen gas atmosphere" and preferably sodium borohydride, or palladium-active carbon under hydrogen gas atmosphere.
An amount of the reducing agent to be used is generally 1 to 100-fold mol, preferably 1 to 50-fold mol based on Compound (5) when the hydride is used, and generally 0.01 to 10-fold weight, preferably 0.05 to 5-fold weight when metal palladiums or metal platinums are used under hydrogen gas atmosphere.
The organic solvent to be used is not particularly limited so long as it does not inhibit the reaction and dissolves the starting materials with a certain extent, and there may be mentioned, for example, alcohols such as methanol, ethanol, propanol, butanol and ethylene glycol, etc.; ethers such as tetrahydrofuran, diethyl ether, diisopropyl ether, ehyleneglycol dimethyl ether, 1,4-dioxane and diglyme, etc.; or a mixed solvent thereof, preferably methanol, ethanol or tetrahydrofuran.
The reaction temperature may vary depending on the starting materials, reagent(s) or solvent(s), etc., and is generally 0°C to 150°C, preferably 0°C to 50°C.
The reaction time may vary depending on a reaction temperature, and is generally 1 hour to 24 hours, preferably 1 hour to 12 hours.

The fourth step and the fifth step are separate steps for preparing Compound (4).
The fourth step is a step for preparing Compound (3) by treating Compound (1) and methanesulfonyl chloride with a base in an organic solvent.
The base to be used may be mentioned, for example, amines such as triethylamine, pyridine, 1,8-diazabicyclo[5.4.0]-7-undecene and diisopropylethylamine, etc.;
inorganic bases such as sodium hydride, potassium hydride, potassium carbonate, sodium carbonate, sodium hydrogen carbonate, potassium hydroxide and sodium hydroxide, etc.; or organic bases such as methyl lithium, butyl lithium, lithium diisopropylamide, lithium bistrimethylsilylamide, sodium bistrimethylsilylamide, sodium methoxide, sodium ethoxide and potassium tert-butoxide, etc., preferably triethylamine. An amount of the base to be used is generally 1 to 100-fold mol, preferably 1 to 50-fold mol based on Compound (1).
An amount of the methanesulfonyl chloride to be used is generally 1 to 50-fold mol, preferably 1 to 10-fold mol based on Compound (1).
The organic solvent to be used is not particularly limited so long as it does not inhibit the reaction and dissolves the starting materials with a certain extent, and there may be mentioned, for example, halogenated hydrocarbons such as dichloromethane, chloroform and dichloroethane, etc.; ethers such as tetrahydrofuran, diethyl ether, diisopropyl ether, ehyleneglycol dimethyl ether, 1,4-dioxane and diglyme, etc.; or a mixed solvent thereof, preferably halogenated hydrocarbons.
The reaction temperature may vary depending on the starting materials, reagent(s) or solvent(s), etc., and is generally -50°C to 100°C, preferably -20°C to 50°C.
The reaction time may vary depending on a reaction temperature, and is generally 1 hour to 24 hours, preferably 1 hour to 12 hours.

The fifth step is a step for preparing Compound (4) by treating Compound (3) and Compound (2) with a base in an organic solvent.
The base to be used may be mentioned, for example, amines such as triethylamine, pyridine, 1,8-diazabicyclo[5.4.0]-7-undecene and diisopropylethylamine, etc.; inorganic bases such as sodium hydride, potassium hydride, potassium carbonate, sodium carbonate, sodium hydrogen carbonate, potassium hydroxide and sodium hydroxide, etc.; or organic bases such as methyl lithium, butyl lithium, lithium diisopropylamide, lithium bistrimethylsilylamide, sodium bistrimethylsilylamide, sodium methoxide, sodium ethoxide and potassium tert-butoxide, etc., preferably sodium hydride. An amount of the base to be used is generally 1 to 10-fold mol, preferably I to 5-fold mol based on Compound (2).
The organic solvent to be used is not particularly limited so long as it does not inhibit the reaction and dissolves the starting materials with a certain extent, and there may be mentioned, for example, ethers such as tetrahydrofuran, diethyl ether, diisopropyl ether, 1,4-dioxane and diglyme, etc.; amides such as N,N-dimethylformamide, N,N-dimethylacetamide and N-methyl-2-pyrrolidone, etc.; sulfoxides such as dimethylsulfoxide, etc.; or a mixed solvent thereof, preferably ethers, and particularly preferably tetrahydrofuran.
The reaction temperature may vary depending on the starting materials, reagent(s) or solvent(s), etc., and is generally -50°C to 150°C, preferably -20°C to 100°C.
The reaction time may vary depending on a reaction temperature, and is generally 1 hour to 48 hours, preferably 1 hour to 24 hours.

### [Method 2]

"Method 2" is another method for preparing Compound (Ia) of the present invention wherein, in the formula (I), Compound (Ib) of the present invention where R² is a protected carboxyl group, and R² is a carboxyl group.

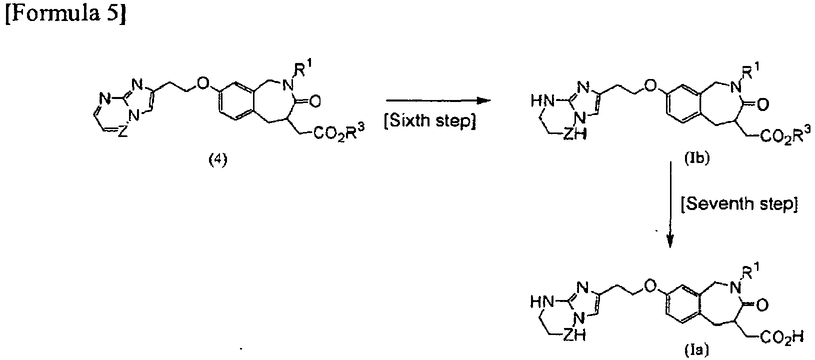

wherein R¹, R³ and Z have the same meanings as defined above.

The sixth step is a step of preparing Compound (Ib) of the present invention by reducing Compound (4) in an organic solvent. This step is carried out according to the above-mentioned "third step" except for using Compound (4) in place of Compound (5).

The seventh step is a step for preparing Compound (Ia) of the present invention by hydrolyzing Compound (Ib) using a base in an organic solvent. This step is carried out according to the above-mentioned "second step" except for using Compound (Ib) in place of Compound (4).

### [Method 3]

"Method 3" is another method for preparing Compound (Ia) of the present invention wherein, in the formula (I), Compound (Ib) of the present invention where R² is a protected carboxyl group, and R² is a carboxyl group.

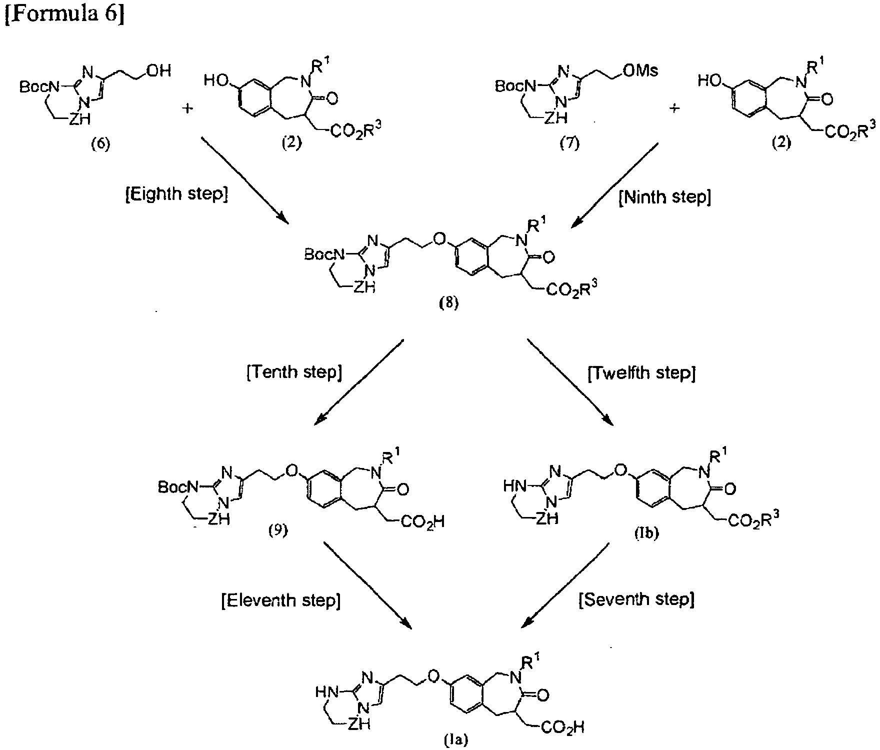

wherein R¹, R³, Z and Ms have the same meanings as defined above, and Boc represents tert-butoxycarbonyl group.

The eighth step is a step of preparing Compound (8) by condensing Compound (6) and Compound (2) in an organic solvent. This step is carried out according to the above-mentioned "first step" except for using Compound (6) in place of Compound (1). Compound (6) can be prepared by the below mentioned "Method 5".

The ninth step is a step for preparing Compound (8) by treating Compound (7) and Compound (2) with a base in an organic solvent. This step is carried out according to the above-mentioned "fifth step" except for using Compound (7) in place of Compound (3). Compound (7) can be prepared by the below mentioned "Method 5".

The tenth step is a step of preparing Compound (9) from Compound (8) in water or a mixed solvent of water and an organic solvent. This step is carried out according to the above-mentioned "second step" except for using Compound (8) in place of Compound (4).

The eleventh step is a step of preparing Compound (Ia) of the present invention by treating Compound (9) with an acidin an organic solvent.
The acid to be used may be mentioned, for example, inorganic acids such as hydrochloric acid, hydrobromic acid, hydroiodic acid, sulfuric acid and nitric acid, etc.; aliphatic carboxylic acids such as formic acid, acetic acid, propionic acid and butyric acid, etc.; or organic sulfonic acids such as methanesulfonic acid, benzenesulfonic acid, p-toluenesulfonic acid, naphthalenesulfonic acid and camphorsulfonic acid, etc., preferably inorganic acids or aliphatic carboxylic acids. An amount of the acid to be used is generally 1 to 100-fold mol, preferably 1 to 50-fold mol based on Compound (9).
The organic solvent to be used is not particularly limited so long as it does not inhibit the reaction and dissolves the starting materials with a certain extent, and there may be mentioned, for example, alcohols such as methanol, ethanol, propanol, butanol and ethylene glycol, etc.; ethers such as tetrahydrofuran, diethyl ether, diisopropyl ether, 1,4-dioxane and diglyme, etc.; amides such as N,N-dimethylformamide, N,N-dimethylacetamide and N-methyl-2-pyrrolidone, etc.; sulfoxides such as dimethylsulfoxide, etc.; or a mixed solvent thereof, preferably alcohols.
The reaction temperature may vary depending on the starting materials, reagent(s) or solvent(s), etc., and is generally 0°C to 150°C, preferably 0°C to 100°C.
The reaction time may vary depending on a reaction temperature, and is generally 1 hour to 72 hours, preferably 1 hour to 48 hours.
This step may be carried out in the presence of an inert gas. The inert gas to be used may be mentioned, for example, nitrogen, helium, or argon, etc.

The twelfth step is a step of preparing Compound (Ib) of the present invention by treating Compound (8) with an acid in an organic solvent. This step is carried out according to the above-mentioned "eleventh step" except for using Compound (8) in place of Compound (9).

By using Compound (Ib) of the present invention obtained in the twelfth step, and via the above-mentioned "seventh step", Compound (Ia) of the present invention can be prepared.

### [Method 4]

"Method 4" is another method for preparing Compound (Ib) of the present invention wherein R² is a protected carboxyl group in the formula (I).

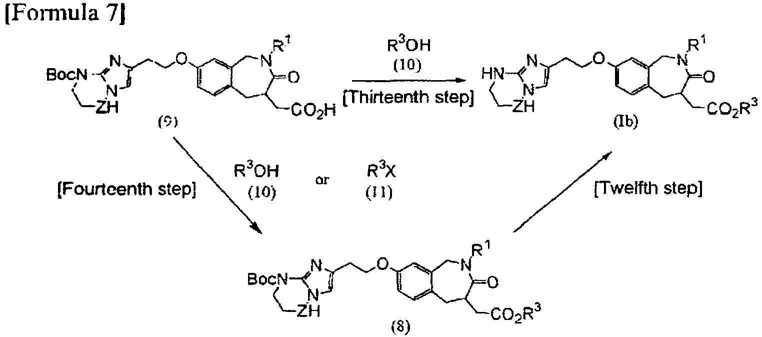

wherein R¹, R³, Z and Boc have the same meanings as defined above, and X represents a chlorine atom, bromine atom or iodine atom.

The thirteenth step is a step of preparing Compound (Ib) by treating Compound (9) and Compound (10) with an acid without aolvent or in an organic solvent.
Compound (10) is a known compound or can be prepared from a known compound by a known method.
An amount of Compound (10) to be used is generally 1 to 50-fold mol, preferably 1 to 10-fold mol based on Compound (9), and it may be used with a far excess amount as a solvent.
The acid to be used may be mentioned, inorganic acids such as hydrochloric acid, hydrobromic acid, hydroiodic acid, sulfuric acid and nitric acid, etc.; or organic sulfonic acids such as methanesulfonic acid, benzenesulfonic acid, p-toluenesulfonic acid, naphthalenesulfonic acid and camphorsulfonic acid, etc., preferably inorganic acids. An amount of the acid to be used is generally 1 to 100-fold mol, preferably 1 to 50-fold mol based on Compound (9).
The organic solvent to be used is not particularly limited so long as it does not inhibit the reaction and dissolves the starting materials with a certain extent, and there may be mentioned, for example, ethers such as tetrahydrofuran, diethyl ether, diisopropyl ether, 1,4-dioxane and diglyme, etc.; amides such as N,N-dimethylformamide, N,N-dimethylacetamide and N-methyl-2-pyrrolidone, etc.; sulfoxides such as dimethylsulfoxide, etc.; or a mixed solvent thereof, preferably ethers.
The reaction temperature may vary depending on the starting materials, reagent(s) or solvent(s), etc., and is generally 0°C to 150°C, preferably 0°C to 100°C.
The reaction time may vary depending on a reaction temperature, and is generally 1 hour to 24 hours, preferably 1 hour to 12 hours.
This step may be carried out in the presence of an inert gas. The inert gas to be used may be mentioned, for example, nitrogen, helium, or argon, etc.

The fourteenth step is a step of preparing Compound (8) by reacting Compound (9) with Compound (10) or Compound (11) in an organic solvent.
Compound (11) is a known compound or can be prepared from a known compound by a known method.
When Compound (10) is used, the reaction is carried out by using a condensing agent and an additive in an organic solvent, and when Compound (11) is used, it can be carried out by using a base in an organic solvent.
When it is reacted with Compound (10), the condensing agent to be used may be mentioned dicyclohexylcarbodiimide, diisopropylcarbodiimide, carbonyldiimidazole, N,N'-dicyclohexylcarbodiimide, 1-ethyl-3-(3-dimethylaminopropyl)carbodiimide hydrochloride, 1-[bis(dimethylamino)methylene]-1H-benzotriazolium-3-oxide hexafluorophosphate, etc., preferably 1-ethyl-3-(3-dimethylaminopropyl)carbodiimide hydrochloride or 1-[bis(dimethylamino)methylene]-1H-benzotriazolium-3-oxide hexafluorophosphate. An amount of the condensing agent to be used is generally 1 to 10-fold mol, preferably 1 to 3-fold mol based on Compound (9).
The additive may be mentioned 1-hydroxybenzotriazole or 4-dimethylaminopyridine. An amount of the additive to be used is generally 0.01 to 10-fold mol, preferably 0.1 to 3-fold mol based on Compound (9).
An amount of Compound (10) to be used is generally 1 to 50-fold mol, preferably 1 to 10-fold mol based on Compound (9).
The organic solvent to be used is not particularly limited so long as it does not inhibit the reaction and dissolves the starting materials with a certain extent, and there may be mentioned, for example, halogenated hydrocarbons such as dichloromethane, chloroform and dichloroethane, etc.; ethers such as tetrahydrofuran, diethyl ether, diisopropyl ether, ehyleneglycol dimethyl ether, 1,4-dioxane and diglyme, etc.; amides such as N,N-dimethylformamide, N,N-dimethylacetamide and N-methyl-2-pyrrolidone, etc.; sulfoxides such as dimethylsulfoxide, etc.; or a mixed solvent thereof, preferably halogenated hydrocarbons, ethers, or amides, more preferably dichloromethane, tetrahydrofuran or N,N-dimethylformamide.
The reaction temperature may vary depending on the starting materials, reagent(s) or solvent(s), etc., and is generally 0°C to 150°C, preferably 0°C to 100°C.
The reaction time may vary depending on a reaction temperature, and is generally 1 hour to 24 hours, preferably 1 hour to 12 hours.
The present reaction may be carried out in the presence of an inert gas. The inert gas to be used may be mentioned, for example, nitrogen, helium, or argon, etc.
When Compound (11) is to be used, the base to be used may be mentioned, for example, amines such as triethylamine, pyridine, 1,8-diazabicyclo[5.4.0]-7-undecene and diisopropylethylamine, etc.; inorganic bases such as sodium hydride, potassium hydride, potassium carbonate, sodium carbonate, sodium hydrogen carbonate, potassium hydroxide and sodium hydroxide, etc.; or organic bases such as methyl lithium, butyl lithium, lithium diisopropylamide, lithium bistrimethylsilylamide, sodium bistrimethylsilylamide, sodium methoxide, sodium ethoxide and potassium tert-butoxide, etc., preferably inorganic bases, more preferably potassium carbonate. An amount of the base to be used is generally 1 to 10-fold mol, preferably 1 to 5-fold mol based on Compound (9).
An amount of Compound (11) to be used is generally 1 to 10-fold mol, preferably 1 to 5-fold mol based on Compound (9).
The organic solvent to be used is not particularly limited so long as it does not inhibit the reaction and dissolves the starting materials with a certain extent, and there may be mentioned, for example, halogenated hydrocarbons such as dichloromethane, chloroform and dichloroethane, etc.; ethers such as tetrahydrofuran, diethyl ether, diisopropyl ether, ehyleneglycol dimethyl ether, 1,4-dioxane and diglyme, etc.; alcohols such as methanol, ethanol, propanol, butanol and ethylene glycol, etc.; amides such as N,N-dimethylformamide, N,N-dimethylacetamide and N-methyl-2-pyrrolidone, etc.; sulfoxides such as dimethylsulfoxide, etc.; nitriles such as acetonitrile, etc.; or a mixed solvent thereof, preferably halogenated hydrocarbons, amides or nitriles, more preferably N,N-dimethylformamide.
The reaction temperature may vary depending on the starting materials, reagent(s) or solvent(s), etc., and is generally 0°C to 150°C, preferably 0°C to 100°C.
The reaction time may vary depending on a reaction temperature, and is generally 1 hour to 48 hours, preferably 1 hour to 24 hours.
The present reaction may be carried out in the presence of an inert gas. The inert gas to be used may be mentioned, for example, nitrogen, helium, or argon, etc.

### [Method 5]

"Method 5" is a method for preparing Compound (1) in "Method 1", and Compound (6) and Compound (7) in "Method 3".

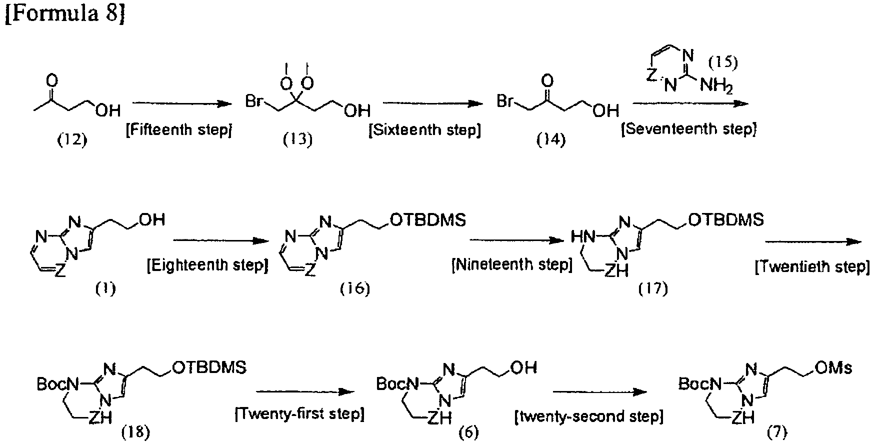

wherein Z, Boc, and Ms have the same meanings as defined above, and TBDMS represents a tert-butyldimethylsilyl group.

The fifteenth step is a step of preparing Compound (13) by reacting Compound (12) with a brominating agent in methanol.
The brominating agent to be used may be mentioned, for example, bromine, hydrogen bromide or tetrabutyl ammonium tribromide, etc., preferably bromine. An amount of the brominating agent to be used is generally 1 to 10-fold mol, preferably 1 to 5-fold mol based on Compound (12).
The reaction temperature may vary depending on the starting materials, reagent(s) or solvent(s), etc., and is generally -50°C to 150°C, preferably -20°C to 100°C.
The reaction time may vary depending on a reaction temperature, and is generally 1 hour to 48 hours, preferably 1 hour to 24 hours.

The sixteenth step is a step of preparing Compound (14) by treating Compound (13) with an acid in an organic solvent.
The acid to be used may be mentioned, for example, inorganic acids such as hydrochloric acid, hydrobromic acid, hydroiodic acid, sulfuric acid and nitric acid, etc.; or organic sulfonic acids such as methanesulfonic acid, benzenesulfonic acid, p-toluenesulfonic acid, naphthalenesulfonic acid and camphorsulfonic acid, etc., preferably inorganic acids, and particularly preferably sulfuric acid. An amount of the acid to be used is generally 1 to 50-fold mol, preferably 1 to 20-fold mol based on Compound (13).
The organic solvent to be used is not particularly limited so long as it does not inhibit the reaction and dissolves the starting materials with a certain extent, and there may be mentioned, for example, alcohols such as methanol, ethanol, propanol, butanol and ethylene glycol, etc.; ethers such as tetrahydrofuran, diethyl ether, diisopropyl ether, 1,4-dioxane and diglyme, etc.; amides such as N,N-dimethylformamide, N,N-dimethylacetamide and N-methyl-2-pyrrolidone, etc.; sulfoxides such as dimethylsulfoxide, etc.; or a mixed solvent thereof, preferably alcohols.
The reaction temperature may vary depending on the starting materials, reagent(s) or solvent(s), etc., and is generally 0°C to 150°C, preferably 0°C to 100°C.
The reaction time may vary depending on a reaction temperature, and is generally 1 hour to 72 hours, preferably 1 hour to 48 hours.

The seventeenth step is a step of preparing Compound (1) by condensing Compound (14) and Compound (15) in an organic solvent.
Compound (15) is a known compound or can be prepared from a known compound by a known method. An amount of Compound (15) to be used is generally 1 to 10-fold mol, preferably 1 to 5-fold mol based on Compound (14).
The organic solvent to be used is not particularly limited so long as it does not inhibit the reaction and dissolves the starting materials with a certain extent, and there may be mentioned, for example, alcohols such as methanol, ethanol, propanol, butanol and ethylene glycol, etc.; ethers such as tetrahydrofuran, diethyl ether, diisopropyl ether, 1,4-dioxane and diglyme, etc.; amides such as N,N-dimethylformamide, N,N-dimethylacetamide and N-methyl-2-pyrrolidone, etc.; sulfoxides such as dimethylsulfoxide, etc.; or a mixed solvent thereof, preferably alcohols.
The reaction temperature may vary depending on the starting materials, reagent(s) or solvent(s), etc., and is generally 0°C to 150°C, preferably 0°C to 100°C.
The reaction time may vary depending on a reaction temperature, and is generally 1 hour to 48 hours, preferably I hour to 24 hours.

The eighteenth step is a step of preparing Compound (16) by treating Compound (1) with imidazole and tert-butyldimethylsilyl chloride in an organic solvent.
An amount of the imidazole to be used is generally 1 to 50-fold mol, preferably 1 to 10-fold mol based on Compound (1).
An amount of the tert-butyldimethylsilyl chloride to be used is generally 1 to 50-fold mol, preferably 1 to 10-fold mol based on Compound (1).
The organic solvent to be used is not particularly limited so long as it does not inhibit the reaction and dissolves the starting materials with a certain extent, and there may be mentioned, for example, halogenated hydrocarbons such as dichloromethane, chloroform and dichloroethane, etc.; ethers such as tetrahydrofuran, diethyl ether, diisopropyl ether, ehyleneglycol dimethyl ether, 1,4-dioxane and diglyme, etc.; amides such as N,N-dimethylformamide, N,N-dimethylacetamide and N-methyl-2-pyrrolidone, etc.; sulfoxides such as dimethylsulfoxide, etc.; or a mixed solvent thereof, preferably amides.
The reaction temperature may vary depending on the starting materials, reagent(s) or solvent(s), etc., and is generally 0°C to 100°C, preferably 0°C to 50°C.
The reaction time may vary depending on a reaction temperature, and is generally 1 hour to 24 hours, preferably 1 hour to 12 hours.

The nineteenth step is a step of preparing Compound (17) by treating Compound (16) with a reducing agent in an organic solvent. This step is carried out according to the above-mentioned "third step" except for using Compound (16) in place of Compound (5).

The twentieth step is a step of preparing Compound (18) by treating Compound (17) with di-tert-butyl dicarbonate and 4-dimethylaminopyridine in the presence of a base in an organic solvent.
The base to be used may be mentioned, for example, amines such as triethylamine, pyridine, 1,8-diazabicyclo[5.4.0]-7-undecene and diisopropylethylamine, etc.; inorganic bases such as sodium hydride, potassium hydride, potassium carbonate, sodium carbonate, sodium hydrogen carbonate, potassium hydroxide and sodium hydroxide, etc.; or organic bases such as methyl lithium, butyl lithium, lithium diisopropylamide, lithium bistrimethylsilylamide, sodium bistrimethylsilylamide, sodium methoxide, sodium ethoxide and potassium tert-butoxide, etc., preferably amines. An amount of the base to be used is generally 1 to 10-fold mol, preferably 1 to 5-fold mol based on Compound (17).
An amount of the di-tert-butyl dicarbonate to be used is generally 1 to 50-fold mol, preferably 1 to 10-fold mol based on Compound (17).
An amount of the 4-dimethylaminopyridine to be used is generally 0.1 to 5-fold mol, preferably 0.1 to 1-fold mol based on Compound (17).
The organic solvent to be used is not particularly limited so long as it does not inhibit the reaction and dissolves the starting materials with a certain extent, and there may be mentioned, for example, halogenated hydrocarbons such as dichloromethane, chloroform and dichloroethane, etc.; ethers such as tetrahydrofuran, diethyl ether, diisopropyl ether, ehyleneglycol dimethyl ether, 1,4-dioxane and diglyme, etc.; or a mixed solvent thereof, preferably halogenated hydrocarbons.
The reaction temperature may vary depending on the starting materials, reagent(s) or solvent(s), etc., and is generally 0°C to 100°C, preferably 0°C to 50°C.
The reaction time may vary depending on a reaction temperature, and is generally 1 hour to 48 hours, preferably 1 hour to 24 hours.

The twenty-first step is a step of preparing Compound (6) by treating Compound (18) with an acid in an organic solvent.
The acid to be used may be mentioned, for example, inorganic acids such as hydrochloric acid, hydrobromic acid, hydroiodic acid, sulfuric acid and nitric acid, etc.; or organic sulfonic acids such as methanesulfonic acid, benzenesulfonic acid, p-toluenesulfonic acid, naphthalenesulfonic acid and camphorsulfonic acid, etc., preferably organic sulfonic acids, and particularly preferably p-toluenesulfonic acid. An amount of the acid to be used is generally 1 to 10-fold mol, preferably 1 to 5-fold mol based on Compound (18).
The organic solvent to be used is not particularly limited so long as it does not inhibit the reaction and dissolves the starting materials with a certain extent, and there may be mentioned, for example, alcohols such as methanol, ethanol, propanol, butanol and ethylene glycol, etc.; ethers such as tetrahydrofuran, diethyl ether, diisopropyl ether, 1,4-dioxane and diglyme, etc.; amides such as N,N-dimethylformamide, N,N-dimethylacetamide and N-methyl-2-pyrrolidone, etc.; sulfoxides such as dimethylsulfoxide, etc.; or a mixed solvent thereof, preferably alcohols.
The reaction temperature may vary depending on the starting materials, reagent(s) or solvent(s), etc., and is generally 0°C to 100°C, preferably 0°C to 50°C.
The reaction time may vary depending on a reaction temperature, and is generally 1 hour to 24 hours, preferably 1 hour to 12 hours.

The twenty-second step is a step of preparing Compound (7) by treating Compound (6) with methanesulfonyl chloride in the presence of a base in an organic solvent. This step is carried out according to the above-mentioned "fourth step" except for using Compound (6) in place of Compound (1).

Compound (III) of the present invention can be prepared in the same manner as in Compound (I) of the present invention in accordance with the above-mentioned "Method 1" to "Method 3" except for using optical isomer ((S)-isomer) (see WO 98/14192A or WO 2004/089890A) in place of racemic Compound (2). Also, Compound (III) of the present invention which is a desired optical isomer can be obtained by optically resolving racemic compound of Compound (I) of the present invention, or optically resolving its preparation intermediate (for example, Compounds (4), (5), (8) and (9)) and derived. As a method of optical resolution, it can be carried out by optionally selecting usual methods, for example, a column chromatography method using an optically resolving column, a preferencial crystallization method, a method of resolving with a diastereomer salt.

The objective compound formed by the above-mentioned respective reactions can be obtained from the reaction mixture according to the conventional method. For example, the reaction mixture is optionally neutralized, and when insoluble materials are present, after removing the same by filtration, an organic solvent which is not miscible with water such as ethyl acetate, etc., is added, followed by washing, the organic layer containing the objective compound is separated, dried over a dryer such as anhydrous magnesium sulfate, etc., and the solvent is distilled to obtain the objective compound.
The obtained objective compound can be, if necessary, separated and purified by optionally combining the conventional methods, for example, recrystallization; reprecipitation; or general methods conventionally used for separation and purification of an organic compound (for example, an adsorption column chromatography method using a carrier such as silica gel, alumina, etc.; ion exchange chromatography method; or a normal phase-reverse phase column chromatography method by silica gel or alkylated silica gel (preferably it is high performance liquid chromatography.)), and eluting with a suitable eluent.

The benzazepinone compound represented by the formula (I) which is an effective ingredient of the present invention can be present as a hydrate or a solvate, and they are also included in the present invention.

The benzazepinone compound represented by the formula (I) which is an effective ingredient of the present invention can be converted, if necessary, into a pharmaceutically acceptable addition salt according to the conventional method, and it can be directly separated from the reaction mixture as an addition salt.
As the pharmaceutically acceptable addition salt, there may be mentioned, for example, inorganic acid addition salts such as hydrochloride, hydrobromide, hydroiodide, nitrate, sulfate or phosphate, etc.; or organic acid addition salts such as acetate, trifluoroacetate, benzoate, oxalate, malonate, succinate, maleate, fumarate, tartarate, citrate, methanesulfonate, ethanesulfonate, trifluoromethanesulfonate, benzenesulfonate, p-toluenesulfonate, glutamate or aspartate, etc., preferably hydrochloride or trifluoroacetate.

When the benzazepinone compound represented by the formula (I) which is an effective ingredient of the present invention or a pharmaceutically acceptable salt thereof is to be used as a treatment agent or prophylaxis agent of the above-mentioned eye diseases, it can be administered as such (bulk powder itself) or optionally mixing with pharmaceutically acceptable vehicles, diluents, etc., by oral administration such as a tablet, capsule, granule, powder and syrup, etc., or by parenteral administration (local administration to the eyes (ocular instillation, administration in conjunctival sac, intravitreal administration, subconjunctive administration, sub-Tenon's administration, etc.)), intravenous administration or transdermal administration, etc.) using an injection solution, ophthalmic solutions, eye ointment, patch, gel or intercalating agent, etc.

These preparations can be prepared according the conventionally known methods by using an additive(s) such as diluents (for example, sugar derivatives such as lactose, sucrose, glucose, mannitol and sorbitol, etc.; starch derivatives such as corn starch, potato starch, α-starch, dextrin or carboxymethyl starch, etc.; cellulose derivatives such as crystalline cellulose, low-substitution degree hydroxypropyl cellulose, hydroxypropylmethyl cellulose, carboxymethyl cellulose, calcium carboxymethyl cellulose and internally cross-linked sodium carboxymethyl cellulose, etc.; Gum Arabic; dextran; pulluran; silicate derivatives such as light silicic anhydride, synthetic aluminum silicate, calcium silicate and magnesium aluminate metasilicate, etc.; phosphate derivatives such as calcium hydrogen phosphate, etc.; carbonate derivatives such as calcium carbonate, etc.; or sulfate derivatives such as calcium sulfate, etc.), binders (for example, the above-mentioned vehicles; gelatin; polyvinylpyrrolidone; or polyethylene glycol, etc.), disintegrators (for example, the above-mentioned vehicles; chemically modified starch or cellulose derivatives such as croscarmellose sodium and carboxymethyl starch, etc.; or cross-linked polyvinylpyrrolidone, etc.), lubricants (for example, talc; stearic acid; metal stearate such as calcium stearate and magnesium stearate, etc.; colloidal silica; waxes such as bee gum, spermaceti, etc.; boric acid; glycol; D,L-leucine; carboxylic acids such as fumaric acid and adipic acid, etc.; sodium carboxylates such as sodium benzoate, etc.; sulfates such as sodium sulfate, etc.; lauryl sulfates such as sodium lauryl sulfate and magnesium lauryl sulfate, etc.; silicic acids such as silicic anhydride, silicic acid hydrate, etc.; or the starch derivatives in the above-mentioned vehicles, etc.), stabilizer (for example, para-oxybenzoates such as methylparaben and propylparaben, etc.; alcohols such as chlorobutanol, benzyl alcohol and phenylethyl alcohol, etc.; benzalkonium chloride; phenols such as phenol and cresol, etc.; thimerosal; acetic anhydride; or sorbic acid), corrigents (for example, generally used sweeteners, sour flavorings or aromatics, etc.), diluents (for example, water, ethanol, propylene glycol, ethoxydized isostearyl alcohol or polyoxyethylene sorbitan aliphatic acid ester, etc.), solvents for injection (for example, water, ethanol or glycerin, etc.), etc.

A dosage of the benzazepinone compound represented by the formula (I) which is an effective ingredient of the present invention or a pharmaceutically acceptable salt thereof may vary depending on the conditions such as a symptom, age, body weight of a patient, etc., and, in the case of oral administration, it can be administered with a lower limit of 0.01 mg/Kg (preferably 0.05 mg/Kg) and an upper limit of 500 mg/Kg (preferably 50 mg/Kg) per each administration, and in the case of parenteral administration, it can be administered with a lower limit of 0.001 mg/Kg (preferably 0.005 mg/Kg) and an upper limit of 50 mg/Kg (preferably 5 mg/Kg) per each administration, with 1 to 6 times per day to an adult person depending on the symptoms.

### EXAMPLES

In the following, the present invention is explained in more detail by referring to Examples to synthesize the benzazepinone compound represented by the formula (I) which is an effective ingredient of the medical composition according to the present invention and a pharmaceutically acceptable salt thereof, and Test Examples to show the effects of the compounds of the present invention, and Preparation examples, but the scope of the present invention is not limited by these.

### Example 1

Ethyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate hydrochloride

### 1-(a) 1-Bromo-4-hydroxy-2-butanone

To 1.9 L of a methanol solution containing 169 g (1.92 mol) of 4-hydroxy-2-butanone was added dropwise 300 g (1.88 mol) of bromine at -5°C over 30 minutes. After completion of the dropwise addition, a temperature of the mixture was gradually raised to room temperature, and the mixture was stirred for 2 hours. Then, 3.84 L (3.84 mol) of 2N sulfuric acid was added to the mixture at 0°C, the mixture was stirred at 10°C for 3.5 hours, and further stirred at room temperature for 22 hours.
After completion of the reaction, 325 g of sodium chloride was added to the reaction mixture, and the mixture was extracted with 5.4 L of a chloroform:methanol= 2:1 (V/V) mixed solution. The aqueous layer was further extracted with 1.7 L of a chloroform:methanol=9:1 (V/V) mixed solution four times. The combined organic layers were washed with a saturated aqueous sodium hydrogen carbonate solution, then, with a saturated aqueous sodium chloride solution, concentrated under reduced pressure to obtain 215 g (pure content: 176 g) of the title compound as pale brown oily product. (Yield: 55%)
Mass Spectrum (CI, m/z): 167 (M⁺).
¹H-NMR Spectrum (CDCl₃, δ ppm): 2.14 (t, J=6.4Hz, 1H), 2.93 (t, J=5.5Hz, 2H), 3.89-3.95 (m, 4H).

### 1-(b) 2-(Imidazo[1,2-a]pyrimidin-2-yl)ethanol

To 4.4 L of an ethanol solution containing 420 g (pure content: 351 g, 2.10 mol) of 1-bromo-4-hydroxy-2-butanone obtained by the same reaction as in Example 1-(a) was added 210 g (2.21 mol) of 2-aminopyrimidine, and the mixture was refluxed for 20 hours.
After completion of the reaction, to the reaction mixture was added 3 L of a supernatant solution of a solution obtained by adding 4 L of water to 392 g (2.84 mol) of potassium carbonate and 565 g of sodium chloride, then, the mixture was extracted with 7 L of chloroform. The aqueous layer was further extracted with 3 L of a mixed solution of chloroform:methanol=3:1 (V/V) three times. The combined organic layers were dried over anhydrous sodium sulfate, and then, concentrated under reduced pressure. The obtained residue was applied to silica gel column chromatography (eluent; ethyl acetate→ethyl acetate:ethanol:28% aqueous ammonia:water=70:25:2.5: 2.5 (V/V/V/V)), and the separated fractions containing the objective material were concentrated under reduced pressure to obtain 104 g of the title compound as pale brown solid. (Yield: 30%)
Mass Spectrum (Cl, m/z): 164 (W⁺+1).
¹H-NMR Spectrum (DMSO-d₆, δ ppm): 2.86 (td, J₁=6.8Hz, J₂=0.5Hz, 2H), 3.74 (t, J=6.8Hz, 2H), 4.68 (s, 1H), 7.00 (dd, J₁=6.7Hz, J₂=4.2Hz, 1H), 7.71 (s, 1H), 8.46 (dd, J₁=4.2Hz, J₂=2.0Hz, 1H), 8.90 (dd, J₁=6.7Hz, J₂=2.0Hz, 1H).

### 1-(c) 8-[2-(Imidazo[1,2-a]pyrimidin-2-yl)ethoxy]-3-oxo-2-(2,2,2-trifluoroethyl)-2,3,4,5-tetrahydro-1H-2-benzazepin-4-ethyl acetate

To 55 mL of a tetrahydrofuran solution containing 0.39 g (2.40 mmol) of 2-(imidazo[1,2-a]pyrimidin-2-yl)ethanol obtained in Example 1-(b) were added 0.69 g (2.00 mmol) of ethyl 8-hydroxy-3-oxo-2-(2,2,2-trifluoroethyl)-2,3,4,5-tetrahydro-1H-2-benzazepin-4-acetate (see WO 98/14192A) and 0.74 g (2.80 mmol) of triphenylphosphine, and the mixture was stirred under ice-cooling. Then, 2.6 mL of a tetrahydrofuran solution containing 0.53 g (2.60 mmol) of diisopropyl azodicarboxylate was added dropwise to the mixture over 3 minutes, and after gradually raised the temperature to room temperature, the mixture was stirred for 14 hours.
After completion of the reaction, the reaction mixture was concentrated under reduced pressure, and the residue was applied to silica gel column chromatography (eluent; chloroform:tetrahydrofuran:methanol=18:1:1 (V/V/V)). The separated fractions containing the objective material were concentrated under reduced pressure to obtain 0.31 g of the title compound as colorless oil. (Yield: 32%)
Mass Spectrum (CI, m/z): 491(M⁺+1).
¹H-NMR Spectrum (CDCl₃, δ ppm): 1.26 (t, J=7.1Hz, 3H), 2.44 (dd, J₁=16.8Hz, J₂=5.4Hz, 1H), 2.83-3.03 (m, 3H), 3.30 (t, J=6.6Hz, 2H), 3.75-3.92 (m, 1H), 3.92-4.03 (m, 1H), 3.96 (d, J=16.6Hz, 1H), 4.10-4.21 (m, 1H), 4.16 (q, J=7.1Hz, 2H), 4.39 (td, J₁=6.6Hz, J₂=1.3Hz, 2H), 5.32 (d, J=16.6Hz, 1H), 6.65 (d, J=2.7Hz, 1H), 6.80-6.85 (m, 2H), 7.01 (d, J=8.5Hz, 1H), 7.43 (s, 1H), 8.36 (dd, J₁ =6.7Hz, J₂=2.1Hz, 1H), 8.51 (dd, J₁=4.0Hz, J₂=2.1Hz,1H).

### 1-(d) Ethyl 3-oxo-8-[2-(5,6,7,8-tetrahydxoimidazo[1,2-a]pyrimidin-2-yl)ethoxy]-2-(2,2,2-trifluoroethyl)-2,3,4,5-tetrahydro-1H-2-benzazepin-4-acetate hydrochloride

To 11 mL of an ethanol solution containing 0.28 g (0.57 mmol) of ethyl 8-[2-(imidazo [1,2-a]pyrimidin-2-yl)ethoxy]-3-oxo-2-(2,2,2-trifluoroethyl)-2,3,4,5-tetrahydro-1H-2-benzazepin-4-acetate obtained in Example 1-(c) were added 5.5 mL of 3.2M hydrogen chloride/ethanol solution and 0.14 g of 10% palladium carbon, and the mixture was stirred under hydrogen atmosphere at room temperature for 1 hour.
After completion of the reaction, the reaction mixture was filtered by Celite (Tradename), the residue was washed with ethanol, and the obtained filtrate was concentrated under reduced pressure to obtain 0.28 g of the title compound as pale yellow foam. (Yield: 93%)
Mass Spectrum (FAB, m/z): 495 (M⁺+1).
¹H-NMR Spectrum (DMSO-d₆,δppm): 1.17 (t, J=7.1Hz, 3H), 1.96-2.00 (m, 2H), 2.63-2.76 (m, 2H), 2.89 (t, J=6.3Hz, 2H), 3.01 (dd, J₁=17.0Hz, J₂=4.0Hz, 1H), 3.78-3.89 (m, 1H), 3.87 (t, J=5.7Hz, 2H), 4.04 (q, J=7.1Hz, 2H), 4.11-4.29 (m, 5H), 5.31 (d, J=16.4Hz, 1H), 6.80-6.85 (m, 3H), 7.04 (d, J=9.3Hz, 1H), 8.23 (brs, 1H), 12.29 (brs, 1H).

### Example 2

### 3-Oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate trifluoroacetate

0.054 g (0.10 mmol) of ethyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate hydrochloride obtained in Example 1-(d) was dissolved in 2 mL of a mixed solution of methanol:tetrahydrofuran=1:1, 0.2 mL of water and 0.3 mL of 1N aqueous sodium hydroxide solution were added to the mixture under ice-cooling, and the mixture was stirred at room temperature for 2 hours.
After completion of the reaction, 0.025 mL of acetic acid was added to the reaction mixture, and then, the mixture was concentrated under reduced pressure. The obtained residue was purified by solid phase extraction (Cartridge; Sep-Pak C18 (5 g/20 mL (available from Waters Co.), eluent; 0.05% trifluoroacetic acid aqueous solution→acetonitrile: 0.05% trifluoroacetic acid aqueous solution=3:7 (V/V)) to obtain 31.6 mg of the title compound as pale yellow foam. (Yield: 54%)
Mass Spectrum (FAB, m/z); 467 (M⁺+1).
¹H-NMR Spectrum (DMSO-d₆, δ ppm): 1.96-2.00 (m, 2H), 2.40 (dd, J₁=17.2Hz, J₂=5.1Hz, 1H), 2.62-2.72 (m, 2H), 2.89 (t, J=6.2Hz, 2H), 3.01 (dd, J₁=17.2Hz, J₂=3.8Hz, 1H), 3.73-3.78 (m, 1H), 3.87 (t, J=5.7Hz, 2H), 4.03-4.30 (m, 5H), 5.32 (d, J=16.1Hz, 1H), 6.57-6.84 (m, 3H), 7.04 (d, J=8.3Hz, 1H), 8.18 (brs, 1H), 12.16 (brs, 2H).

### Example 3

### 3-Oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate trifluoroacetate

### 3-(a) 2-(Imidazo[1,2-b][1,2,4]triazin-6-yl)ethanol

To 40 mL of an ethanol solution containing 1.92 g (20.0 mmol) of 3-amino-1,2,4-triazine was added 3.67 g (22.0 mmol) of 1-bromo-4-hydroxy-2-butanone obtained in Example 1-(a), and the mixture was refluxed for 2 hours.
After completion of the reaction, the reaction mixture was concentrated under reduced pressure, and 200 mL of a mixed solution of chloroform:methanol=9:1 (V/V) was added to the obtained residue and insoluble material was filtered off. The filtrate was successively washed with water and saturated aqueous sodium chloride solution, dried over anhydrous sodium sulfate, and then, concentrated under reduced pressure. The obtained residue was applied to silica gel column chromatography (eluent; chloroform:methanol=30:1→9: (V/V)), and the separated fractions containing the objective material were concentrated under reduced pressure to obtain 0.24 g of the title compound as yellow solid. (Yield: 7%)
Mass Spectrum (CI, m/z): 165 (M+⁺1).
¹H-NMR Spectrum (CDCl₃, δ ppm): 3.13 (td, J₁=5.5Hz, J₂=0.5Hz, 2H), 4.09 (t, J=5.5Hz, 2H), 7.85 (s, 1H), 8.33 (d, J=1.9Hz, 1H), 8.40 (d, J=1.9Hz, 1H).

### 3-(b) Ethyl 8-[2-(imidazo[1,2-b][1,2,4]triazin-6-yl)ethoxy]-3-oxo-2-(2,2,2-trifluoroethyl)-2,3,4,5-tetrahydro-1H-2-benzazepin-4-acetate

To 50 mL of a tetrahydrofuran solution containing 0.81 g (4.94 mmol) of 2-(imidazo[1,2-b][1,2,4]triazin-6-yl)ethanol obtained by the same reaction as in Example 3-(a) were added 1.66 g (4.80 mmol) of ethyl 8-hydroxy-3-oxo-2-(2,2,2-trifluoroethyl)-2,3,4,5-tetrahydro-1H-2-benzazepin-4-acetate (see WO 98/14192A) and 1.76 g (6.71 mmol) of triphenylphosphine, and the mixture was stirred under ice-cooling. Then, 9 mL of a tetrahydrofuran solution containing 1.26 g (6.23 mmol) of diisopropyl azodicarboxylate was added dropwise to the mixture over 45 minutes, and after gradually raised the temperature to room temperature, the mixture was stirred for 12 hours.
After completion of the reaction, the reaction mixture was concentrated under reduced pressure, and the residue was applied to silica gel column chromatography (eluent; chloroform:tetrahydrofuran=9:1 (V/V)). The separated fractions containing the objective material were concentrated under reduced pressure to obtain 0.77 g of the title compound as yellow foam. (Yield: 33%)
Mass Spectrum (CI, m/z): 492 (M⁺+1).
¹H-NMR Spectrum (CDCl₃, δ ppm): 1.26 (t, J=7.2Hz, 3H), 2.44 (dd, J₁=16.8Hz, J₂=5.4Hz, 1H), 2.83-3.05 (m, 3H), 3.35-3.39 (m, 2H), 3.78-3.88 (m, 1H), 3.91-4.02 (m, 2H), 4.10-4.24 (m, 1H), 4.16 (q, J=7.2Hz, 2H), 4.37-4.44 (m, 2H), 5.32 (d, J=16.8Hz, 1H), 6.65 (d, J=2.7Hz, 1H), 6.82 (dd, J₁=8.5Hz, J₂=2.7Hz, 1H), 7.02 (d, J=8.5Hz, 1H), 7.90 (s, 1H), 8.31 (d, J=2.0Hz, 1H), 8.40 (d, J=2.0Hz, 1H).

### 3-(c) 8-[2-(Imidazo[1,2-b][1,2,4]triazin-6-yl)ethoxy]-3-oxo-2-(2,2,2-trifluoroethyl)-2,3,4,5-tetrahydro-1H-2-benzazepin-4-acetic acid

To 1 mL of an ethanol solution containing 49.2 mg (0.100 mmol) of ethyl 8-[2-(imidazo[1,2-b][1,2,4]triazin-6-yl)ethoxy]-3-oxo-2-(2,2,2-trifluoroethyl)-2,3,4,5-tetrahydro-1H-2-benzazepin-4-acetate obtained in Example 3-(b) was added 200 µL (200 µmol) of 1N aqueous sodium hydroxide solution, and the mixture was stirred under room temperature for 20 hours.
After completion of the reaction, 1 mL of a saturated aqueous ammonium chloride solution was added to the reaction mixture, and the mixture was extracted with 30 ml. of a mixed solution of chloroform:methanol=9:1 (V/V). The organic layer was dried over anhydrous sodium sulfate, and then, concentrated under reduced pressure. The obtained residue was applied to silica gel column chromatography (eluent; chloroform:methanol=25:1 (V/V)→chloroform:methanol:acetic acid=25:1:0.1 (V/V/V)), and the separated fractions containing the objective material were concentrated under reduced pressure to obtain 28.7 mg of the title compound as pale yellow solid. (Yield: 62%)
Mass Spectrum (CI, m/z): 464(M⁺+1).
¹H-NMR Spectrum (DMSO-d₆, δ ppm): 2.36 (dd, J₁=16.8Hz, J₂=5.1Hz, 1H), 2.59-2.73 (m, 2H), 2.97-3.03 (m, 1H), 3.24 (t, J=6.6Hz, 2H), 3.71-3.82 (m, 1H), 4.13-4.25 (m, 3H), 4.35 (t, J=6.6Hz, 2H), 5.30 (d, J=16.8Hz, 1H), 6.81-6.85 (m, 2H), 7.01-7.04 (m, 1H), 8.27 (s, 1H), 8.53 (d, J=2.0Hz, 1H), 8.63 (d, J=2.0Hz, 1H).

### 3-(d) 3-Oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate trifluoroacetate

To 1.7 mL of an ethanol solution containing 26.4 mg (0.057 mmol) of 8-[2-(imidazo[1,2-b][1,2,4]triazin-6-yl)ethoxy]-3-oxo-2-(2,2,2-trifluoroethyl)-2,3,4,5-tetrahydro-1H-2-benzazepin-4-acetic acid obtained in Example 3-(c) was added 3.5 mg (0.093 mmol) of sodium borohydride, the mixture was stirred under room temperature for 1 hour, 2.5 mg (0.066 mmol) of sodium borohydride was further added to the mixture, and the resulting mixture was stirred at the same temperature for 1 hour.
After completion of the reaction, the reaction mixture was concentrated under reduced pressure, and the residue was purified by high performance liquid chromatography (column; TSK-GEL ODS-80Ts (20 mm×250 mm (available from TOSOH CORPORATION), eluent; acetonitrile:water:trifluoroacetic acid=300:700:1 (V/V/V), flow rate; 20 mL/min) to obtain 8.9 mg of the title compound as white foamy product.
(Yield: 27%)
Mass Spectrum (CI, m/z): 468 (M⁺+1).
¹H-NMR Spectrum (CD₃CN, δ ppm): 2.45 (dd, J₁=16.8Hz, J₂=4.8Hz, 1H), 2.72-2.82 (m, 2H), 2.87-2.92 (m, 2H), 2.99-3.06 (m, 1H), 3.18-3.25 (m, 2H), 3.37-3.41 (m, 2H), 3.74-3.85 (m, 1H), 4.01-4.22 (m, 5H), 5.30 (d, J=16.8Hz, 1H), 6.50 (s, 1H), 6.80-6.85 (m, 2H), 7.04 (d, J=8.5Hz, 1H), 8.96 (s, 1H), 13.62 (brs, 1H).

### Example 4

### (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetic acid

### 4-(a) 2-(2-tert-Butyldimethylsilyloxyethyl)-imidazo[1,2-a]pyrimidine

To 300 mL of a dimethylformamide solution containing 60.0 g (368 mmol) of 2-(imidazo[1,2-a]pyrimidin-2-yl)ethanol obtained in Example 1-(b) were added 61.0 g (405 mmol) of tert-butyldimethylsilyl chloride and 55.1 g (810 mmol) of imidazole at room temperature, and the mixture was stirred at the same temperature for 12 hours.
After completion of the reaction, the reaction mixture was poured into 500 mL of a saturated sodium hydrogen carbonate solution, and extracted with 800 mL of toluene twice. The combined organic layers were washed with a saturated aqueous sodium chloride solution, dried over anhydrous magnesium sulfate, and concentrated under reduced pressure to obtain 70.3 g of the title compound as pale brown solid. (Yield: 69%)
Mass Spectrum (CI, m/z): 278 (M⁺+1).
¹H-NMR Spectrum (CDCl₃, δ ppm): 0.03 (s, 6H), 0.88 (s, 9H), 3.05 (td, J₁=6.8Hz, J₂=0.6Hz, 2H), 4.01 (t, 2H), 6.81 (dd, J₁=6.6Hz, J₂=4.2Hz, 1H), 7.39 (s, 1H), 8.36 (dd, J₁=6.6Hz, J₂=2.0Hz, 1H), 8.49 (dd, J₁=4.2Hz, J₂=2.0Hz, 1H).

### 4-(b) tert-Butyl 2-(2-tert-butyldimethylsilyloxyethyl)-6,7-dihydro-5H-imidazo[1,2-a]-pyrimidine-8-carboxylate

To 650 mL of a methanol solution containing 60.0 g (216 mmol) of 2-(2-tert-butyldimethylsilyloxyethyl)-imidazo[1,2-a]pyrimidine obtained in Example 4-(a) was added 20.5 g (542 mmol) of sodium borohydride at room temperature, and the resulting mixture was stirred at the same temperature for 5 hours.
After completion of the reaction, the reaction mixture was concentrated under reduced pressure to a volume of 350 mL, and poured into 500 mL of water. The mixture was extracted with 500 mL of ethyl acetate twice, the extract was dried over anhydrous magnesium sulfate, and then, concentrated under reduced pressure. The obtained residue was dissolved in 350 mL of dichloromethane, and to the solution were added 35.6 mL (256 mmol) oftriethylamine, 2.60 g (21.3 mmol) of 4-dimethylaminopyridine, and 55.9 g (256 mmol) of di-tert-butyl dicarbonate at room temperature, and the resulting mixture was stirred at the same temperature for 2 hours. After completion of the reaction, the reaction mixture was concentrated under reduced pressure, the residue was poured into 500 mL of a saturated sodium hydrogen carbonate solution, and extracted with 500 mL of ethyl acetate twice. The combined organic layers were washed with a saturated aqueous sodium chloride solution, dried over anhydrous magnesium sulfate, and concentrated under reduced pressure to obtain 80.2 g of the title compound as yellow oily product. (Yield: 97%)
Mass Spectrum (CI, m/z): 381 (M⁺).
¹H-NMR Spectrum (CDCl₃, δ ppm): 0.04 (s, 6H), 0.89 (s, 9H), 1.54 (s, 9H), 2.03-2.11 (m, 2H), 2.78 (td, J₁=7.2Hz, J₂=0.7Hz, 2H), 3.80-3.90 (m, 6H), 6.43 (s, 1H).

### 4-(c) tert-Butyl 2-(2-hydroxyethyl)-6,7-dihydro-5H-imidazo[1,2-a]pyrimidine-8-carboxylate

To 150 mL of a methanol solution containing 80.2 g (210 mmol) of tert-butyl 2-[2-(tert-butyldimethylsilyloxy)ethyl]-6,7-dihydro-5H-imidazo[1,2-a]pyrimidine-8-carboxylate obtained in Example 4-(b) was added 47.9 g (252 mmol) of p-toluene-sulfonic acid monohydrate at room temperature, and the mixture was stirred at the same temperature for 3.5 hours.
After completion of the reaction, the reaction mixture was poured into 500 mL of a saturated sodium hydrogen carbonate solution, and extracted with 300 mL of ethyl acetate eight times, and then, 200 mL of a mixed solution of chloroform/methanol=4:1 (V/V) twice. The combined organic layers were dried over anhydrous sodium sulfate, and then, concentrated under reduced pressure to obtain 58.0 g of the title compound as yellow solid. (Yield: quantitative)
Mass Spectrum (CI, m/z): 267(M⁺).
¹H-NMR Spectrum (CDCl₃, δ ppm): 1.56 (s, 9H), 2.08-2.16 (m, 2H), 2.36 (s, 1H), 2.72 (td, J₁=5.4Hz, J₂=0.7Hz, 2H), 3.84-3.93 (m, 6H), 6.44 (s, 1H).

### 4-(d) tert-Butyl 2-(2-methanesulfonyloxyethyl)-6, 7-dihydro-5H-imidazo[1,2-a]-pyrimidine-8-carboxylate

To 150 mL of a dichloromethane solution containing 20.0 g (74.8 mmol) of tert-butyl 2-(2-hydroxyethyl)-6, 7-dihydro-5H-imidazo[1,2-a]pyrimidine-8-carboxylate obtained in Example 4-(c) were added 11.5 mL (82.3 mmol) of triethylamine and 6.37 mL (82.3 mmol) of methanesulfonyl chloride at room temperature, and the resulting mixture was stirred at the same temperature for 2 hours.
After completion of the reaction, the reaction mixture was poured into 500 mL of a saturated sodium hydrogen carbonate solution, and extracted with 300 mL of ethyl acetate twice. The combined organic layers were dried over anhydrous magnesium sulfate, and concentrated under reduced pressure to obtain 20.6 g of the title compound as brown oily product. (Yield: 80%)
Mass Spectrum (CI, m/z): 345 (M⁺).
¹H-NMR Spectrum (CDCl₃, δ ppm): 1.55 (s, 9H), 2.06-2.14 (m, 2H), 2.97 (s, 3H), 3.01 (td, J₁=6.7Hz, J₂=0.5Hz, 2H), 3.82-3.86 (m, 2H), 3.91 (t, J=6.2Hz, 2H), 4.48 (t, J=6.7Hz, 2H), 6.52 (s, 1H).

### 4-(e) Ethyl (4S)-3-oxo-8-[2-(8-tert-butoxycarbonyl-5,6,7,8-tetrahydroimidazo[1,2-a]-pyrimidin-2-yl)ethoxy]-2-(2,2,2-trifluoroethyl)-2,3,4,5-tetrahydro-1H-2-benzazepin-4-acetate

To 40 mL of a tetrahydrofuran solution containing 3.87 g (11.2 mmol) of ethyl (4S)-8-hydroxy-3-oxo-2-(2,2,2-trifluoroethyl)-2,3,4,5-tetrahydro-1H-2-benzazepin-4-acetate (see WO 2004/089890A) was added 469 mg (11.7 mmol) of sodium hydride (60% dispersed material in mineral oil) at room temperature, and the mixture was stirred for 30 minutes. Then, to the mixture was added 10 mL of a tetrahydrofuran solution containing 3.54 g (10.2 mmol) of tert-butyl 2-(2-methanesulfonyloxyethyl)-6,7-dihydro-5H-imidazo[1,2-a]pyrimidine-8-carboxylate obtained in Example 4-(d) at room temperature, and the resulting mixture was stirred at the same temperature for 20 hours.
After completion of the reaction, the reaction mixture was poured into 200 mL of water, and extracted with 100 mL of ethyl acetate twice. The combined organic layers were dried over anhydrous magnesium sulfate, and then, concentrated under reduced pressure. The obtained residue was applied to silica gel column chromatography (eluent; ethyl acetate→chloroform:methanol=14:1 (V/V)), and the separated fractions containing the objective material were concentrated under reduced pressure to obtain 4.60 g the title compound as pale brown foam. (Yield: 76%)
Mass Spectrum (CI, m/z): 595 (M⁺+1).
H-NMR Spectrum (CDCl₃, δ ppm): 1.27 (t, J=7.2Hz, 3H), 1.54 (s, 9H), 2.05-2.13 (m, 2H), 2.44 (dd, J₁=16.8Hz, J₂=5.6Hz, 1H), 2.87-3.05 (m, 5H), 3.82-3.99 (m, 7H), 4.11-4.24 (m, 5H), 5.33 (d, J=16.8Hz, 1H), 6.50 (s, 1H), 6.64 (d, J=2.4Hz, 1H), 6.80 (dd, J₁=8.5Hz, J₂=2.7Hz, 1H), 7.00 (d, J=8.5Hz, 1H).

### 4-(f) Ethyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate hydrochloride

To 8 mL of a tetrahydrofuran solution containing 4.60 g (7.74 mmol) of ethyl (4S)-3-oxo-8-[2-(8-tert-butoxycarbonyl-5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate obtained in Example 4-(e) were added 8 mL of acetic acid and 24 mL of water, and the mixture was stirred at 70°C for 7 hours.
After completion of the reaction, the reaction mixture was concentrated under reduced pressure, and the residue was applied to silica gel column chromatography (eluent; chloroform:methanol:acetic acid=90:10:3 (V/V/V)). The separated fractions containing the objective material were concentrated under reduced pressure, and the resulting oily product was dissolved in 4 mL of ethanol. To the solution was added 8.6 mL of 2.7N hydrogen chloride/ethanol solution, and then, the mixture was concentrated under reduced pressure to obtain 3.10 g of the title compound as pale yellow foam. (Yield: 75%)
Mass Spectrum (FAB, m/z): 495 (M⁺+1).
¹H-NMR Spectrum (DMSO-d₆, δ ppm): 1.17 (t, J=7.1Hz, 3H), 1.94-2.02 (m, 2H), 2.63-2.76 (m, 2H), 2.89 (t, J=6.2Hz, 2H), 3.01 (dd, J₁=17.5Hz, J₂=3.9Hz, 1H), 3.78-3.89 (m, 3H), 4.04 (q, J=7.1Hz, 2H), 4.11-4.29 (m, 5H), 5.32 (d, J=16.6Hz, 1H), 6.81-6.85 (m, 3H), 7.05 (d, J=9.3Hz, 1H), 8.23 (brs, 1H), 12.28 (brs, 1H).

### 4-(g) (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetic acid

In 1.0 mL of a mixed solution of tetrahydrofuran/ethanol=1:1 (V/V) was dissolved 100 mg (0.188 mmol) of ethyl (4S)-3-oxo-8-[2-(5,6,7,8-tetrahydroimidazo-[1,2-a]pyrimidin-2-yl)ethoxy]-2-(2,2,2-trifluoroethyl)-2,3,4,5-tetrahydro-1H-2-benzazepin-4-acetate hydrochloride obtained in Example 4-(f), 1.13 mL (1.13 mmol) of 1N aqueous sodium hydroxide solution was added to the mixture, and the resulting mixture was stirred at 0°C for 10 hours.
After completion of the reaction, to the reaction mixture was added 75 µL (1.32 mmol) of acetic acid, inorganic acid salt was removed by Sep-Pak (Tradename), and then, concentrated under reduced pressure. The obtained residue was applied to silica gel column chromatography (eluent; chloroform:methanol:acetic acid=90:10:6 (V/V/V)), and the separated fractions containing the objective material were concentrated under reduced pressure to obtain 67.2 mg of the title compound as white solid. (Yield: 77%)
Mass Spectrum (FAB, m/z): 467 (M⁺+1).
¹H-NMR Spectrum (CDCl₃, δ ppm): 2.06-2.12 (m, 3H), 2.41 (dd, J₁=15.5Hz, J₂=8.2Hz, 1H), 2.78-2.95 (m, 4H), 3.10 (dd, J₁=17.6Hz, J₂=4.4Hz, 1H), 3.44 (t, J=5.4Hz, 2H), 3.74-3.85 (m, 5H), 4.13-4.27 (m, 3H), 5.16 (d, J=16.6Hz, 1H), 6.20 (s, 1H), 6.54 (d, J=2.4Hz, 1H), 6.76 (dd, J₁=8.5Hz, J₂=2.7Hz, 1H), 6.86 (d, J=8.5Hz, 1H).

### Example 5

Propyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate hydrochloride 5-(a) (4S)-3-oxo-8-[2-(8-tert-butoxycarbonyl-5,6,7,8-tetrahydroimidazo[1,2-a]-pyrimidin-2-yl)ethoxy]-2-(2,2,2-trifluoroethyl)-2,3,4,5-tetrahydro-1H-2-benzazepin-4-acetic acid
In 20 mL of a tert-butyl methyl ether solution was dissolved 7.00 g (11.8 mmol) of ethyl (4S)-3-oxo-8-[2-(8-tert-butoxycarbonyl-5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate obtained in the same manner as in Example 4-(e), 10 mL of purified water and 1.04 g (24.8 mmol) of lithium hydroxide monohydrate were added to the solution and the resulting mixture was stirred at 50°C for 3 hours.
After completion of the reaction, to the reaction mixture were added 100 mL of purified water and 50 mL of tert-butyl methyl ether, and the aqueous layer was separated. To the obtained aqueous layer was added 1N aqueous hydrogen chloride solution to adjust a pH thereof to 5.0, and extracted with 500 mL of a mixed solution of chloroform:methanol=8:2 (V/V). The organic layer was washed with a saturated aqueous sodium chloride solution, dried over anhydrous sodium sulfate, and then, concentrated under reduced pressure to obtain 7.25 g of the title compound as pale yellow foam. (Yield: quantitative)
Mass Spectrum (FAB, m/z): 567 (M⁺+1).
¹H-NMR Spectrum (DMSO-d₆, δ ppm): 1.47 (s, 9H), 1.97-2.04 (m, 2H), 2.39 (dd, J₁=16.8Hz, J₂=4.9Hz, 1H), 2.61-2.72 (m, 2H), 2.85 (t, J=6.7Hz, 2H), 3.00 (dd, J₁=17.6Hz, J₂=3.7Hz, 1H), 3.72-3.92 (m, 5H), 4.10-4.28 (m, 5H), 5.30 (d, J=16.6Hz, 1H), 6.75-6.83 (m, 3H), 7.01-7.05 (m, 1H), 12.15 (brs, 1H).

### 5-(b) Propyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate hydrochloride

703 mg (1.24 mmol) of (4S)-3-oxo-8-[2-(8-tert-butoxycarbonyl-5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetic acid obtained in Example 5-(a) and 1.86 mL (24.8 mmol) of 1-propyl alcohol were mixed, 12.4 mL (49.6 mmol) of 4N hydrogen chloride/1,4-dioxane solution was added to the mixture, and the resulting mixture was stirred under nitrogen gas atmosphere at 45°C for 4.5 hours.
After completion of the reaction, the reaction mixture was concentrated under reduced pressure, and the residue was applied to silica gel column chromatography (eluent; ethyl acetate→chloroform: methanol=19:1 (V/V)), and the separated fractions containing the objective material were concentrated under reduced pressure to obtain 533 mg of the title compound as pale yellow foam. (Yield: 79%)
Mass Spectrum (FAB, m/z): 509 (M⁺+1)
¹H-NMR Spectrum (DMSO-d₆, δ ppm): 0.88 (t, J=7.4Hz, 3H), 1.51-1.63 (m, 2H), 1.94-2.01 (m, 2H), 2.63-2.77 (m, 2H), 2.89 (t, J=6.2Hz, 2H), 3.01 (dd, J₁=17.8Hz, J₂=4.2Hz, 1H), 3.79-3.89 (m, 3H), 3.96 (t, J=6.6Hz, 2H), 4.10-4.29 (m, 5H), 5.31 (d, J=16.4Hz, 1H), 6.80-6.85 (m, 3H), 7.04 (d, J=9.3Hz, 1H), 8.18 (brs, 1H), 12.24 (brs, 1H).

### Example 6

### Isopropyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate hydrochloride

640 mg (1.13 mmol) of (4S)-3-oxo-8-[2-(8-tert-butoxycarbonyl-5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetic acid obtained in Example 5-(a) and 2.00 mL (26.1 mmol) of 2-propyl alcohol were mixed, 11.3 mL (45.2 mmol) of 4N hydrogen chloride/1,4-dioxane solution was added to the mixture, and the resulting mixture was stirred under nitrogen gas atmosphere at 45°C for 4.5 hours.
After completion of the reaction, the reaction mixture was concentrated under reduced pressure, and the residue was applied to silica gel column chromatography (eluent; chloroform:methanol=19: (V/V)), and the separated fractions containing the objective material were concentrated under reduced pressure to obtain 424 mg of the title compound as pale yellow foam. (Yield: 69%)
Mass Spectrum (FAB, m/z): 509 (M⁺+1)
¹H-NMR Spectrum (DMSO-d₆, δ ppm): 1.16 (d, J=6.2Hz, 3H), 1.17 (d, J=6.2Hz, 3H), 1.94-2.01 (m, 2H), 2.63-2.72 (m, 2H), 2.89 (t, J=6.3, 2H), 3.00 (dd, J₁=17.7Hz, J₂=4.0Hz, 1H), 3.76-3.89 (m, 3H), 4.11-4.26 (m, 5H), 4.86 (septet, J=6.2Hz, 1H), 5.31 (d, J=16.4l1z, 1H), 6.80-6.85 (m, 3H), 7.04 (d, J=9.3Hz, 1H), 8.19 (brs, 1H), 12.26 (brs, 1H).

### Example 7

### Heptyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate hydrochloride

70.0 mg (0.124 mmol) of (4S)-3-oxo-8-[2-(8-tert-butoxycarbonyl-5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetic acid obtained in Example 5-(a) and 88 µL (0.62 mmol) of heptyl alcohol were mixed, 1.55 mL (6.20 mmol) of 4N hydrogen chloride/ 1,4-dioxane solution was added to the mixture, and the resulting mixture was stirred under nitrogen gas atmosphere at 50°C for 4 hours.
After completion of the reaction, the reaction mixture was concentrated under reduced pressure, and the residue was applied to silica gel column chromatography (eluent; ethyl acetate→chloroform:methanol=14:1 (V/V)), and the separated fractions containing the objective material were concentrated under reduced pressure to obtain 58.2 mg of the title compound as pale yellow foam. (Yield: 78%)
Mass Spectrum (FAB, m/z): 565 (M⁺+1).
¹H-NMR Spectrum (DMSO-d₆, δ ppm): 0.86 (t, J=6.8Hz, 3H), 1.24-1.28 (m, 8H), 1.52-1.57 (m, 2H), 1.94-2.01 (m, 2H), 2.63-2.76 (m, 2H), 2.89 (t, J=6.2Hz, 2H), 3.01 (dd, J₁=17.1Hz, J₂=3.9Hz, 1H), 3.77-3.89 (m, 3H), 3.99 (t, J=6.6Hz, 2H), 4.11-4.28 (m, 5H), 5.31 (d, J=16.4Hz, 1H), 6.80-6.85 (m, 3H), 7.04 (d, J=9.3Hz, 1H), 8.25 (brs, 1H), 12.33 (brs, 1H).

### Example 8

### Undecyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate hydrochloride

704 mg (1.24 mmol) of (4S)-3-oxo-8-[2-(8-tert-butoxycarbonyl-5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetic acid obtained in Example 5-(a) and 1.30 mL (6.26 mmol) of 1-undecyl alcohol were mixed, 12.4 mL (49.6 mmol) of 4N hydrogen chloride/1,4-dioxane solution was added to the mixture, and the resulting mixture was stirred under nitrogen gas atmosphere at 45°C for 4 hours.
After completion of the reaction, the reaction mixture was concentrated under reduced pressure, and the residue was applied to silica gel column chromatography (eluent; ethyl acetate→chloroform:methanol=19:1 (V/V)), and the separated fractions containing the objective material were concentrated under reduced pressure to obtain 527 mg of the title compound as pale yellow foam. (Yield: 65%)
Mass Spectrum (FAB, m/z): 621 (M+1)
¹H-NMR Spectrum (DMSO-d₆, δ ppm): 0.85 (t, J=6.7Hz, 3H), 1.21-1.31 (m, 16H), 1.50-1.58 (m, 2H), 1.94-2.02 (m, 2H), 2.63-2.76 (m, 2H), 2.89 (t, J=6.3Hz, 2H), 3.01 (dd, J₁=17.3Hz, J₂=3.7Hz, 1H), 3.78-3.89 (m, 3H), 3.99 (t, J=6.5Hz, 2H), 4.11-4.28 (m, 5H), 5.31 (d, J=16.6Hz, 1H), 6.80-6.85 (m, 3H), 7.04 (d, J=9.5Hz, 1H), 8.22 (brs, 1H), 12.29 (brs, 1H).

### Example 9

### (10-Phenyl)decyl (4S)-3-oxo-8-[2-(5,6,7,8-tetrahydroimidazo[1,2-a]pyrimidin-2-yl)-ethoxy]-2-(2,2,2-trifluoroethyl)-2,3,4,5-tetrahydro-1H-2-benzazepin-4-acetate hydrochloride

984 mg (1.74 mmol) of (4S)-3-oxo-8-[2-(8-tert-butoxycarbonyl-5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetic acid obtained in Example 5-(a) and 2.12 g (8.68 mmol) of 10-phenyl-1-decyl alcohol were mixed, 17.4 mL (69.6 mmol) of 4N hydrogen chloride/1,4-dioxane solution was added to the mixture, and the resulting mixture was stirred under nitrogen gas atmosphere at 50°C for 4 hours.
After completion of the reaction, the reaction mixture was concentrated under reduced pressure, and the residue was applied to silica gel column chromatography (eluent; ethyl acetate→chloroform:methanol=19:1 (V/V)), and the separated fractions containing the objective material were concentrated under reduced pressure to obtain 815 mg of the title compound as pale yellow foam. (Yield: 65%)
Mass Spectrum (FAB, m/z): 683 (M⁺+1)
¹H-NMR Spectrum (DMSO-d₆, δ ppm): 1.25-1.59 (m, 16H), 1.94-2.01 (m, 2H), 2.63-2.76 (m, 2H), 2.89 (t, J=6.2Hz, 2H), 3.00 (dd, J₁=17.6Hz, J₂=3.9Hz, 1H), 3.78-3.89 (m, 3H), 3.98 (t, J=6.6Hz, 2H), 4.10-4.28 (m, 5H), 5.31 (d, J=16.4Hz, 1H), 6.80-6.85 (m, 3H), 7.04 (d, J=9.3Hz, 1H), 7.13-7.29 (m, 5H), 8.21 (brs, 1H), 12.23 (brs, 1H).

### Example 10

[2-(Morpholin-4-yl)]ethyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate hydrochloride

10-(a) [2-(Morpholin-4-yl)]ethyl(4S)-3-oxo-8-[2-(8-tert-butoxycarbonyl-5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate
To 1 mL of a dichloromethane solution containing 77.4 mg (0.137 mmol) of (4S)-3-oxo-8-[2-(8-tert-butoxycarbonyl-5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetic acid obtained in Example 5-(a) were added 33 µL (0.27 mmol) of N-(2-hydroxylethyl)-morpholine, 1.7 mg (0.014 mmol) of 4-dimethylaminopyridine and 53.0 mg (0.276 mmol) of 1-ethyl-3-(3-dimethylaminopropyl)carbodiimide hydrochloride, under argon gas atmosphere, and the resulting mixture was stirred at room temperature for 16 hours.
After completion of the reaction, 3 mL of dichloromethane was poured into the reaction mixture, the mixture was washed with a saturated aqueous ammonium chloride solution, saturated sodium hydrogen carbonate solution and saturated aqueous sodium chloride solution, dried over anhydrous magnesium sulfate, and then, concentrated under reduced pressure to obtain 71.7 mg of the title compound as pale yellow foam. (Yield: 77%)
Mass Spectrum (FAB, m/z): 680 (M⁺+1)
H-NMR Spectrum (DMSO-d₆, δ ppm): 1.45 (s, 9H), 1.94-2.02 (m, 2H), 2.38-2.41 (m, 4H), 2.63-2.83 (m, 4H), 3.02 (dd, J₁=17.2Hz, J₂=4.3Hz, 1H), 3.52-3.55 (m, 4H), 3.70-3.90 (m, 5H), 4.09-4.26 (m, 7H), 5.29 (d, J=16.4Hz, 1H), 6.71 (s, 1H), 6.78-6.82 (m, 2H), 7.01 (d, J=9.3Hz, 1H).

### 10-(b) [2-(Morpholin-4-yl)]ethyl (4S)-3-oxo-8-[2-(5,6,7,8-tetrahydroimidazo[1,2-a]-pyrimidin-2-yl)ethoxy]-2-(2,2,2-trifluoroethyl)-2,3,4,5-tetrahydro-1H-2-benzazepin-4-acetate hydrochloride

To 1 mL of a dichloromethane solution containing 71.7 mg (0.105 mmol) of [2-(morpholin-4-yl)]ethyl (4S)-3-oxo-8-[2-(8-tert-butoxycarbonyl-5,6,7,8-tetrahydro-imidazo[1,2-a]pyrimidin-2-yl)ethoxy]-2-(2,2,2-trifluoroethyl)-2,3,4,5-tetrahydro-1H-2-benzazepin-4-acetate obtained in Example 10-(a) was added 2.00 mL (8.00 mmol) of 4N hydrogen chloride/1,4-dioxane solution, and the mixture was stirred under nitrogen gas atmosphere at room temperature for 12 hours.
After completion of the reaction, the reaction mixture was concentrated under reduced pressure, and the residue was applied to silica gel column chromatography (eluent; ethyl acetate→chloroform:methanol=8:1 (V/V)), and the separated fractions containing the objective material were concentrated under reduced pressure to obtain 55.1 mg of the title compound as pale yellow foam. (Yield: 80%)
Mass Spectrum (FAB, m/z): 580 (M+1)
H-NMR Spectrum (DMSO-d₆, δ ppm): 1.94-2.01 (m, 2H), 2.56-2.82 (m, 3H), 2.89 (t, J=6.1Hz, 2H), 3.04 (dd, J₁=17.5Hz, J₂=4.3Hz, 1H), 3.75-3.96 (m, 7H), 4.11-4.41 (m, 7H), 5.38 (d, J=16.6Hz, 1H), 6.80-6.86 (m, 3H), 7.05 (d, J=9.0Hz, 1H), 8.26 (brs, 1H), 11.31 (brs, 1H), 12.35 (brs, 1H).

### Example 11

(5-Methyl-2-oxo-[1,3]dioxolen-4-yl)methyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate hydrochloride

11-(a)(5-Methyl-2-oxo-[1,3]dioxolen-4-yl)methyl (4S)-3-oxo-8-[2-(8-tert-butoxycarbonyl-5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate
To 1.0 mL of a dimethylformamide solution containing 113 mg (0.200 mmol) of (4S)-3-oxo-8-[2-(8-tert-butoxycarbonyl-5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetic acid obtained in Example 5-(a) was added 21 mg (0.25 mmol) of sodium hydrogen carbonate, and the mixture was stirred under argon gas atmosphere and under ice-cooling. Then, to the mixture was added 29.7 mg (0.200 mmol) of 4-chloromethyl-5-methyl-1,3-dioxol-2-one, and the resulting mixture was stirred at the same temperature for 2.5 hours. After raising the temperature to room temperature, 55.3 mg (0.400 mmol) of potassium carbonate was added to the mixture, and the resulting mixture was stirred at room temperature for 6 hours. Further, 7.4 mg (0.050 mmol) of 4-chloromethyl-5-methyl-1,3-dioxol-2-one was added to the mixture, the mixture was stirred at room temperature for 15.5 hours.
After completion of the reaction, the reaction mixture was poured into 10 ml of water, and extracted with 30 mL of ethyl acetate. The organic layer was washed with a saturated aqueous sodium chloride solution, dried over anhydrous magnesium sulfate, and then, concentrated under reduced pressure. The obtained residue was applied to silica gel column chromatography (eluent; chloroform:methanol=39:1→ 25:1 (V/V)), and the separated fractions containing the objective material were concentrated under reduced pressure to obtain 116 mg of the title compound as brown oily product. (Yield: 86%)
Mass Spectrum (FAB, m/z): 679 (M⁺+1).
¹H-NMR Spectrum (CDCl₃, δ ppm): 1.54 (s, 9H), 2.05-2.13 (m, 2H), 2.16 (s, 3H), 2.49 (dd, J₁=17.0Hz, J₂=4.8Hz, 1H), 2.84-3.06 (m, 5H), 3.78-4.00 (m, 7H), 4.15-4.29 (m, 3H), 4.81 (d, J=13.8Hz, 1H), 4.91 (d, J=13.8Hz, 1H), 5.31 (d, J=16.4Hz, 1H), 6.50 (s, 1H), 6.64 (d, J=2.7Hz, 1H), 6.81 (dd, J₁=8.4Hz, J₂=2.7Hz, 1H), 7.00 (d, J=8.6Hz, 1H).

### 11-(b) (5-Methyl-2-oxo-[1,3]dioxolen-4-yl)methyl (4S)-3-oxo-8-[2-(5,6,7,8-tetrahydroimidazo 1,2-a]pyrimidin-2-yl)ethoxy]-2-(2,2,2-trifluoroethyl)-2,3,4,5-tetrahydro-1H-2-benzazepin-4-acetate hydrochloride

To 3 mL of a dichloromethane solution containing 107 mg (0.158 mmol) of (5-methyl-2-oxo-[1,3]dioxolen-4-yl)methyl (4S)-3-oxo-8-[2-(8-tert-butoxycarbonyl-5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate obtained in Example 11-(a) was added 593 µL (2.37 mmol) of 4N hydrogen chloride/1,4-dioxane solution, and the resulting mixture was stirred under argon gas atmosphere at room temperature for 23 hours. Further, 319 µL (1.28 mmol) of 4N hydrogen chloride/1,4-dioxane solution was added to the mixture, and the resulting mixture was stirred for 20 hours.
After completion of the reaction, the reaction mixture was concentrated under reduced pressure, and the residue was applied to silica gel column chromatography (eluent; chloroform:methanol=19:1→9:1 (V/V)). The separated fractions containing the objective material were concentrated under reduced pressure, the resulting oily product was dissolved in 5 mL of dichloromethane, and after adding 49 µL (0.20 mmol) of 4N hydrogen chloride/1,4-dioxane solution, the mixture was concentrated under reduced pressure to obtain 57.4 mg of the title compound as white foamy product. (Yield: 59%)
Mass Spectrum (FAB, m/z): 579 (M⁺+1).
¹H-NMR Spectrum (DMSO-d₆, δ ppm): 1.94-2.01 (m, 2H), 2.15 (s, 3H), 2.54-2.80 (m, 3H), 2.89 (t, J=6.1Hz, 2H), 3.01 (dd, J₁=17.2Hz, J₂=3.5Hz, 1H), 3.81-3.89 (m, 3H), 4.10-4.29 (m, 5H), 4.91 (d, J=14.2Hz, 1H), 4.98 (d, J=14.2Hz, 1H), 5.32 (d, J=16.4Hz, 1H), 6.80-6.85 (m, 3H), 7.04 (d, J=8.8Hz, 1H), 8.17 (brs, 1H), 12.19 (brs, 1H).

### Example 12

(N,N-dimethylaminocarbonyl)methyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate hydrochloride

### 12-(a) (N,N-dimethylaminocarbonyl)methyl (4S)-3-oxo-8-[2-(8-tert-butoxycarbonyl-5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate

To 1 mL of a dimethylformamide solution containing 113 mg (0.200 mmol) of (4S)-3-oxo-8-[2-(8-tert-butoxycarbonyl-5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetic acid obtained in Example 5-(a) was added 55.3 mg (0.400 mmol) of potassium carbonate, and the mixture was stirred under argon gas atmosphere at room temperature. Then, 26 µL (0.25 mmol) of 2-chloro-N,N-dimethylacetamide was added to the mixture, and the resulting mixture was stirred at the same temperature for 8 hours.
After completion of the reaction, the reaction mixture was poured into 10 ml of water, and extracted with 30 mL of ethyl acetate. The organic layer was washed with a saturated aqueous sodium chloride solution, dried over anhydrous magnesium sulfate, and then, concentrated under reduced pressure. The obtained residue was applied to silica gel column chromatography (eluent; chloroform: methanol=40:1→ 19:1 (V/V)), and the separated fractions containing the objective material were concentrated under reduced pressure to obtain 131 mg of the title compound as yellow oily product. (Yield: quantitative)
Mass Spectrum (FAB, m/z): 652 (M⁺+1).
¹H-NMR Spectrum (CDCl₃, δ ppm): 1.54 (s, 9H), 2.05-2.13 (m, 2H), 2.64 (dd, J₁=17.0Hz, J₂=5.5Hz, 1H), 2.84-3.17 (m, 11H), 3.82-3.98 (m, 7H), 4.15-4.26 (m, 3H), 4.64 (d, J=14.4Hz, 1H), 4.85 (d, J=14.4Hz, 1H), 5.31 (d, J=16.1Hz, 1H), 6.49 (s, 1H), 6.63 (d, J=2.7Hz, 1H), 6.80 (dd, J₁=8.5Hz, J₂=2.7Hz, 1H), 7.02 (d, J=8.5Hz, 1H).

### 12-(b) (N,N-dimethylaminocarbonyl)methyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate hydrochloride

To 4 mL of a dichloromethane solution containing 125 mg (0.192 mmol) of (N,N-dimethylaminocarbonyl)methyl (4S)-3-oxo-8-[2-(8-tert-butoxycarbonyl-5,6,7,8-tetrahydroimidazo[1,2-a]pyrimidin-2-yl)ethoxy]-2-(2,2,2-trifluoroethyl)-2,3,4,5-tetra-hydro-1H-2-benzazepin-4-acetate obtained in Example 12-(a) was added 864 µL (3.46 mmol) of 4N hydrogen chloride/1,4-dioxane solution, and the mixture was stirred under argon gas atmosphere at room temperature for 19.5 hours.
After completion of the reaction, the reaction mixture was concentrated under reduced pressure, and the residue was applied to silica gel column chromatography (eluent; chloroform:methanol= 19:1-9:1 (V/V)). The separated fractions containing the objective material were concentrated under reduced pressure to obtain 50.2 mg of the title compound as white foamy product. (Yield: 44%)
Mass Spectrum (FAB, m/z): 552 (M⁺+1).
¹H-NMR Spectrum (DMSO-d₆, δ ppm): 1.93-2.01 (m, 2H), 2.55 (dd, J₁=16.8Hz, J₂=5.9Hz, 1H), 2.66-2.92 (m, 10H), 3.12 (dd, J₁=17.1Hz, J₂=3.9Hz, 1H), 3.76-3.88 (m, 3H), 4.10-4.31 (m, 5H), 4.70 (d, J=14.6Hz, 1H), 4.81 (d, J=14.6Hz, 1H), 5.30 (d, J=16.1Hz, 1H), 6.76-6.85 (m, 3H), 7.05 (d, J=8.1Hz, 1H), 7.97 (brs, 1H).

### Example 13

Pivaloyloxymethyl (4S)-3-oxo-8-[2-(5,6,7,8-tetrahydroimidazo[1,2-a]pyrimidin-2-yl)-ethoxy]-2-(2,2,2-trifluoroethyl)-2,3,4,5-tetrahydro-1H-2-benzazepin-4-acetate hydrochloride

### 13-(a) Pivaloyloxymethyl (4S)-3-oxo-8-[2-(8-tert-butoxycarbonyl-5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate

To 5 mL of an acetone solution containing 900 mg (1.59 mmol) of (4S)-3-oxo-8-[2-(8-tert-butoxycarbonyl-5,6,7,8-tetrahydroimidazo[1,2-a]pyrimidin-2-yl)-ethoxy]-2-(2,2,2-trifluoroethyl)-2,3,4,5-tetrahydro-1H-2-benzazepin-4-acetic acid obtained in Example 5-(a) were added 0.66 mL (4.8 mmol) of triethylamine and 0.69 mL (4.8 mmol) of pivaloyloxymethyl chloride, and the mixture was stirred at room temperature for 6 hours.
After completion of the reaction, the reaction mixture was poured into 50 ml of water, and extracted with 20 mL of ethyl acetate three times. The combined organic layers were dried over dried over anhydrous magnesium sulfate, and the residue was applied to silica gel column chromatography (eluent; ethyl acetate→ chloroform:methanol=13:1 (V/V)). The separated fractions containing the objective material were concentrated under reduced pressure to obtain 570 mg of the title compound as colorless foamy product. (Yield: 53%)
Mass Spectrum (FAB, m/z): 681 (M⁺+1).
¹H-NMR Spectrum (DMSO-d₆, δ ppm): 1.14 (s, 9H), 1.45 (s, 9H), 1.94-2.01 (m, 2H), 2.58 (dd, J₁=16.8Hz, J₂=4.9Hz, 1H), 2.70-2.82 (m, 4H), 3.01 (dd, J₁=17.6Hz, J₂=3.9Hz, 1H), 3.69-3.73 (m, 2H), 3.80-3.90 (m, 3H), 4.10-4.23 (m, 5H), 5.30 (d, J=16.4Hz, 1H), 5.69 (s, 2H), 6.70 (s, 1H), 6.78-6.82 (m, 2H), 7.00 (d, J=9.3Hz, 1H).

### 13-(b) Pivaloyloxymethyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate hydrochloride

To 2 mL of a tetrahydrofuran solution containing 550 mg (0.808 mmol) of pivaloyloxymethyl (4S)-3-oxo-8-[2-(8-tert-butoxycarbonyl-5,6,7,8-tetrahydroimidazo-[1,2-a]pyrimidin-2-yl)ethoxy]-2-(2,2,2-trifluoroethyl)-2,3,4,5-tetrahydro-1H-2-benz-azepin-4-acetate obtained in Example 13-(a) were added 2 mL of acetic acid and 2 mL of water, and the mixture was stirred at 50°C for 19 hours.
After completion of the reaction, the reaction mixture was concentrated under reduced pressure, and the residue was applied to silica gel column chromatography (eluent; chloroform:methanol=14:1→9:1 (V/V)). The separated fractions containing the objective material were concentrated under reduced pressure, the resulting oily product was dissolved in 1.0 mL (4.0 mmol) of 4N hydrogen chloride/1,4-dioxane solution, and then, concentrated under reduced pressure to obtain 341 mg of the title compound as pale yellow foam. (Yield: 68%)
Mass Spectrum (FAB, m/z): 581 (M⁺+1).
¹H-NMR Spectrum (DMSO-d₆, δ ppm) 1.14 (s, 9H), 1.93-2.01 (m, 2H), 2.58 (dd, J₁=16.9Hz. J₂= 4.8Hz, 1H), 2.65-2.81 (m, 2H), 2.89 (t, J=6.2Hz, 2H), 3.02 (dd, J1=17.3Hz. J2=3.9Hz, 1H), 3.80-3.89 (m, 3H), 4.11-4.32 (m, 5H), 5.31 (d, J=16.4Hz, 1H), 5.69 (s, 2H), 6.80-6.85 (m, 3H), 7.03 (d, J=9.3Hz, 1H), 8.24 (brs, 1H), 12.32 (brs, I H).

### [Test Example 1]

### αvβ3 Protein binding test

The method of Davis et al. (Proc.Natl.Acad.Sci., 96(16), 9269(1999)) was partially modified and carried out.
By using Echistatin (available from SIGMA), Fluorescein Amine Labeling Kit for Peptides and Proteins (available from Pan Vera Corp., P2058) and Gel Filtration of Proteins Kit (P2101, PanVera Corp.), fluorescein isothiocyanate (hereinafter abbreviated to as FITC)-Echistatin was prepared. Then, with 137 µL, of Tris-buffer (10 mM Tris base, 1 mM CaCl₂, 1 mM MgCl₂, 1 mM MnCl₂, pH 7.4) were mixed 1 µL of 200 nM FITC-Echistatin (final concentration: 1 nM) and 2 µL of 100 mg/mL bovine serum albumin (hereinafter abbreviated to as BSA; available from SIGMA) (final concentration: 1 mg/mL).
To the mixture was added 10 µL of the test compound, then, 50 µL of 4 nM αvβ3 (CC1019, available from CHEMICON CORP.) (final concentration: 1 nM) was added to the mixture and the resulting mixture was incubated at 37°C for 30 minutes. By measuring fluorescence polarization degrees with BEACON2000 (available from PanVera Corp.), binding of Echistatin and αvβ3 protein was evaluated. The concentration (IC₅₀ value) of the test compound necessary for inhibiting 50% of binding of Echistatin and αvβ3 protein was calculated by using EXSAS (available from Arm Systecs Co. Ltd.). The test results are shown in Table 1.

**[Table 1]**

| Test Compound Example No. | αvβ3 protein binding inhibition IC₅₀ value (nM) |
|---|---|
| Example 2 | 1.2 |
| Example 3 | 1.3 |
| Example 4 | 0.6 |
| Compund A | 7.1 |
| Compund B | 2.6 |

Among the compounds to be tested, Compound A is 8-[2-[6-(methylamino)-pyridin-2-yl]-1-ethoxy]-3-oxo-2-(2,2,2-trifluoroethyl)-2,3,4,5-tetrahydro-1H-2-benzazepin-4-acetic acid which is a racemic material of the compound of Example 40 of WO 98/14192A, and compound B is (4S)-3-oxo-8-[3-(pyridin-2-ylamino)-1-propyloxy]-2-(2,2,2-trifluoroethyl)-2,3,4,5-tetrahydro-1H-2-benzazepin-4-acetic acid which is a compound of Example 37 of the above-mentioned literature. Compounds A and B are control compounds having αvβ3 and αvβ5 inhibitory activity.

In the present test, the compounds of the present invention showed excellent αvβ3 inhibitory activity as compared with the control compounds.

### [Test Example 2]

### αvβ3 Expressing cell adhesion test

The method of Juliano, et al. (Exp.Cell Res., 225(1), 132 (1996)) was partially modified and carried out.
Human vitronectin (available from CHEMICON CORP.) was adjusted to 10 µg/mL with phosphate buffered saline (hereinafter abbreviated to as PBS). This was apportioned on a 96-well plate (MS9586F, available from SUMIRON Co., Ltd.) with 100 µL/well, subjected to coating at room temperature for 1 hour, then, the coating solution was removed and dried. Thereafter, blocking treatment was carried out by 1.0% BSA dissolved in Dulbecco's Modified Eagle's Medium (hereinafter abbreviated to as DMEM) for 1 hour, followed by washing with PBS, and the obtained material was used for the adhesion test as a coating plate. HUVEC (human umbilical vein endothelial cell) (available from Clonetics Corp.) cultured in an endocerial cell basal medium EBM2 (available from Clonetics Corp.) containing growth factor EGM2 (available from Clonetics Corp.) and 10% fatal bovin serum (hereinafter abbreviated to as FBS) was peeled by using trypsin-ethylenediamine tetraacetic acid, washed by using PBS and recovered, and then, suspended in 50% human serum-containing DMEM to prepare a cell solution of HUVEC. To human vitronectin coating plates was added a 50% human serum-containing DMEM solution containing the HUVEC cell solution and the test compound each 50 µL/plate to start the adhesion test.
The adhesion test was carried out in a 5% carbon dioxide gas incubator (37°C) for 1 hour. After completion of the adhesion test, a supernatant was removed by suction, the plate was washed with PBS, and Crystal Violet stain was carried out to measure the number of cells. The concentration (IC₅₀ value) of the test compound necessary for inhibiting 50% of binding to human vitronectin was calculated by using EXSAS (available from Arm Systecs Co., Ltd.). The test results are shown in Table 2.

**[Table 2]**

| Test Compound Example No. | αvβ3 developed cell adhesion inhibition IC₅₀ value (nM) |
|---|---|
| Example 2 | 61 |
| Compund A | 161 |

In the present test, the compound of the present invention showed excellent αvβ3 expressing cell adhesion-inhibitory activity as compared with that of the control compound.

### [Test Example 3]

### αvβ5 Protein binding test

The method of C.P Carron et al. (J.Endocrinology, 165, 587(2000)) was partially modified and carried out.
By using Echistatin (available from Bachem) and EZ-Link NHS-Biotin Reagent (available from Pierce Biotechnology Inc.), Biotin-Echistatin was prepared. Then, αvβ5 (CC1023, available from CHEMICON Corp.) was mixed with a Tris buffer solution (20 mM tris(hydroxymethyl)aminomethane, 150 mM NaCl, 1 mM CaCl₂, 1 mM MgCl₂, 1 mM MnCl₂, pH 7.5), the mixture was added to Nunc immunoplate Maxisoap (available from Nalge Nunc Int. Co.), and incubated at room temperature overnight. The solution was removed, a Block Ace (available from Dai Nippon Sumitomo Pharma Co., Ltd.) solution was added to the residue and the mixture was incubated at room temperature for 2 hours, then, the solution was again removed and the residue was washed with 0.05% Tween-PBS. Next, a Tris buffer solution containing 10 mg/mL of bovine serum albumin (hereinafter abbreviated to as BSA; available from SIGMA) and the test compound was added to the mixture, and the resulting mixture was incubated at room temperature for 30 minutes. Subsequently, a Tris buffer solution containing 10 mg/mL of BSA and 250 ng/mL of Biotin-Echistatin was added to the mixture, and the resulting mixture was incubated at room temperature for 1 hour, then, the solution was removed and the residue was washed with 0.05% Tween-PBS. Moreover, 0.025 µg/mL of an avidin-peroxidase (Neutr Avidin^{™} Horseradish Peroxidase Conjugated, available from Pierce Biotechnology Inc.) solution was added to the mixture, the resulting mixture was incubated at room temperature for 1 hour, then, the solution was removed and the residue was washed with 0.05% Tween-PBS. Finally, a substrate solution containing o-phenylenediamine (available from SIGMA) was added thereto to start the reaction. After incubation at room temperature under shielding light for 15 minutes, the reaction was stopped by adding 1N sulfuric acid. An absorbance at 495 nm was measured by Microplate reader (Spectra Fluor, available from TECAN Group, Ltd.) to evaluate binding of Echistatin and αvβ5 protein. The concentration (IC₅₀ value) of the test compound necessary for inhibiting 50% of binding between Echistatin and αvβ5 protein was calculated by using EXSAS (available from Arm Systecs Co., Ltd.). The test results are shown in Table 3.

[Table 3]

**Table 3**

| Test Compound Example No. | αvβ5 protein binding inhibition IC₅₀ value (nM) |
|---|---|
| Example 4 | 0.2 |
| Compund B | 1.3 |

In the present test, the compound of the present invention showed excellent αvβ5 inhibitory activity as compared with that of the control compound.

### [Test Example 4]

### Matrigel angiogenesis test

Under ice-cooling, bFGF (basic fibroblast growth factor; available from PEPRO TECH Inc.) was mixed with a liquid state Matrigel (available from BD Bioscience Inc.) so that the final concentration became 4 µg/mL. To C57BL mice (male, 8-weeks old, supplied by Charles River Laboratories Japan Ltd.) grouped by the weight (5 mice per group) were injected each 0.5 mL of Matrigel at 2 portions of the right and left abdomen subcutaneously under Fluothane inhalation anethesia to start administration of the test compound. The test compound was dissolved in distilled water for injection, and orally administered compulsorily twice a day with an administration amount of 10 mL/kg with 10 mg/kg, 30 mg/kg and 100 mg/kg for 4 days. In control group (bFGF-non-treated Matrigel injected, no test compound-administered group) and Vehicle group (bFGF-treated Matrigel injected, no test compound-administered group), distilled water for injection was administered in the same manner. On the next day of the final administration, the mice were administered with 5 mL/kg of FITC-dextran (available from SIGMA) dissolved in a physiological saline solution in an amount of 40 mg/mL via jagular vein under Fluothane inhalation anesthesia. After 20 minutes, Matrigel was excised from the mice, and after measuring the weight of the excised Matrigel, it was crushed with 1 mL of a physiological saline solution containing 0.5% BSA by a tissue cell crushing apparatus (FastPrep FP100A Instrument, available from Qbiogene Inc.) to recover the supernatant. An amount of the FITC-dextran contained in the supernatant was measured by a Microplate reader (Spectra Fluor, available from TECAN Group, Ltd.). The amount of the FITC-dextran extracted from the excised Matrigel was made a blood amount in the newborn blood vessel to evaluate an angiogenetic inhibition activity. As the test compound, the compound of Example 1 of the present invention was used. The test results are shown in Fig. 1.

In this test, the compound of the present invention showed excellent angiogenetic inhibition activity

### [Test Example 5]

### VEGF-induced HUVEC proliferation test

The method of HARA, et al. (Evidence-based Complementary and Alternative Medicine, 6(4), 489-494(2009)) was partially modified and carried out.
Cultured HUVEC (human umbilical vein endothelial cells) (available from Cell Applications, Inc.) were suspended in Humedia EG2 medium (available from KURABO Industries, Ltd.), seeded on a 96-well plate, and cultured in a 5% carbon dioxide gas incubator (37°C) for 1 day After culturing, the medium was replaced with a 2% FBS-added Humedia EB2 medium (available from KURABO Industries, Ltd.), and further cultured in a 5% carbon dioxide gas incubator (37°C) for 6 hours. The test compounds adjusted to the respective concentrations were added, and then, Recombinant Human VEGF₁₆₅ (available from R&D Systems, Inc.) was so added that the final concentration became 30 ng/mL, and cultured in a 5% carbon dioxide gas incubator (37°C) for 3 days.
After culturing for 3 days, Cell Counting Kit-8 (available from DOJINDO Inc.) was added with 20 µL/well, and cultured in a 5% carbon dioxide gas incubator (37°C) for 4 hours. To 100 µL of culture medium of each well was added 100 µL of Dulbecco's Phosphate Buffered Saline (available from SIGMA). Absorbance (450 nm) of each well was measured by a Microplate reader (infinite F200, available from TECAN Group, Ltd.). A cell proliferation inhibitory effect was evaluated from the amount of WST-8 formazan formed in proportion to the number of living cells. The concentration (IC₅₀ value) of the test compound necessary for inhibiting 50% of proliferation of HUVEC by VEGF was calculated by using EXSAS (available from Arm Systecs Co., Ltd.). As the test compound, the compound of Example 4 of the present invention was used.

In the present test, the compound of the present invention showed excellent VEGF-induced HUVEC proliferation inhibitory effect.

### [Test Example 6]

### VEGF-induced HUVEC migration test

According to the protocol of BD BioCoat^{™} Angiogenesis System: Endothelial Cell Migration (available from BD Biosciences), the experiment was carried out.
HUVEC (human umbilical vein endothelial cells) (available from Cell Applications, Inc.) was cultured in 0.1% BSA-containing Medium 200S (available from Cascade Biologics, Inc.), and then HUVEC was prepared as cell suspension. To the cell suspension were added the test compound adjusted to the respective concentrations to prepare the compound-added cell suspensions. To 0.1% BSA-containing Medium 200S was so added Recombinant Human VEGF₁₆₅ (available from R&D Systems, Inc.) that the final concentration thereof became 10 ng/mL, and then, the test compound adjusted to the respective concentrations was each added thereto to prepare the compound VEGF-added Medium. To Top chamber of BD BioCoat^{™} Angiogenesis System: Endothelial Cell Migration 24-well insert plate was so added the compound-added cell suspension that it became 250 µL/well, and after adding the compound VEGF-added Medium to Bottom chamber so that it became 750 µL/well, it was cultured in a 5% carbon dioxide gas incubator (37°C) for 1 day.
To 24-well plate was added 500 µL/well of a Calcein AM (available from Invitrogen Corp.) solution which had been adjusted to 4 ng/mL, the Top chamber in which Medium had been removed was set, and cultured in a 5% carbon dioxide gas incubator (37°C) for 90 minutes. Fluorescence intensity (Ex/Em 485/535) of each well was measured by using a Microplate reader (infinite F200, available from TECAN Group, Ltd.), and a cell migration inhibitory effect was evaluated from the amount of the formed Calcein. The concentration (IC₅₀ value) of the test compound necessary for inhibiting 50% of migration of HUVEC by VEGF was calculated by using EXSAS (available from Arm Systecs Co., Ltd.). As the test compound, the compound of Example 4 of the present invention was used.

In the present test, the compound of the present invention showed excellent VEGF-induced HUVEC migration inhibitory effect.

The compounds of the present invention have potent integrin αvβ3 and αvβ5 protein binding-inhibitory activity, and further have cell adhesion-inhibitory activity, angiogenesis inhibitory activity, etc., so that they are useful as a medicine for treatment or prophylaxis of diseases caused by ocular angiogenesis.

### Preparation example

### (Preparation example 1) (Hard capsules)

50 mg of the compound of Example 1, 128.7 mg of lactose, 70 mg of cellulose and 1.3 mg of magnesium stearate were mixed, the mixture was passed through 60 mesh sieve, and then, 250 mg of the powder was placed in No. 3 gelatin capsule to prepare a capsule.

### (Preparation example 2) (Tablets)

50 mg of the compound of Example 1, 124 mg of lactose, 25 mg of cellulose and 1 mg of magnesium stearate were mixed, the mixture was tableted by a tableting machine to prepare a tablet with 200 mg per one tablet. Sugar coating can be applied to this tablet, if necessary.

### UTILIZABILITY IN INDUSTRY

The medical composition of the present invention comprising a benzazepinone compound represented by the formula (1) or a pharmaceutically acceptable salt thereof as an effective ingredient has excellent angiogenesis inhibitory activity such to excellent αv integrin receptor (in particular αvβ3 and αvβ5) antagonistic action, and has excellent properties in the points of excellent solubility, oral absorbability, concentration in blood, metabolic stability, tissue distribution, bioavailability (BA), *in vitro* activity, *in vivo* activity, fast-acting pharmaceutical effect, sustained pharmaceutical effect, physical stability, drug interaction, toxicity, etc., and has high safety, so that it is useful as a medicine (in particular, a medicine for treatment or prophylaxis of diseases caused by ocular angiogenesis) for warm blooded one (in particular, for human).

## Claims

1. A medical composition for use in the treatment or prophylaxis of diseases caused by angiogenesis of eyes which comprises a compound represented by the following formula (I): 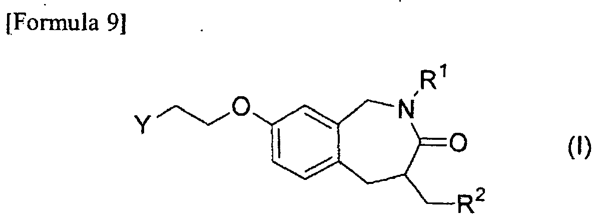 wherein R¹ represents a C₁-C₆ alkyl group or halogeno-C₁-C₆ alkyl group, R² represents a carboxyl group which may be protected, Y represents a group represented by the formula (II): 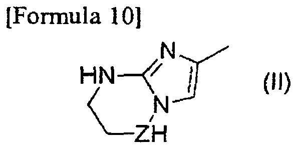 wherein Z represents CH or nitrogen atom,
or a pharmaceutically acceptable salt thereof as an active ingredient.

2. The medical composition for use according to Claim 1, wherein R¹ is a C₁-C₄ alkyl group, fluoro-C₁-C₄ alkyl group or chloro-C₁-C₄ alkyl group.

3. The medical composition for use according to Claim 1, wherein R¹ is an ethyl group, propyl group, isopropyl group, butyl group, isobutyl group, sec-butyl group, difluoromethyl group, trifluoromethyl group, 2-fluoroethyl group, 2,2-difluoroethyl group, 2,2,2-trifluoroethyl group or 2,2,2-trichloroethyl group.

4. The medical composition for use according to Claim 1, wherein R¹ is an ethyl group, difluoromethyl group, trifluoromethyl group, 2-fluoroethyl group, 2,2-difluoroethyl group or 2,2,2-trifluoroethyl group.

5. The medical composition for use according to Claim 1, wherein R¹ is an ethyl group, trifluoromethyl group or 2,2,2-trifluoroethyl group.

6. The medical composition for use according to Claim 1, wherein R² is a carboxyl group which may be protected by a C₁-C₁₂ alkyl group, C₇-C₁₈ aralkyl group, C₁-C₂ alkyl group substituted by C₂-C₅ alkanoyloxy group, C₁-C₂ alkyl group substituted by (C₁-C₄ alkoxy)carbonyloxy group, N,N-dimethylaminocarbonylmethyl group, 2-(morpholin-4-yl)ethyl group, (5-methyl-2-oxo-1,3-dioxolen-4-yl)methyl group or (5-phenyl-2-oxo-1,3-dioxolen-4-yl)methyl group.

7. The medical composition for use according to Claim I, wherein R² is a carboxyl group which may be protected by a methyl group, ethyl group, propyl group, isopropyl group, 1-ethylpropyl group, butyl group, isobutyl group, sec-butyl group, tert-butyl group, 3,3-dimethylbutyl group, pentyl group, isopentyl group, neopentyl group, tert-pentyl group, 1-methylbutyl group, hexyl group, 1-methylpentyl group, 2-methylpentyl group, 3-methylpentyl group, 1-ethylbutyl group, 2-ethylbutyl group, heptyl group, octyl group, nonyl group, decyl group, undecyl group, dodecyl group, benzyl group, phenethyl group, phenylpropyl group, phenylbutyl group, phenylpentyl group, phenylhexyl group, phenylheptyl group, phenyloctyl group, phenylnonyl group, phenyldecyl group, phenylundecyl group, phenyldodecyl group, acetoxymethyl group, 1-acetoxyethyl group, pivaloyloxymethyl group, 1-pivaloyloxyethyl group, ethoxycarbonyloxymethyl group, 1-ethoxycarbonyloxyethyl group, N,N-dimethylaminocarbonylmethyl group, 2-(morpholin-4-yl)ethyl group or (5-methyl-2-oxo-1,3-dioxolen-4-yl)methyl group.

8. The medical composition for use according to Claim 1, wherein R² is a carboxyl group which may be protected by a methyl group, ethyl group, propyl group, isopropyl group, 1-ethylpropyl group, butyl group, 3,3-dimethylbutyl group, pentyl group, hexyl group, heptyl group, octyl group, nonyl group, decyl group, undecyl group, dodecyl group, benzyl group, phenethyl group, phenylpropyl group, phenylbutyl group, phenylpentyl group, phenylhexyl group, phenylheptyl group, phenyloctyl group, phenylnonyl group, phenyldecyl group, phenylundecyl group, phenyldodecyl group, acetoxymethyl group, pivaloyloxymethyl group, 1-pivaloyloxyethyl group, N,N-dimethylaminocarbonylmethyl group, 2-(morpholin-4-yl)ethyl group or (5-methyl-2-oxo-1,3-dioxolen-4-yl)methyl group.

9. The medical composition for use according to Claim 1, wherein R² is a carboxyl group which may be protected by an ethyl group, propyl group, isopropyl group, butyl group, pentyl group, hexyl group, heptyl group, octyl group, nonyl group, decyl group, undecyl group, benzyl group, 2-phenethyl group, 3-phenylpropyl group, 4-phenylbutyl group, 5-phenylpentyl group, 6-phenylhexyl group, 7-phenylheptyl group, 8-phenyloctyl group, 9-phenylnonyl group, 10-phenyldecyl group, pivaloyloxymethyl group, N,N-dimethylaminocarbonylmethyl group, 2-(morpholin-4-yl)ethyl group or (5-methyl-2-oxo-1,3-dioxolen-4-yl)methyl group.

10. The medical composition for use according to Claim 1, wherein R¹ is a C₁-C₄ alkyl group, fluoro-C₁-C₄ alkyl group or chloro-C₁-C₄ alkyl group, and
R² is a carboxyl group which may be protected by a C₁-C₁₂ alkyl group, C₇-C₁₈ aralkyl group, C₁-C₂ alkyl group substituted by C₂-C₅ alkanoyloxy group, C₁-C₂ alkyl group substituted by (C₁-C₄ alkoxy)carbonyloxy group, N,N-dimethylaminocarbonylmethyl group, 2-(morpholin-4-yl)ethyl group, (5-methyl-2-oxo-1,3-dioxolen-4-yl)methyl group or (5-phenyl-2-oxo-1,3-dioxolen-4-yl)methyl group.

11. The medical compositio for use according to Claim 1, wherein R¹ is an ethyl group, propyl group, isopropyl group, butyl group, isobutyl group, sec-butyl group, difluoromethyl group, trifluoromethyl group, 2-fluoroethyl group, 2,2-difluoroethyl group, 2,2,2-trifluoroethyl group or 2,2,2-trichloroethyl group, and
R² is a carboxyl group which may be protected by a methyl group, ethyl group, propyl group, isopropyl group, 1-ethylpropyl group, butyl group, isobutyl group, sec-butyl group, tert-butyl group, 3,3-dimethylbutyl group, pentyl group, isopentyl group, neopentyl group, tert-pentyl group, 1-methylbutyl group, hexyl group, 1-methylpentyl group, 2-methylpentyl group, 3-methylpentyl group, 1-ethylbutyl group, 2-ethylbutyl group, heptyl group, octyl group, nonyl group, decyl group, undecyl group, dodecyl group, benzyl group, phenethyl group, phenylpropyl group, phenylbutyl group, phenylpentyl group, phenylhexyl group, phenylheptyl group, phenyloctyl group, phenylnonyl group, phenyldecyl group, phenylundecyl group, phenyldodecyl group, acetoxymethyl group, 1-acetoxyethyl group, pivaloyloxymethyl group, 1-pivaloyloxyethyl group, ethoxycarbonyloxymethyl group, 1-ethoxycarbonyloxyethyl group, N,N-dimethylaminocarbonylmethyl group, 2-(morpholin-4-yl)ethyl group or (5-methyl-2-oxo-1,3-dioxolen-4-yl)methyl group.

12. The medical composition for use accordmg to Claim 1, wherein R¹ is an ethyl group, difluoromethyl group, trifluoromethyl group, 2-fluoroethyl group, 2,2-difluoroethyl group or 2,2,2-trifluoroethyl group, and
R² is a carboxyl group which may be protected by a methyl group, ethyl group, propyl group, isopropyl group, 1-ethylpropyl group, butyl group, 3,3-dimethylbutyl group, pentyl group, hexyl group, heptyl group, octyl group, nonyl group, decyl group, undecyl group, dodecyl group, benzyl group, phenethyl group, phenylpropyl group, phenylbutyl group, phenylpentyl group, phenylhexyl group, phenylheptyl group, phenyloctyl group, phenylnonyl group, phenyldecyl group, phenylundecyl group, phenyldodecyl group, acetoxymethyl group, pivaloyloxymethyl group, 1-pivaloyloxyethyl group, N,N-dimethylaminocarbonylmethyl group, 2-(morpholin-4-yl)ethyl group or (5-methyl-2-oxo-1,3-dioxolen-4-yl)methyl group.

13. The medical composition for use according to Claim 1, wherein R¹ is an ethyl group, trifluoromethyl group or 2,2,2-trifluoroethyl group, and
R² is a carboxyl group which may be protected by an ethyl group, propyl group, isopropyl group, butyl group, pentyl group, hexyl group, heptyl group, octyl group, nonyl group, decyl group, undecyl group, benzyl group, 2-phenethyl group, 3-phenylpropyl group, 4-phenylbutyl group, 5-phenylpentyl group, 6-phenylhexyl group, 7-phenylheptyl group, 8-phenyloctyl group, 9-phenylnonyl group, 10-phenyldecyl group, pivaloyloxymethyl group, N,N-dimethylaminocarbonylmethyl group, 2-(morpholin-4-yl)ethyl group or (5-methyl-2-oxo-1,3-dioxolen-4-yl)methyl group.

14. The medical composition for use according to Claim 1, wherein the compound is represented by the following formula (III): 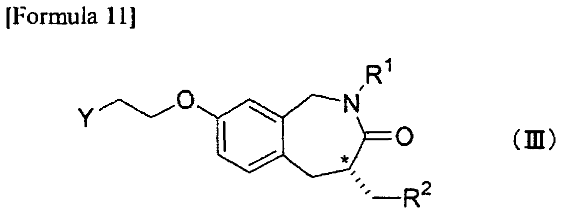 wherein the carbon atom to which * is attached has a configuration (S), R¹, R² and Y have the same meanings as defined above.

15. The medical composition for use according to any one of Claims 2 to 13, wherein the compound is represented by the formula (III).

16. The medical composition for use according to Claim 1, wherein the benzazepinone compound is
3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetic acid,
(4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetic acid,
ethyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
ethyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
propyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
propyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
isopropyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
isopropyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
heptyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
heptyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
undecyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
undecyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
benzyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
benzyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
(10-phenyl)decyl 3-oxo-8-[2-(5,6,7,8-tetrahydroimidazo[1,2-a]pyrimidin-2-yl)-ethoxy]-2-(2,2,2-trifluoroethyl)-2,3,4,5-tetrahydro-1H-2-benzazepin-4-acetate,
(10-phenyl)decyl (4S)-3-oxo-8-[2-(5,6,7,8-tetrahydroimidazo[1,2-a]pyrimidin-2-yl)-ethoxy]-2-(2,2,2-trifluoroethyl)-2,3,4,5-tetrahydro-1H-2-benzazepin-4-acetate,
pivaloyloxymethyl 3-oxo-8-[2-(5,6,7,8-tetrahydroimidazo[1,2-a]pyrimidin-2-yl)-ethoxy]-2-(2,2,2-trifluoroethyl)-2,3,4,5-tetrahydro-1H-2-benzazepin-4-acetate,
pivaloyloxymethyl (4S)-3-oxo-8-[2-(5,6,7,8-tetrahydroimidazo[1,2-a]pyrimidin-2-yl)-ethoxy]-2-(2,2,2-trifluoroethyl)-2,3,4,5-tetrahydro-1H-2-benzazepin-4-acetate,
(N,N-dimethylaminocarbonyl)methyl 3-oxo-8-[2-(5,6,7,8-tetrahydroimidazo[1,2-a]-pyrimidin-2-yl)ethoxy]-2-(2,2,2-trifluoroethyl)-2,3,4,5-tetrahydro-1H-2-benzazepin-4-acetate,
(N,N-dimethylaminocarbonyl)methyl (4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
[2-(morpholin-4-yl)]ethyl 3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
[2-(morpholin-4-yl)]ethyl(4S)-3-oxo-8-[2-(5,6,7,8-tetrahydroimidazo[1,2-a]pyrimidin-2-yl)ethoxy]-2-(2,2,2-trifluoroethyl)-2,3,4,5-tetrahydro-1H-2-benzazepin-4-acetate,
(5-methyl-2-oxo-[1,3]dioxolen-4-yl)methyl 3-oxo-8-[2-(5,6,7,8-tetrahydroimidazo-[1,2-a]pyrimidin-2-yl)ethoxy]-2-(2,2,2-trifluoroethyl)-2,3,4,5-tetrahydro-1H-2-benzazepin-4-acetate
(5-methyl-2-oxo-[1,3]dioxolen-4-yl)methyl (4S)-3-oxo-8-[2-(5,6,7,8-tetrahydro-imidazo[1,2-a]pyrimidin-2-yl)ethoxy]-2-(2,2,2-trifluoroethyl)-2,3,4,5-tetrahydro-1H-2-benzazepin-4-acetate,
3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetic acid,
(4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetic acid,
ethyl 3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
ethyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
propyl 3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
propyl (4S)-3-oxo-8-[2-(1,2,3,4-tetrahydroimidazo[1,2-b][1,2-,4]triazin-6-yl)ethoxy]-2-(2,2,2-trifluoroethyl)-2,3,4,5-tetrahydro-H-2-benzazepin-4-acetate,
isopropyl 3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
isopropyl (4S)-3-oxo-8-[2-(1,2,3,4-tetrahydroimidazo[1,2-b][1,2,4]triazin-6-yl)-ethoxy]-2-(2,2,2-trifluoroethyl)-2,3,4,5-tetrahydro-1H-2-benzazepin-4-acetate,
heptyl 3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
heptyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
undecyl 3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
undecyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
benzyl 3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
benzyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
(10-phenyl)decyl 3-oxo-8-[2-(1,2,3,4-tetrahydroimidazo[1,2-b][1,2,4]triazin-6-yl)-ethoxy]-2-(2,2,2-trifluoroethyl)-2,3,4,5-tetrahydro-1H-2-benzazepin-4-acetate,
(10-phenyl)decyl(4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
pivaloyloxymethyl 3-oxo-8-[2-(1,2,3,4-tetrahydroimidazo[1,2-b][1,2,4]triazin-6-yl)-ethoxy]-2-(2,2,2-trifluoroethyl)-2,3,4,5-tetrahydro-1H-2-benzazepin-4-acetate,
pivaloyloxymethyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
(N,N-dimethylaminocarbonyl)methyl 3-oxo-8-[2-(1,2,3,4-tetrahydroimidazo[1,2-b]-[1,2,4]triazin-6-yl)ethoxy]-2-(2,2,2-trifluoroethyl)-2,3,4,5-tetrahydro-1H-2-benz-azepin-4-acetate,
(N,N-dimethylaminocarbonyl)methyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
[2-(morpholin-4-yl)]ethyl 3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1H-2-benzazepin-4-acetate,
[2-(morpholin-4-yl)]ethyl(4S)-3-oxo-8-[2-(1,2,3,4-tetrahydroimidazo[1,2-b][1,2,4]-triazin-6-yl)ethoxy]-2-(2,2,2-trifluoroethyl)-2,3,4,5-tetrahydro-1H-2-benzazepin-4-acetate,
(5-methyl-2-oxo-[1,3]dioxolen-4-yl)methyl 3-oxo-8-[2-(1,2,3,4-tetrahydroimidazo-[1,2-b][1,2,4]triazin-6-yl)ethoxy]-2-(2,2,2-trifluoroethyl)-2,3,4,5-tetrahydro-1H-2-benzazepin-4-acetate, or
(5-methyl-2-oxo-[1,3]dioxolen-4-yl)methyl (4S)-3-oxo-8-[2-(1,2,3,4-tetrahydroimidazo[1,2-b][1,2,4]triazin-6-yl)ethoxy]-2-(2,2,2-trifluoroethyl)-2,3,4,5-tetrahydro-1 H-2-benzazepin-4-acetate.

17. The medical composition for use according to any one of Claims 1 to 16, wherein administration form is oral administration.

18. The medical composition for use according to any one of Claims 1 to 16, wherein the eye disease is intraocular neovascular disease.

19. The medical composition for use according to any one of Claims 1 to 16, wherein the eye disease is an age-related macular degeneration.

## Patentansprüche

1. Arzneimittelzusammensetzung zur Verwendung bei der Behandlung oder Prophylaxe von Erkrankungen, die durch Angiogenese der Augen verursacht werden, umfassend eine Verbindung der nachstehenden Formel (I) : 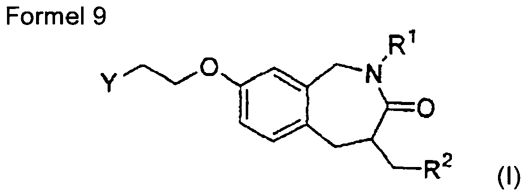 worin R¹ eine C₁₋₆-Alkylgruppe oder eine Halogen-C₁₋₆-alkyl-Gruppe darstellt, R² eine Carboxylgruppe darstellt, die geschützt sein kann, Y eine Gruppe der Formel (II) darstellt: 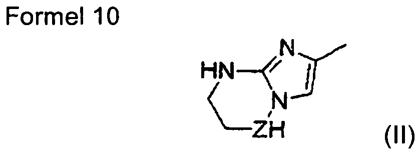 worin Z CH oder ein Stickstoffatom darstellt,
oder ein pharmazeutisch annehmbares Salz davon, als Wirkstoff.

2. Arzneimittelzusammensetzung zur Verwendung gemäss Anspruch 1, worin R¹ eine C₁₋₄-Alkylgruppe, Fluor-C₁₋₄-alkyl-Gruppe oder Chlor-C₁₋₄-alkyl-Gruppe ist.

3. Arzneimittelzusammensetzung zur Verwendung gemäss Anspruch 1, worin R¹ eine Ethylgruppe, Propylgruppe, Isopropylgruppe, Butylgruppe, Isobutylgruppe, sek-Butylgruppe, Difluormethylgruppe, Trifluormethylgruppe, 2-Fluorethylgruppe, 2,2-Difluorethylgruppe, 2,2,2-Trifluorethylgruppe oder 2,2,2-Trichlorethylgruppe ist.

4. Arzneimittelzusammensetzung zur Verwendung gemäss Anspruch 1, worin R¹ eine Ethylgruppe, Difluormethylgruppe, Trifluormethylgruppe, 2-Fluorethylgruppe, 2,2-Difluorethylgruppe oder 2,2,2-Trifluorethylgruppe ist.

5. Arzneimittelzusammensetzung zur Verwendung gemäss Anspruch 1, worin R¹ eine Ethylgruppe, Trifluormethylgruppe oder 2,2,2-Trifluorethylgruppe ist.

6. Arzneimittelzusammensetzung zur Verwendung gemäss Anspruch 1, worin R² eine Carboxylgruppe ist, die durch eine C₁₋₁₂-Alkylgruppe, eine C₇₋₁₈-Aralkylgruppe, eine mit einer C₂₋₅-Alkanoyloxygruppe substituierte C₁₋₂-Alkylgruppe, eine mit einer (C₁₋₄-Alkoxy)carbonyloxy-Gruppe substituierte C₁₋₂-Alkylgruppe, eine N,N-Dimethylaminocarbonylmethylgruppe, eine 2-(Morpholin-4-yl)ethyl-Gruppe, eine (5-Methyl-2-oxo-1,3-dioxolen-4-yl)methyl-Gruppe oder eine (5-Phenyl-2-oxo-1,3-dioxolen-4-yl)methylGruppe geschützt sein kann.

7. Arzneimittelzusammensetzung zur Verwendung gemäss Anspruch 1, worin R² eine Carboxylgruppe ist, die durch eine Methylgruppe, Ethylgruppe, Propylgruppe, Isopropylgruppe, 1-Ethylpropylgruppe, Butylgruppe, Isobutylgruppe, sek-Butylgruppe, tert-Butylgruppe, 3,3-Dimethylbutylgruppe, Pentylgruppe,
Isopentylgruppe, Neopentylgruppe, tert-Pentylgruppe, 1-Methylbutylgruppe, Hexylgruppe, 1-Methylpentylgruppe, 2-Methylpentylgruppe, 3-Methylpentylgruppe, 1-Ethylbutylgruppe, 2-Ethylbutylgruppe, Heptylgruppe, Octylgruppe, Nonylgruppe, Decylgruppe, Undecylgruppe, Dodecylgruppe, Benzylgruppe, Phenethylgruppe, Phenylpropylgruppe, Phenylbutylgruppe, Phenylpentylgruppe, Phenylhexylgruppe, Phenylheptylgruppe, Phenyloctylgruppe, Phenylnonylgruppe, Phenyldecylgruppe, Phenylundecylgruppe, Phenyldodecylgruppe, Acetoxymethylgruppe, 1-Acetoxyethylgruppe, Pivaloyloxymethylgruppe, 1-Pivaloyloxyethylgruppe, Ethoxycarbonyloxymethylgruppe, 1-Ethoxycarbonyloxyethylgruppe, N,N-Dimethylaminocarbonylmethylgruppe, 2-(Morpholin-4-yl)ethyl-Gruppe oder (5-Methyl-2-oxo-1,3-dioxolen-4-yl)methyl-Gruppe geschützt sein kann.

8. Arzneimittelzusammensetzung zur Verwendung gemäss Anspruch 1, worin R² eine Carboxylgruppe ist, die durch eine Methylgruppe, Ethylgruppe, Propylgruppe, Isopropylgruppe, 1-Ethylpropylgruppe, Butylgruppe, 3,3-Dimethylbutylgruppe, Pentylgruppe, Hexylgruppe, Heptylgruppe, Octylgruppe, Nonylgruppe, Decylgruppe, Undecylgruppe, Dodecylgruppe, Benzylgruppe, Phenethylgruppe, Phenylpropylgruppe, Phenylbutylgruppe, Phenylpentylgruppe, Phenylhexylgruppe, Phenylheptylgruppe, Phenyloctylgruppe, Phenylnonylgruppe, Phenyldecylgruppe, Phenylundecylgruppe, Phenyldodecylgruppe, Acetoxymethylgruppe, Pivaloyloxymethylgruppe, 1-Pivaloyloxyethylgruppe, N,N-Dimethylaminocarbonylmethylgruppe, 2-(Morpholin-4-yl)ethyl-Gruppe oder (5-Methyl-2-oxo-1,3-dioxolen-4-yl)methyl-Gruppe geschützt sein kann.

9. Arzneimittelzusammensetzung zur Verwendung gemäss Anspruch 1, worin R² eine Carboxylgruppe ist, die durch eine Ethylgruppe, Propylgruppe, Isopropylgruppe, Butylgruppe, Pentylgruppe, Hexylgruppe, Heptylgruppe, Octylgruppe, Nonylgruppe, Decylgruppe, Undecylgruppe, Benzylgruppe, 2-Phenethylgruppe, 3-Phenylpropylgruppe, 4-Phenylbutylgruppe, 5-Phenylpentylgruppe, 6-Phenylhexylgruppe, 7-Phenylheptylgruppe, 8-Phenyloctylgruppe, 9-Phenylnonylgruppe, 10-Phenyldecylgruppe, Pivaloyloxymethylgruppe, N,N-Dimethylaminocarbonylmethylgruppe, 2-(Morpholin-4-yl)ethyl-Gruppe oder (5-Methyl-2-oxo-1,3-dioxolen-4-yl)methyl-Gruppe geschützt sein kann.

10. Arzneimittelzusammensetzung zur Verwendung gemäss Anspruch 1, worin
R¹ eine C₁₋₄-Alkylgruppe, Fluor-C₁₋₄-alkyl-Gruppe oder Chlor-C₁₋₄-alkyl-Gruppe ist und
R² eine Carboxylgruppe ist, die durch eine C₁₋₁₂-Alkylgruppe, C₇₋₁₈-Aralkylgruppe, eine mit einer C₂₋₅₋Alkanoyloxygruppe substituierte C₁₋₂-Alkylgruppe, eine mit einer (C₁₋₄-Alkoxy)carbonyloxy-Gruppe substituierte C₁₋₂-Alkylgruppe, eine N,N-Dimethylaminocarbonylmethylgruppe, 2-(Morpholin-4-yl)ethyl-Gruppe, (5-Methyl-2-oxo-1,3-dioxolen-4-yl)methyl-Gruppe oder (5-Phenyl-2-oxo-1,3-dioxolen-4-yl)methyl-Gruppe geschützt sein kann.

11. Arzneimittelzusammensetzung zur Verwendung gemäss Anspruch 1, worin
R¹ eine Ethylgruppe, Propylgruppe, Isopropylgruppe, Butylgruppe, Isobutylgruppe, sek-Butylgruppe, Difluormethylgruppe, Trifluormethylgruppe, 2-Fluorethylgruppe, 2,2-Difluorethylgruppe, 2,2,2-Trifluorethylgruppe oder 2,2,2-Trifluorethylgruppe ist, und
R² eine Carboxylgruppe ist, die durch eine Methylgruppe, Ethylgruppe, Propylgruppe, Isopropylgruppe, 1-Ethylpropylgruppe, Butylgruppe, Isobutylgruppe, sek-Butylgruppe, tert-Butylgruppe, 3,3-Dimethylbutylgruppe, Pentylgruppe, Isopentylgruppe, Neopentylgruppe, tert-Pentylgruppe, 1-Methylbutylgruppe, Hexylgruppe, 1-Methylpentylgruppe, 2-Methylpentylgruppe, 3-Methylpentylgruppe, 1-Ethylbutylgruppe, 2-Ethylbutylgruppe, Heptylgruppe, Octylgruppe, Nonylgruppe, Decylgruppe, Undecylgruppe, Dodecylgruppe, Benzylgruppe, Phenethylgruppe, Phenylpropylgruppe, Phenylbutylgruppe, Phenylpentylgruppe, Phenylhexylgruppe, Phenylheptylgruppe, Phenyloctylgruppe, Phenylnonylgruppe, Phenyldecylgruppe, Phenylundecylgruppe, Phenyldodecylgruppe, Acetoxymethylgruppe, 1-Acetoxyethylgruppe, Pivaloyloxymethylgruppe, 1-Pivaloyloxyethylgruppe, Ethoxycarbonyloxymethylgruppe, 1-Ethoxycarbonyloxyethylgruppe, N,N-Dimethylaminocarbonylmethylgruppe, 2-(Morpholin-4-yl)ethyl-Gruppe oder (5-Methyl-2-oxo-1,3-dioxolen-4-yl)methyl-Gruppe geschützt sein kann.

12. Arzneimittelzusammensetzung zur Verwendung gemäss Anspruch 1, worin
R¹ eine Ethylgruppe, Difluormethylgruppe, Trifluormethylgruppe, 2-Fluorethylgruppe, 2,2-Difluorethylgruppe oder 2,2,2-Trifluorethylgruppe ist, und
R² eine Carboxylgruppe ist, die durch eine Methylgruppe, Ethylgruppe, Propylgruppe, Isopropylgruppe, 1-Ethylpropylgruppe, Butylgruppe, 3,3-Dimethylbutylgruppe, Pentylgruppe, Hexylgruppe, Heptylgruppe, Octylgruppe, Nonylgruppe, Decylgruppe, Undecylgruppe, Dodecylgruppe, Benzylgruppe, Phenethylgruppe, Phenylpropylgruppe Phenylbutylgruppe, Phenylpentylgruppe, Phenylhexylgruppe, Phenylheptylgruppe, Phenyloctylgruppe, Phenylnonylgruppe, Phenyldecylgruppe, Phenylundecylgruppe, Phenyldodecylgruppe, Acetoxymethylgruppe, Pivaloyloxymethylgruppe, 1-Pivaloyloxyethylgruppe, N,N-Dimethylaminocarbonylmethylgruppe, 2-(Morpholin-4-yl)ethyl-Gruppe oder (5-Methyl-2-oxo-1,3-dioxolen-4-yl)methyl-Gruppe geschützt sein kann.

13. Arzneimittelzusammensetzung zur Verwendung gemäss Anspruch 1, worin
R¹ eine Ethylgruppe, Trifluormethylgruppe oder 2,2,2-Trifluorethylgruppe ist, und
R² eine Carboxylgruppe ist, die durch eine Ethylgruppe, Propylgruppe, Isopropylgruppe, Butylgruppe, Pentylgruppe, Hexylgruppe, Heptylgruppe, Octylgruppe, Nonylgruppe, Decylgruppe, Undecylgruppe, Benzylgruppe, 2-Phenethylgruppe, 3-Phenylpropylgruppe, 4-Phenylbutylgruppe, 5-Phenylpentylgruppe, 6-Phenylhexylgruppe, 7-Phenylheptylgruppe, 8-Phenyloctylgruppe, 9-Phenylnonylgruppe, 10-Phenyldecylgruppe, Pivaloyloxymethylgruppe, N,N-Dimethylaminocarbonylmethylgruppe, 2-(Morpholin-4-yl)ethyl-Gruppe oder (5-Methyl-2-oxo-1,3-dioxolen-4-yl)methyl-Gruppe geschützt sein kann.

14. Arzneimittelzusammensetzung zur Verwendung gemäss Anspruch 1, wobei die Verbindung durch die nachstehende Formel (III) dargestellt ist: 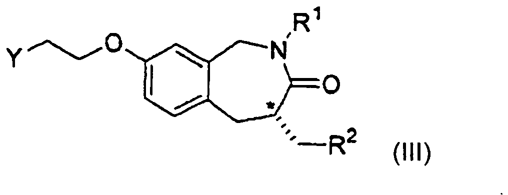 worin das Kohlenstoffatom, an das * angefügt ist, die Konfiguration (S) aufweist und R¹, R² und Y die gleichen Bedeutungen haben wie vorstehend definiert.

15. Arzneimittelzusammensetzung zur Verwendung gemäss irgendeinem der Ansprüche 2 bis 13, wobei die Verbindung durch die Formel (III) dargestellt ist.

16. Arzneimittelzusammensetzung zur Verwendung gemäss Anspruch 1, wobei die Benzazepinonverbindung ist:
3-Oxo-8-[2-(5,6,7,8-tetrahydroimidazo[1,2-a]-pyrimidin-2-yl)ethoxy]-2-(2,2,2-trifluorethyl)-2,3,4,5-tetrahydro-1H-2-benzazepin-4-essigsäure,
(4S)-3-Oxo-8-[2-(5,6,7,8-tetrahydroimidazo[1,2-a]-pyrimidin-2-yl)ethoxy]-2-(2,2,2-trifluorethyl)-2,3,4,5-tetrahydro-1H-2-benzazepin-4-essigsäure,
Ethyl-3-oxo-8-[2-(5,6,7,8-tetrahydroimidazo[1,2-a]-pyrimidin-2-yl)ethoxy]-2-(2,2,2-trifluorethyl)-2,3,4,5-tetrahydro-1H-2-benzazepin-4-acetat,
Ethyl-(4S)-3-oxo-8-[2-(5,6,7,8-tetrahydroimidazo-[1,2-a]pyrimidin-2-yl)ethoxy]-2-(2,2,2-trifluorethyl)-2,3,4,5-tetrahydro-1H-2-benzazepin-4-acetat, Propyl-3-oxo-8-[2-(5,6,7,8-tetrahydroimidazo[1,2-a]-pyrimidin-2-yl)ethoxy]-2-(2,2,2-trifluorethyl)-2,3,4,5-tetrahydro-1H-2-benzazepin-4-acetat,
Propyl-(4S)-3-oxo-8-[2-(5,6,7,8-tetrahydroimidazo-[1,2-a]pyrimidin-2-yl)ethoxy]-2-(2,2,2-trifluorethyl)-2,3,4,5-tetrahydro-1H-2-benzazepin-4-acetat,
Isopropyl-3-oxo-8-[2-(5,6,7,8-tetrahydroimidazo-[1,2-a]pyrimidin-2-yl)ethoxy]-2-(2,2,2-trifluorethyl)-2,3,4,5-tetrahydro-1H-2-benzazepin-4-acetat,
Isopropyl-(4S)-3-oxo-8-[2-(5,6,7,8-tetrahydroimidazo-[1,2-a]pyrimidin-2-yl)ethoxy]-2-(2,2,2-trifluorethyl)-2,3,4,5-tetrahydro-1H-2-benzazepin-4-acetat,
Heptyl-3-oxo-8-[2-(5,6,7,8-tetrahydroimidazo[1,2-a]-pyrimidin-2-yl)ethoxy]-2-(2,2,2-trifluorethyl)-2,3,4,5-tetrahydro-1H-2-benzazepin-4-acetat,
Heptyl-(4S)-3-oxo-8-[2-(5,6,7,8-tetrahydroimidazo-[1,2-a]pyrimidin-2-yl)ethoxy]-2-(2,2,2-trifluorethyl)-2,3,4,5-tetrahydro-1H-2-benzazepin-4-acetat,
Undecyl-3-oxo-8-[2-(5,6,7,8-tetrahydroimidazo[1,2-a]-pyrimidin-2-yl)ethoxy]-2-(2,2,2-trifluorethyl)-2,3,4,5-tetrahydro-1H-2-benzazepin-4-acetat,
Undecyl-(4S)-3-oxo-8-[2-(5,6,7,8-tetrahydroimidazo-[1,2-a]pyrimidin-2-yl)ethoxy]-2-(2,2,2-trifluorethyl)-2,3,4,5-tetrahydro-1H-2-benzazepin-4-acetat,
Benzyl-3-oxo-8-[2-(5,6,7,8-tetrahydroimidazo[1,2-a]-pyrimidin-2-yl)ethoxy]-2-(2,2,2-trifluorethyl)-2,3,4,5-tetrahydro-1H-2-benzazepin-4-acetat, Benzyl-(4S)-3-oxo-8-[2-(5,6,7,8-tetrahydroimidazo-[1,2-a]pyrimidin-2-yl)ethoxy]-2-(2,2,2-trifluorethyl)-2,3,4,5-tetrahydro-1H-2-benzazepin-4-acetat,
(10-Phenyl)decyl-3-oxo-8-[2-(5,6,7,8-tetrahydroimidazo[1,2-a]pyrimidin-2-yl)ethoxy]-2-(2,2,2-trifluorethyl)-2,3,4,5-tetrahydro-1H-2-benzazepin-4-acetat,
(10-Phenyl)decyl-(4S)-3-oxo-8-[2-(5,6,7,8-tetrahydroimidazo[1,2-a]pyrimidin-2-yl)ethoxy]-2-(2,2,2-trifluorethyl)-2,3,4,5-tetrahydro-1H-2-benzazepin-4-acetat,
Pivaloyloxymethyl-3-oxo-8-[2-(5,6,7,8-tetrahydroimidazo[1,2-a]pyrimidin-2-yl)ethoxy]-2-(2,2,2-trifluorethyl)-2,3,4,5-tetrahydro-1H-2-benzazepin-4-acetat,
Pivaloyloxymethyl-(4S)-3-oxo-8-[2-(5,6,7,8-tetrahydroimidazo[1,2-a]pyrimidin-2-yl)ethoxy]-2-(2,2,2-trifluorethyl)-2,3,4,5-tetrahydro-1H-2-benzazepin-4-acetat,
(N,N-Dimethylaminocarbonyl)methyl-3-oxo-8-[2-(5,6,7,8-tetrahydroimidazo[1,2-a]pyrimidin-2-yl)-ethoxy]-2-(2,2,2-trifluorethyl)-2,3,4,5-tetrahydro-1H-2-benzazepin-4-acetat,
(N,N-Dimethylaminocarbonyl)methyl-(4S)-3-oxo-8-[2-(5,6,7,8-tetrahydroimidazo[1,2-a]pyrimidin-2-yl)-ethoxy]-2-(2,2,2-trifluorethyl)-2,3,4,5-tetrahydro-1H-2-benzazepin-4-acetat,
[2-(Morpholin-4-yl)]ethyl-3-oxo-8-[2-(5,6,7,8-tetrahydroimidazo[1,2-a]pyrimidin-2-yl)ethoxy]-2-(2,2,2-trifluorethyl)-2,3,4,5-tetrahydro-1H-2-benzazepin-4-acetat,
[2-(Morpholin-4-yl)]ethyl-(4S)-3-oxo-8-[2-(5,6,7,8-tetrahydroimidazo[1,2-a]pyrimidin-2-yl)ethoxy]-2-(2,2,2-trifluorethyl)-2,3,4,5-tetrahydro-1H-2-benzazepin-4-acetat,
(5-Methyl-2-oxo-[1,3]dioxolen-4-yl)methyl-3-oxo-8-[2-(5,6,7,8-tetrahydroimidazo[1,2-a]pyrimidin-2-yl)-ethoxy]-2-(2,2,2-trifluorethyl)-2,3,4,5-tetrahydro-1H-2-benzazepin-4-acetat,
(5-Methyl-2-oxo-[1,3]dioxolen-4-yl)methyl-(45)-3-oxo-8-[2-(5,6,7,8-tetrahydroimidazo[1,2-a]pyrimidin-2-yl)ethoxy]-2-(2,2,2-trifluorethyl)-2,3,4,5-tetrahydro-1H-2-benzazepin-4-acetat,
3-Oxo-8-[2-(1,2,3,4-tetrahydroimidazo[1,2-b][1,2,4]-triazin-6-yl)ethoxy]-2-(2,2,2-trifluorethyl)-2,3,4,5-tetrahydro-1H-2-benzazepin-4-essigsäure,
(4S)-3-Oxo-8-[2-(1,2,3,4-tetrahydroimidazo[1,2-b]-[1,2,4]triazin-6-yl)ethoxy]-2-(2,2,2-trifluorethyl)-2,3,4,5-tetrahydro-1H-2-benzazepin-4-essigsäure,
Ethyl-3-oxo-8-[2-(1,2,3,4-tetrahydroimidazo[1,2-b]-[1,2,4]triazin-6-yl)ethoxy]-2-(2,2,2-trifluorethyl)-2,3,4,5-tetrahydro-1H-2-benzazepin-4-acetat,
Ethyl-(4S)-3-oxo-8-[2-(1,2,3,4-tetrahydroimidazo-[1,2-b][1,2,4]triazin-6-yl)ethoxy]-2-(2,2,2-trifluorethyl)-2,3,4,5-tetrahydro-1H-2-benzazepin-4-acetat,
Propyl-3-oxo-8-[2-(1,2,3,4-tetrahydroimidazo[1,2-b]-[1,2,4]triazin-6-yl)ethoxy]-2-(2,2,2-trifluorethyl)-2,3,4,5-tetrahydro-1H-2-benzazepin-4-acetat, Propyl-(4S)-3-oxo-8-[2-(1,2,3,4-tetrahydroimidazo-[1,2-b][1,2,4]triazin-6-yl)ethoxy]-2-(2,2,2-trifluorethyl)-2,3,4,5-tetrahydro-1H-2-benzazepin-4-acetat,
Isopropyl-3-oxo-8-[2-(1,2,3,4-tetrahydroimidazo-[1,2-b][1,2,4]triazin-6-yl)ethoxy]-2-(2,2,2-trifluorethyl)-2,3,4,5-tetrahydro-1H-2-benzazepin-4-acetat,
Isopropyl-(4S)-3-oxo-8-[2-(1,2,3,4-tetrahydroimidazo-[1,2-b][1,2,4]triazin-6-yl)ethoxyl-2-(2,2,2-trifluorethyl)-2,3,4,5-tetrahydro-1H-2-benzazepin-4-acetat,
Heptyl-3-oxo-8-[2-(1,2,3,4-tetrahydroimidazo[1,2-b]-[1,2,4]triazin-6-yl)ethoxy]-2-(2,2,2-trifluorethyl)-2,3,4,5-tetrahydro-1H-2-benzazepin-4-acetat,
Heptyl-(4S)-3-oxo-8-[2-(1,2,3,4-tetrahydroimidazo-[1,2-b][1,2,4]triazin-6-yl)ethoxy]-2-(2,2,2-trifluorethyl)-2,3,4,5-tetrahydro-1H-2-benzazepin-4-acetat,
Undecyl-3-oxo-8-[2-(1,2,3,4-tetrahydroimidazo[1,2-b]-[1,2,4]triazin-6-yl)ethoxy]-2-(2,2,2-trifluorethyl)-2,3,4,5-tetrahydro-1H-2-benzazepin-4-acetat,
Undecyl-(4S)-3-oxo-8-[2-(1,2,3,4-tetrahydroimidazo-[1,2-b][1,2,4]triazin-6-yl)ethoxy]-2-(2,2,2-trifluorethyl)-2,3,4,5-tetrahydro-1H-2-benzazepin-4-acetat,
Benzyl-3-oxo-8-[2-(1,2,3,4-tetrahydroimidazo[1,2-b]-[1,2,4]triazin-6-yl)ethoxy]-2-(2,2,2-trifluorethyl)-2,3,4,5-tetrahydro-1H-2-benzazepin-4-acetat,
Benzyl-(4S)-3-oxo-8-[2-(1,2,3,4-tetrahydroimidazo-[1,2-b] [1,2,4]triazin-6-yl)ethoxy]-2-(2,2,2-trifluorethyl)-2,3,4,5-tetrahydro-1H-2-benzazepin-4-acetat, (10-Phenyl)decyl-3-oxo-8-[2-(1,2,3,4-tetrahydroimidazo[1,2-b][1,2,4]triazin-6-yl)ethoxyl-2-(2,2,2-trifluorethyl)-2,3,4,5-tetrahydro-1H-2-benzazepin-4-acetat,
(10-Phenyl)decyl-(4S)-3-oxo-8-[2-(1,2,3,4-tetrahydroimidazo[1,2-b][1,2,4]triazin-6-yl)ethoxyl-2-(2,2,2-trifluorethyl)-2,3,4,5-tetrahydro-1H-2-benzazepin-4-acetat,
Pivaloyloxymethyl-3-oxo-8-[2-(1,2,3,4-tetrahydroimidazo[1,2-b][1,2,4]triazin-6-yl)ethoxyl-2-(2,2,2-trifluorethyl)-2,3,4,5-tetrahydro-1H-2-benzazepin-4-acetat,
Pivaloyloxymethyl-(4S)-3-oxo-8-[2-(1,2,3,4-tetrahydroimidazo[1,2-b][1,2,4]triazin-6-yl)ethoxy]-2-(2,2,2-trifluorethyl)-2,3,4,5-tetrahydro-1H-2-benzazepin-4-acetat,
(N,N-Dimethylaminocarbonyl)methyl-3-oxo-8-[2-(1,2,3,4-tetrahydroimidazo[1,2-b][1,2,4]triazin-6-yl)ethoxy]-2-(2,2,2-trifluorethyl)-2,3,4,5-tetrahydro-1H-2-benzazepin-4-acetat,
(N,N-Dimethylaminocarbonyl)methyl-(4S)-3-oxo-8-[2-(1,2,3,4-tetrahydroimidazo[1,2-b][1,2,4]triazin-6-yl)ethoxy]-2-(2,2,2-trifluorethyl)-2,3,4,5-tetrahydro-1H-2-benzazepin-4-acetat,
[2-(Morpholin-4-yl)]ethyl-3-oxo-8-[2-(1,2,3,4-tetrahydroimidazo[1,2-b][1,2,4]triazin-6-yl)ethoxy]-2-(2,2,2-trifluorethyl)-2,3,4,5-tetrahydro-1H-2-benzazepin-4-acetat,
[2-(Morpholin-4-yl)]ethyl-(4S)-3-oxo-8-[2-(1,2,3,4-tetrahydroimidazo[1,2-b][1,2,4]-triazin-6-yl)ethoxy]-2-(2,2,2-trifluorethyl)-2,3,4,5-tetrahydro-1H-2-benzazepin-4-acetat,
(5-Methyl-2-oxo-[1,3]dioxolen-4-yl)methyl-3-oxo-8-[2-(1,2,3,4-tetrahydroimidazo[1,2-b][1,2,4]triazin-6-yl)ethoxy]-2-(2,2,2-trifluorethyl)-2,3,4,5-tetrahydro-1H-2-benzazepin-4-acetat oder
(5-Methyl-2-oxo-[1,3]dioxolen-4-yl)methyl-(4S)-3-oxo-8-[2-(1,2,3,4-tetrahydroimidazo[1,2-b][1,2,4]triazin-6-yl)ethoxy]-2-(2,2,2-trifluorethyl)-2,3,4,5-tetrahydro-1H-2-benzazepin-4-acetat.

17. Arzneimittelzusammensetzung zur Verwendung gemäss irgendeinem der Ansprüche 1 bis 16, wobei die Verabreichungsform eine orale Verabreichung ist.

18. Arzneimittelzusammensetzung zur Verwendung gemäss irgendeinem der Ansprüche 1 bis 16, wobei die Augenerkrankung eine intraokulare neovaskuläre Erkrankung ist.

19. Arzneimittelzusammensetzung zur Verwendung gemäss irgendeinem der Ansprüche 1 bis 16, wobei die Augenerkrankung eine altersbedingte Makuladegeneration ist.

## Revendications

1. Composition médicale pour une utilisation dans le traitement ou la prophylaxie de maladies provoquées par une angiogenèse des yeux qui comprend un composé représenté par la formule (I) : 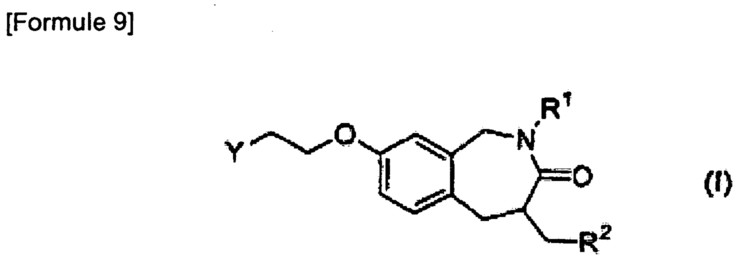 dans laquelle R¹ représente un groupe alkyle en C₁-C₆ ou un groupe halogénoalkyle en C₁-C₆, R² représente un groupe carboxyle qui peut être protégé, Y représente un groupe représenté par la formule (II) : 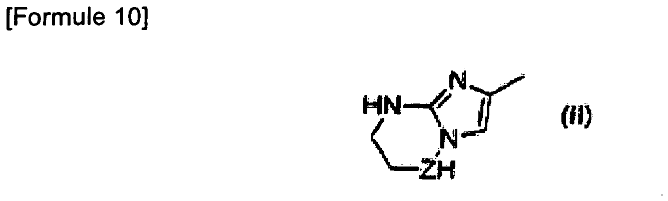 dans laquelle Z représente CH ou un atome d'azote,
ou un sel pharmaceutiquement acceptable de celui-ci comme principe actif.

2. Composition médicale pour une utilisation selon la revendication 1, dans laquelle R¹ est un groupe alkyle en C₁-C₄, un groupe fluoroalkyle en C₁-C₄ ou un
groupe chloroalkyle en C₁-C₄.

3. Composition médicale pour une utilisation selon la revendication 1, dans laquelle R¹ est un groupe éthyle, un groupe propyle, un groupe isopropyle, un groupe butyle, un groupe isobutyle, un groupe sec-butyle, un groupe difluorométhyle, un groupe trifluorométhyle, un groupe 2-fluoroéthyle, un groupe 2,2-difluoroéthyle, un groupe 2,2,2-trifluoroéthyle ou un groupe 2,2,2-trichloroéthyle.

4. Composition médicale pour une utilisation selon la revendication 1, dans laquelle R¹ est un groupe éthyle, un groupe difluorométhyle, un groupe trifluorométhyle, un groupe 2-fluoroéthyle, un groupe 2,2-difluoroéthyle ou un groupe 2,2,2-trifluoroéthyle.

5. Composition médicale pour une utilisation selon la revendication 1, dans laquelle R¹ est un groupe éthyle, un groupe trifluorométhyle ou un groupe 2,2,2-trifluoroéthyle.

6. Composition médicale pour une utilisation selon la revendication 1, dans laquelle R² est un groupe carboxyle qui peut être protégé par un groupe alkyle en C₁-C₁₂, un groupe aralkyle en C₇-C₁₈, un groupe alkyle en C₁-C₂ substitué par un groupe alcanoyloxy en C₂-C₅, un groupe alkyle en C₁-C₂ substitué par un groupe (alcoxy en C₁-C₄)carbonyloxy, un groupe N,N-diméthylaminocarbonylméthyle, un groupe 2-(morpholin-4-yl)éthyle, un groupe (5-méthyl-2-oxo-1,3-dioxolèn-4-yl)méthyle ou un groupe (5-phényl-2-oxo-1,3-dioxolèn-4-yl)méthyle.

7. Composition médicale pour une utilisation selon la revendication 1, dans laquelle R² est un groupe carboxyle qui peut être protégé par un groupe méthyle, un groupe éthyle, un groupe propyle, un groupe isopropyle, un groupe 1-éthylpropyle, un groupe butyle, un groupe isobutyle, un groupe sec-butyle, un groupe tert-butyle, un groupe 3,3-diméthylbutyle, un groupe pentyle, un groupe isopentyle, un groupe néopentyle, un groupe tert-pentyle, un groupe 1-méthylbutyle, un groupe hexyle, un groupe 1-méthylpentyle, un groupe 2-méthylpentyle, un groupe 3-méthylpentyle, un groupe 1-éthylbutyle, un groupe 2-éthylbutyle, un groupe heptyle, un groupe octyle, un groupe nonyle, un groupe décyle, un groupe undécyle, un groupe dodécyle, un groupe benzyle, un groupe phénéthyle, un groupe phénylpropyle, un groupe phénylbutyle, un groupe phénylpentyle, un groupe phénylhexyle, un groupe phénylheptyle, un groupe phényloctyle, un groupe phénylnonyle, un groupe phényldécyle, un groupe phénylundécyle, un groupe phényldodécyle, un groupe acétoxyméthyle, un groupe 1-acétoxyéthyle, un groupe pivaloyloxyméthyle, un groupe 1-pivaloyloxyéthyle, un groupe éthoxycarbonyloxyméthyle, un groupe 1-éthoxycarbonyloxyéthyle, un groupe N,N-diméthylaminocarbonylméthyle, un groupe 2-(morpholin-4-yl)éthyle ou un groupe (5-méthyl-2-oxo-1 ,3-dioxolèn-4-yl)méthyle.

8. Composition médicale pour une utilisation selon la revendication 1, dans laquelle R² est un groupe carboxyle qui peut être protégé par un groupe méthyle, un groupe éthyle, un groupe propyle, un groupe isopropyle, un groupe 1-éthylpropyle, un groupe butyle, un groupe 3,3-diméthylbutyle, un groupe pentyle, un groupe hexyle, un groupe heptyle, un groupe octyle, un groupe nonyle, un groupe décyle, un groupe undécyle, un groupe dodécyle, un groupe benzyle, un groupe phénéthyle, un groupe phénylpropyle, un groupe phénylbutyle, un groupe phénylpentyle, un groupe phénylhexyle, un groupe phénylheptyle, un groupe phényloctyle, un groupe phénylnonyle, un groupe phényldécyle, un groupe phénylundécyle, un groupe phényldodécyle, un groupe acétoxyméthyle, un groupe pivaloyloxyméthyle, un groupe 1-pivaloyloxyéthyle, un groupe N,N-diméthylaminocarbonylméthyle, un groupe 2-(morpholin-4-yl)éthyle ou un groupe (5-méthyl-2-oxo-1,3-dioxolèn-4-yl)méthyle.

9. Composition médicale pour une utilisation selon la revendication 1, dans laquelle R² est un groupe carboxyle qui peut être protégé par un groupe éthyle, un groupe propyle, un groupe isopropyle, un groupe butyle, un groupe pentyle, un groupe hexyle, un groupe heptyle, un groupe octyle, un groupe nonyle, un groupe décyle, un groupe undécyle, un groupe benzyle, un groupe 2-phénéthyle, un groupe 3-phénylpropyle, un groupe 4-phénylbutyle, un groupe 5-phénylpentyle, un groupe 6-phénylhexyle, un groupe 7-phénylheptyle, un groupe 8-phényloctyle, un groupe 9-phénylnonyle, un groupe 10-phényldécyle, un groupe pivaloyloxyméthyle, un groupe N,N-diméthylaminocarbonylméthyle, un groupe 2-(morpholin-4-yl)éthyle ou un groupe (5-méthyl-2-oxo-1,3-dioxolèn-4-yl)méthyle.

10. Composition médicale pour une utilisation selon la revendication 1, dans laquelle R¹ est un groupe alkyle en C₁-C₄, un groupe fluoroalkyle en C₁-C₄ ou un groupe chloroalkyle en C₁-C₄, et
R² est un groupe carboxyle qui peut être protégé par un groupe alkyle en C₁-C₁₂, un groupe aralkyle en C₇-Cₗ₈, un groupe alkyle en C₁-C₂ substitué par un groupe alcanoyloxy en C₂-C₅, un groupe alkyle en C₁-C₂ substitué par un groupe (alcoxy en C₁-C₄)carbonyloxy, un groupe N,N-diméthylaminocarbonylméthyle, un groupe 2-(morpholin-4-yl)éthyle, un groupe (5-méthyl-2-oxo-1,3-dioxolèn-4-yl)méthyle ou un groupe (5-phényl-2-oxo-1,3-dioxolèn-4)yl)méthyle.

11. Composition médicale pour une utilisation selon la revendication 1, dans laquelle R¹ est un groupe éthyle, un groupe propyle, un groupe isopropyle, un groupe butyle, un groupe isobutyle, un groupe sec-butyle, un groupe difluorométhyle, un groupe trifluorométhyle, un groupe 2-fluoroéthyle, un groupe 2,2-difluoroéthyle, un groupe 2,2,2-trifluoroéthyle ou un groupe 2,2,2-trichloroéthyle, et
R² est un groupe carboxyle qui peut être protégé par un groupe méthyle, un groupe éthyle, un groupe propyle, un groupe isopropyle, un groupe 1-éthylpropyle, un groupe butyle, un groupe isobutyle, un groupe sec-butyle, un groupe tert-butyle, un groupe 3,3-diméthylbutyle, un groupe pentyle, un groupe isopentyle, un groupe néopentyle, un groupe tert-pentyle, un groupe 1-méthylbutyle, un groupe hexyle, un groupe 1-méthylpentyle, un groupe 2-méthylpentyle, un groupe 3-méthylpentyle, un groupe 1-éthylbutyle, un groupe 2-éthylbutyle, un groupe heptyle, un groupe octyle, un groupe nonyle, un groupe décyle, un groupe undécyle, un groupe dodécyle, un groupe benzyle, un groupe phénéthyle, un groupe phénylpropyle, un groupe phénylbutyle, un groupe phénylpentyle, un groupe phénylhexyle, un groupe phénylheptyle, un groupe phényloctyle, un groupe phénylnonyle, un groupe phényldécyle, un groupe phénylundécyle, un groupe phényldodécyle, un groupe acétoxyméthyle, un groupe 1-acétoxyéthyle, un groupe pivaloyloxyméthyle, un groupe 1-pivaloyloxyéthyle, un groupe éthoxycarbonyloxyméthyle, un groupe 1-éthoxycarbonyloxyéthyle, un groupe N,N-diméthylaminocarbonylméthyle, un groupe 2-(morpholin-4-yl)éthyle ou un groupe (5-méthyl-2-oxo-1,3-dioxolèn-4-yl)méthyle.

12. Composition médicale pour une utilisation selon la revendication 1, dans laquelle R¹ est un groupe éthyle, un groupe difluorométhyle, un groupe trifluorométhyle, un groupe 2-fluoroéthyle, un groupe 2,2-difluoroéthyle ou un groupe 2,2,2-trifluoroéthyle, et
R² est un groupe carboxyle qui peut être protégé par un groupe méthyle, un groupe éthyle, un groupe propyle, un groupe isopropyle, un groupe 1-éthylpropyle, un groupe butyle, un groupe 3,3-diméthylbutyle, un groupe pentyle, un groupe hexyle, un groupe heptyle, un groupe octyle, un groupe nonyle, un groupe décyle, un groupe undécyle, un groupe dodécyle, un groupe benzyle, un groupe phénéthyle, un groupe phénylpropyle, un groupe phénylbutyle, un groupe phénylpentyle, un groupe phénylhexyle, un groupe phénylheptyle, un groupe phényloctyle, un groupe phénylnonyle, un groupe phényldécyle, un groupe phénylundécyle, un groupe phényldodécyle, un groupe acétoxyméthyle, un groupe pivaloyloxyméthyle, un groupe 1-pivaloyloxyéthyle, un groupe N,N-diméthylaminocarbonylméthyle, un groupe 2-(morpholin-4-yl)éthyle ou un groupe (5-méthyl-2-oxo-1,3-dioxolèn-4-yl)méthyle.

13. Composition médicale pour une utilisation selon la revendication 1, dans laquelle R¹ est un groupe éthyle, un groupe trifluorométhyle ou un groupe 2,2,2-trifluoroéthyle, et
R² est un groupe carboxyle qui peut être protégé par un groupe éthyle, un groupe propyle, un groupe isopropyle, un groupe butyle, un groupe pentyle, un groupe hexyle, un groupe heptyle, un groupe octyle, un groupe nonyle, un groupe décyle, un groupe undécyle, un groupe benzyle, un groupe 2-phénéthyle, un groupe 3-phénylpropyle, un groupe 4-phénylbutyle, un groupe 5-phénylpentyle, un groupe 6-phénylhexyle, un groupe 7-phénylheptyle, un groupe phényloctyle, un groupe 9-phénylnonyle, un groupe 10-phényldécyle, un groupe pivaloyloxyméthyle, un groupe N,N-diméthylaminocarbonylméthyle, un groupe 2-(morpholin-4-yl)éthyle ou un groupe (5-méthyl-2-oxo-1,3-dioxolèn-4-yl)méthyle.

14. Composition médicale pour une utilisation selon la revendication 1, dans laquelle le composé est représenté par la formule suivante (III) : 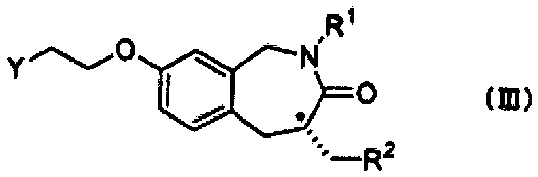 dans laquelle l'atome de carbone auquel * est attaché a une configuration (S), R¹, R² et Y ont les mêmes significations que définies ci-dessus.

15. Composition médicale pour une utilisation selon l'une quelconque des revendications 2 à 13, dans laquelle le composé est représenté par la formule (III).

16. Composition médicale pour une utilisation selon la revendication 1, dans laquelle le composé benzazépinone est
l'acide 3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétique,
l'acide (4S)-3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétique,
le 3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétate d'éthyle,
le (4S)-3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétate d'éthyle,
le 3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétate de propyle,
le (4S)-3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétate de propyle,
le 3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétate d'isopropyle,
le (4S)-3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétate d'isopropyle,
le 3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétate d'heptyle,
le (4S)-3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétate d'heptyle,
le 3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétate d'undécyle,
le (4S)-3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétate d'undécyle,
le 3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétate de benzyle,
le (4S)-3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétate de benzyle,
le 3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétate de (10-phényl)décyle,
le (4S)-3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétate de (10-phényl)décyle,
le 3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétate de pivaloyloxyméthyle,
le (45)-3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétate de pivaloyloxyméthyle,
le 3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétate de (N,N-diméthylaminocarbonyl)méthyle,
le (4S)-3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétate de (N,N-diméthylaminocarbonyl)méthyle,
le 3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétate de [2-(morpholin-4-yl)]éthyle,
le (4S)-3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétate de [2-(morpholin-4-yl)]éthyle,
le 3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétate de (5-méthyl-2-oxo-[1,3]dioxolèn-4-yl)méthyle,
le (4S)-3-oxo-8-[2-(5,6,7,8-tétrahydroimidazo[1,2-a]pyrimidin-2-yl)éthoxy]-2-(2,2,2-trifluoroéthyl)-2,3,4,5-tétrahydro-1H-2-benzazépin-4-acétate de (5-méthyl-2-oxo-[1,3]dioxolèn-4-yl)méthyle,
l'acide 3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétique,
l'acide (4S)-3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétique,
le 3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétate d'éthyle,
le (4S)-3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétate d'éthyle,
le 3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétate de propyle,
le (4S)-3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétate de propyle,
le 3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétate d'isopropyle,
le (4S)-3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétate d'isopropyle,
le 3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétate d'heptyle,
le (4S)-3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétate d'heptyle,
le 3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétate d'undécyle,
le (4S)-3-oxo-8-[2-(1,2,3,4-tétrahydroimidazo[1,2-b][1,2,4](triazin-6-yl)éthoxy]-2-(2,2,2-trrfluoroéthyl)-2,3,4,5-tétrahydro-1H-2-benzazépin-4-acétate d'undécyle,
le 3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétate de benzyle,
le (4S)-3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétate de benzyle,
le 3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétate de (10-phényl)décyle,
le (4S)-3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétate de (10-phényl)décyle,
le 3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétate de pivaloyloxyméthyle,
le (4S)-3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétate de pivaloyloxyméthyle,
le 3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétate de (N,N-diméthylaminocarbonyl)méthyle,
le (4S)-3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétate de (N,N-diméthylaminocarbonyl)méthyle,
le 3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétate de [2-(morpholin-4-yl)]éthyle,
le (4S)-3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétate de [2-(morpholin-4-yl)]éthyle,
le 3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétate de (5-méthyl-2-oxo-[1,3]dioxolèn-4-yl)méthyle ou
le (4S)-3-oxo-8-[2-(1,2,3,4-tétrahydroimidazo[1,2-b][1,2,4](triazin-6-yl)éthoxy]-2-(2,2,2-trifluoroéthyl)-2,3,4,5-tétrahydro-1H-2-benzazépin-4-acétate de (5-méthyl-2-oxo-[1,3]dioxolèn-4-yl)méthyle.

17. Composition médicale pour une utilisation selon l'une quelconque des revendications 1 à 16, dans laquelle la forme d'administration est une administration orale.

18. Composition médicale pour une utilisation selon l'une quelconque des revendications 1 à 16, dans laquelle la maladie ophtalmique est la maladie néovasculaire intraoculaire.

19. Composition médicale pour une utilisation selon l'une quelconque des revendications 1 à 16, dans laquelle la maladie ophtalmique est une dégénérescence maculaire associée à l'âge.
